# EUROPÄISCHE PATENTSCHRIFT

(11) **EP 2 280 941 B1**
(45) Veröffentlichungstag und Bekanntmachung des Hinweises auf die Patenterteilung: **06.05.2015**
(21) Anmeldenummer: 09725704.2
(22) Anmeldetag: 25.03.2009
(51) Int. Cl.: C07D 209/30, C07D 401/06, C07D 417/08, A61K 31/404, A61P 25/00

(54) **(HETERO-)ARYL-CYCLOHEXAN-DERIVATE**
(HETERO-)ARYL CYCLOHEXANE DERIVATIVES
DÉRIVÉS DE (HÉTÉRO-)ARYL-CYCLOHEXANE

(30) Priorität: 27.03.2008 EP 08005797
(43) Veröffentlichungstag der Anmeldung: 09.02.2011
(73) Patentinhaber: Grünenthal GmbH, 52078 Aachen (DE)
(72) Erfinder: ZEMOLKA, Saskia, 52066 Aachen (DE); SCHUNK, Stefan, 52080 Aachen (DE); NOLTE, Bert, 53902 Bad Münstereifel (DE); LINZ, Klaus, 53359 Rheinbach (DE); SCHRÖDER, Wolfgang, 52074 Aachen (DE); ENGLBERGER, Werner, 52223 Stolberg (DE); SCHICK, Hans, 13086 Berlin (DE); SONNENSCHEIN, Helmut, 10245 Berlin (DE); HENKEL, Birgitta, 12555 Berlin (DE); BÁLINT, József, 12526 Berlin (DE)
(74) Vertreter: Bülle, Jan
(86) Internationale Anmeldenummer: PCT/EP2009/002172
(87) Internationale Veröffentlichungsnummer: WO 2009/118163

(56) Entgegenhaltungen:
- WO-A-2008/009415
- WO-A-2008/009416

## Beschreibung

Die vorliegende Erfindung betrifft substituierte Cyclohexan-Derivate, welche eine Affinität an den µ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2002/090317, WO2002/90330, WO2003/008370, WO2003/008731, WO2003/080557, WO2004/043899, WO2004/043900, WO2004/043902, WO2004/043909, WO2004/043949, WO2004/043967, WO2005/063769, WO2005/066183, WO2005/110970, WO2005/110971. WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner zeigen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner zeigen die bekannten Verbindungen mitunter eine nur geringe Selektivität gegenüber dem kappa-Opioid-Rezeptor auf, welcher für Nebenwirkungen, insbesondere Dysphorie, Sedation, Diurese verantwortlich ist. Darüber hinaus zeigen die bekannten Verbindungen mitunter eine sehr hohe Affinität an den µ-Opioid-Rezeptor, welcher im Zusammenhang mit anderen Nebenwirkungen, insbesondere Atemdepression, Obstipation und Suchtabhängigkeit zu stehen scheint.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte Cyclohexan-Derivate hergestellt werden können, welche eine Affinität an den µ-Opioid-Rezeptor und den ORL1-Rezeptor aufweisen.

Die Erfindung betrifft [X].

Bei der Zusammenfassung verschiedener Reste, beispielsweise Y₁, Y₁', Y₂, Y₂', Y₃, Y₃', Y₄ und Y₄' sowie der Zusammenfassung von Resten an deren Substituenten, wie z. B. -OR₀, -OC(=O)R₀, -OC(=O)NHR₀, kann ein Substituent, z.B. R₀, für zwei oder mehrere Reste, beilspielsweise -OR₀, -OC(=O)R₀, -OC(=O)NHR₀, innerhalb einer Substanz unterschiedliche Bedeutungen annehmen.

Die erfindungsgemäßen Verbindungen zeigen gute Bindung an den ORL1-Rezeptor und den µ-Opiold-Rezeptor.

In einer bevorzugten Ausführungsform weisen die erfindungsgemäßen Verbindungen ein Verhältnis ORL1/µ-Affinität von wenigstens 0,1 auf. Das ORL1/µ-Verhältnis ist definiert als 1/[K_{i(ORL1)}/K_{i(µ)}]. Besonders bevorzugt beträgt das ORL1/µ-Verhältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1/µ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/µ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen Kᵢ-Wert am µ-Opioid-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und µ-Opioid-Rezeptor, bei denen das durch 1/[K_{i(ORL1)}/K_{i(µ)}] definierte Verhältnis von ORL1 zu µ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan-oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Bevorzugt sind Y₁, Y₁', Y₂, Y₂ , Y₃, Y₃', Y₄ und Y₄' jeweils unabhängig voneinander ausgewählt aus der Gruppe bestehend aus -H, -F, -Cl, -Br, -I, -CN, -NH₂, -NH-C₁₋₆-Aliphat, -NH-C₃-₈-Cycloaliphat, -NH-C₁₋₆-Aliphat-OH, -N(C₁₋₆-Aliphat)₂, -N(C₃₋₈-Cycloaliphat)₂, -N(C₁₋₆-Aliphat-OH)₂, -NO₂, -NH-C₁₋₆-Aliphat-C₃₋₈-Cycloaliphat, -NH-C₁₋₆-Aliphat-Aryl, -NH-C₁-₆-Aliphat-Heteroaryl, -NH-Aryl, -NH-Heteroaryl, -SH, -S-C₁₋₆-Aliphat, -S-C₃₋₈-Cycloaliphat, -S-C₁₋₆-Aliphat-C₃₋₈-Cycloaliphat, -S-C₁₋₆-Aliphat-Aryl, -S-C₁₋₆-Aliphat-Heteroaryl, -S-Aryl, -S-Heteroaryl, -OH, -O-C₁₋₆-Aliphat, -O-C₃₋₆-Cycloatlphat, -O-C₁₋₆-Aliphat-OH, -O-C₁₋₆-Aliphat-C₃₋₆-Cycloaliphat, -O-C₁₋₆-Aliphat-Aryl, -O-C₁₋₆-Aliphat-Heretoaryl, -O-Aryl, -O-Heteroaryl, -O-C(=O)C₁₋₆-Aliphat, -O-C(=O)C₃₋₆-Cycloaliphat, -O-C(=O)C₁₋₆-Aliphat-OH, -O-C(=O)C₁₋₆-Aliphat-C₃₋₈-Cycloaliphat, -O-C(=O)C₁₋₈-Aliphat-Aryl, -O-C(=O)C₁₋₆-Aliphat-Heretoaryl, -O-C(=O)Aryl, -O-C(=O)Heteroaryl, -C₁₋₆-Aliphat, -C₃₋₆-Cycloaliphat, -C₁₋₆-Aliphat-C₃₋₈-Cycloaliphat, -C₁₋₆-Aliphat-Aryl, -C₁₋₆-Aliphat-Heteroaryl, -Aryl, -Heteroaryl, -C(=O)C₁₋₆Aliphat, -C(=O)C₃₋₈-Cycloaliphat, -C(=O)C₁₋₆-Aliphat-C₃₋₈-Cycloaliphat, -C(=O)C₁₋₆-Aliphat-Ary, - C(=O)C₁₋₆-Aliphat-Heteroaryl, -C(=O)Aryl, -C(=O)Heteroaryl, -CO₂H, -CO₂-C₁₋₆-Aliphat, -CO₂-C₃₋₈-Cycloaliphat, -CO₂-C₁₋₆-Aliphat-C₃₋₈-Cycloaliphat, -CO₂-C₁₋₆-Aliphat-Aryl, -CO₂-C₁₋₆-Aliphat-Heteroaryl, -CO₂-Aryl. -CO₂-Heteroaryl; oder Y₁ und Y₁', oder Y₂ und Y₂', oder Y₃ und Y₃', oder Y₄ und Y₄' stehen gemeinsam für =O Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -NH₂ und -OH.

In einer bevorzugten Ausführungsform ist einer der Reste Y₁, Y₁', Y₂, Y₂', Y₃, Y₃', Y₄ und Y₄' ungleich -H und die übrigen Reste stehen für -H.

Besonders bevorzugt stehen Y₁, Y₁', Y₂, V₂', Y₃, V₃', Y₄ und Y₄' jeweils für -H.

R₀ steht bevorzugt jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₄-Aliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂-Cycloaliphat, -Aryl oder -Heteroaryl jeweils über eine zweibindige Brücke -C₁₋₈-Aliphat gebunden sind. Bevorzugte Beispiele für -C₁₋₈-Aliphat-Aryl sind -CH₂-C₆H₅, -CH₂CH₂-C₈H₅, und -CH=CH-C₆H₅.

Bevorzugt stehen R, und R₂, unabhängig voneinander für -H; -C₁₋₆-Aliphat; -C₃₋₈-Cycloaliphat, -C₁₋₆-Aliphat-Aryl. -C₁₋₆-Aliphat-C₃₋₈-Cycloaliphat oder -C₁₋₆-Aliphat-Heteroaryl; oder die Reste R₁ und R₂ bilden zusammen einen Ring und bedeuten -CH₂CH₂OCH₂CH₂-, -CH₂CH₂NR₄CH₂CH₂- oder -(CH₂)₃₋₆-. Bevorzugter stehen R₁ und R₂ unabhängig voneinander für -H; -C₁₋₅-Aliphat, oder die Reste R₁ und R₂ bilden zusammen einen Ring und bedeuten -CH₂CH₂OCH₂CH₂-, -CH₂CH₂NR₄-CH₂CH₂- oder -(CH₂)₃₋₆-, wobei R₄ bevorzugt -H oder -C₁₋₆-Aliphat bedeutet. Besonders bevorzugt sind Verbindungen, worin R₁ und R₂ unabhängig voneinander für -CH₃ oder -H stehen, wobei R₁ und R₂ nicht gleichzeitig -H bedeuten; oder R₁ und R₂ bilden einen Ring und bedeuten -(CH₂)₃₋₄-. Ganz besonders bevorzugt sind Verbindungen, worin R₁ und R₂ für -CH₃ oder worin R₁ für -H und R₂ für -CH₃ stehen.

Besonders bevorzugt bilden R₁ und R₂ zusammen mit dem Stickstoffatom, an welches sie gebunden sind, eine der folgenden funktionellen Gruppen:

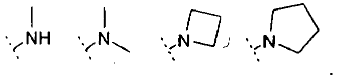

Bevorzugt steht R₃ für -C₁₋₈-Aliphat. -C₃₋₃-Cycloaliphat, -Aryl, -Heteroaryl; oder für jeweils über eine -C₁₋₃-Aliphat-Gruppe gebundenes -Aryl, -Heteroaryl oder -C₃₋₈-Cycloaliphat.

Besonders bevorzugt steht R₃ für -Ethyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl, -Benzothiophenyl, -Furyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -C₁₋₃-Aliphat-Gruppe gebundenes -C₅₋₆-Cycloaliphat, -Phenyl, -Naphthyl, -Anthracenyl, -Thiophenyl, -Benzothiophenyl, -Pyridyl, -Furyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert oder ein- oder mehrfach substituiert.

Bevorzugter steht R₃ für -Propyl, -Butyl, -Pentyl, -Hexyl, -Phenyl, -Furyl, -Thiophenyl, -Naphthyl, -Benzyl, -Benzofuranyl, -Indolyl, -Indanyl, -Benzodioxanyl, -Benzodioxolanyl, -Pyridyl, -Pyrimidyl, -Pyrazinyl, -Triazolyl oder -Benzothiophenyl, jeweils unsubstituiert oder ein- oder mehrfach substituiert; über eine gesättigte, unverzweigte -C₁₋₃-Aliphat-Gruppe gebundenes -Phenyl, -Furyl oder -Thiophenyl, jeweils unsubstituiert oder ein- oder mehrfach substituiert.

Noch bevorzugter steht R₃ für -Propyl, -Butyl, -Pentyl, -Hexyl, -Phenyl, -Phenethyl, -Thiophenyl, -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder -Phenethyl.

Ganz besonders bevorzugt steht R₃ für -Butyl, -Ethyl, -3-Methoxypropyl, -Benzothiophenyl, -Phenyl, -3-Methylphenyl, -3-Fluorphenyl, -Benzo[1,3]-dioxolyl, -Benzyl, -1-Methyl-1,2,4-triazolyl, -Thienyl oder -Phenethyl.

Am bevorzugtesten steht R₃ für -Phenyl, -Benzyl oder -Phenethyl, jeweils unsubstituiert oder am Ring ein- oder mehrfach substituiert; -C₁₋₅-Aliphat, -C₄₋₆-Cycloaliphat, -Pyridyl, -Thienyl, -Thiazolyl, -Imidazolyl, -1,2,4 Triazolyl oder -Benzimidazolyl, unsubstituiert oder ein- oder mehrfach substituiert.

Insbesondere bevorzugt steht R₃ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -OCH₃ oder -OC₂H₅, wobei vorzugsweise -Thienyl, -Pyridyl, -Thiazolyl, -Imidazolyl, -1,2,4 Triazolyl und -Benzimidazolyl unsubstituiert sind.

Insbesondere bevorzugt steht R₃ für -Phenyl, unsubstituiert oder einfach substituiert mit -F, -Cl, -CN, -CH₃; -Thienyl; -Ethyl, -n-Propyl oder -n-Butyl, unsubstituiert oder ein- oder mehrfach substituiert mit -OCH₃, -OH oder -OC₂H₅; insbesondere mit -OCH₃.

Bevorzugt steht R₄ für -H, -C₁₋₅-Aliphat, -C₃₋₈-Cycloaliphat, -Aryl, -Heteroaryl, -C₁₋₆-Aliphat-Aryl, -C₁₋₆-Aliphat-C₃₋₈-Cycloaliphat, -C₁₋₆-Aliphat-Heteroaryl, -C(=O)Aryl, -C(=O)Heteroaryl, oder -C(=O)C₁₋₆-Aliphat, bevorzugter für -H oder -C₁₋₅-Aliphat.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen-.

Bevorzugt ist Aliphat jeweils ein verzweigter oder unverzweigter, gesättigter oder ein einoder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -NO₂, -CHO, =O, -R₀, -C(=O)R₀, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O-, -NHC(=O)R₀, -NHC(=O)OR₀, -NHC(=O)NH₂, -NHC(=O)NHR₀, -NHC(=O)N(R₀)₂, -NHS(=O)₁₋₂R₀, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C=C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂-CH₂CH₃ und -CH₂CH₂-CH₂CH₂CH₂CH₃; aber auch -CH=CH₂, -C=CH, -CH₂CH=CH₂, -CH=CHCH₃, -CH₂C=CH, -C=CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH_{z}CH(CH₃)-CH₂-, -CH_{Z}CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡CCH₂-. Bevorzugte substituierte einbindige Aliphaten umfassen -CH₂F, -CHF₂, -CF₃, -CH₂CF₃, -CF₂CF₃, -CH₂OH, -CH₂CH₂OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂- und -CH₂CHOHCH₂-. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -NHS(=O)₁₋₂R₀, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alkyl, -OC(=O)C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅oder -N(CH₃)₂ bedeutet. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂-OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

(Hetero-)aryl steht für Heteroaryl oder Aryl. Dabei können -Aryl und -Heteroaryl jeweils unsubstituiert oder ein- oder mehrfach substituiert sein, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F, -Cl, -Br, -NO₂, -CN, -Heteroaryl, -C₁₋₈-Aliphat-Aryl und -C₁₋₈-Aliphat-Heteroaryl (z.B. -Ethyl-4-Pyridyl).

In einer bevorzugten Ausführungsform ist (Hetero-)aryl ausgewählt aus der Gruppe bestehend aus Phenyl, Benzyl, Pyrrolyl, Furyl, Thienyl, Pyridyl, Indolyl, Benzofuryl und Benzothienyl, wobei diese jeweils unsubstituiert oder ein- oder mehrfach substituiert sein können, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F, -Cl, -Br, -NO₂, -CN, -Aryl, -Heteroaryl, -C₁₋₈-Aliphat-Aryl und -C₁₋₈-Aliphat-Heteroaryl (z.B. -Ethyl-4-Pyridyl).

Besonders bevorzugt ist (Hetero-)aryl ausgewählt aus der Gruppe bestehend aus:

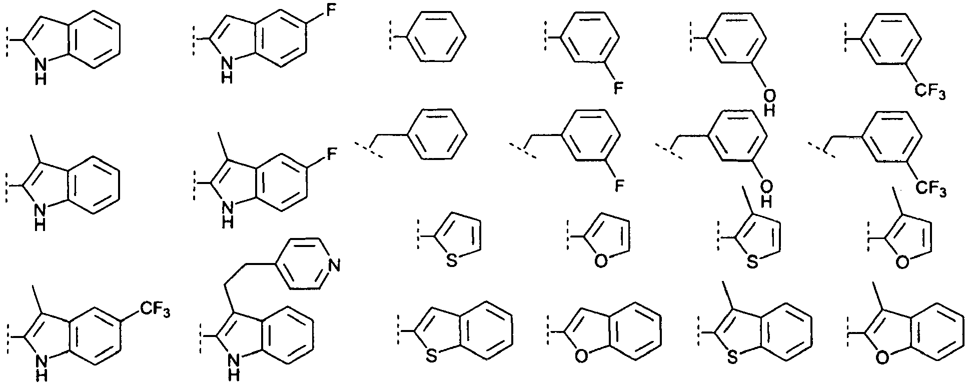

Bevorzugt steht Aryl jeweils unabhangig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)-NHR₁₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -Si(R₀)₃, -PO(OR₀)₂, -C₁₋₈Aliphat-NHC(=O)R₀, -C₁₋₈Aliphat-NHC(=O)OR₀, -C₁₋₈Aliphat-NHC(=O)NHR₀, -C₁₋₈Aliphat-NHC(=O)N(R₀)₂ und -C₁₋₈Aliphat-NHS(=O)₁₋₂R₀. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R_{O}, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NHC(=O)N(R₀)₂, -Si(R₀)₃, -PO(OR₀)₂, -C₁₋₈Aliphat-NHC(=O)R₀, -C₁₋₈Aliphat-NHC(=O)OR₀, - C₁₋₈Aliphat-NHC(=O)NHR₀, -C₁₋₈Aliphat-NHC(=O)N(R₀)₂ und -C₁₋₈Aliphat-NHS(=O)₁₋₂R₀.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀, -C(=O)N(R₀)₂, -S(=O)₁₋₂R₀, -S(=O)₂NH₂, -SO₃H, =O oder -R₀. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen kann es sich je nach Substitution in Bezug auf den Cyclohexanring um Isomere handeln, bei denen das Substitutionsmuster in 1,4-Position (1-Position: >C(NR₁R)R₃; 4-Position: >C((Hetero-)aryl)Q) auch mit syn/anti bezeichnet werden kann. "Syn/anti-Isomere" sind eine Untergruppe der Stereoisomere (Konfigurationsisomere).

In einer bevorzugten Ausführungsform beträgt der Diastereomerenüberschuss des syn-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des anti-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Sind die efindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure. Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, a-Liponsäure, Acetylglycin, Acetyisalicylsäure, Hippursäure undloderAsparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kallum-, Magnesium- oder Calcium-Salze.

Nachfolgend werden jeweils bevorzugte Ausführungsformen der erfindungsgemäßen Verbindungen erläutert. Sofern nicht ausdrücklich spezifiziert gelten alle jeweils zuvor erläuterten Definitionen der Substituenten (d.h. z.B. von R₀ bis R₄, Y₁ bis Y₄', etc.) und deren jeweilige bevorzugte Ausführungsformen entsprechend und werden daher nicht wiederholt.

Ausführungsformen der erfindungsgemäßen Verbindungen haben die allgemeine Formel (3) 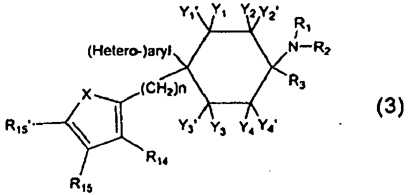 wobei
X für -O-, -S-, -NR₁₆-, -CR₁₇=CR₁₈-, -CR₁₇=N- oder -N=CR₁₈- steht; vorzugsweise für -O-, -S-. -NR₁₆- oder -CR₁₇=CR₁₈;
R₁₄, R₁₅, R₁₅'_{,} R₁₆, R₁₇ und R₁₈ jeweils unabhängig voneinander für -H, -F, -Cl, -Br. -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂,-C₁₋₈Aliphat-NHC(=O)R₀, -C₁₋₈Aliphat-NHC(=O)OR₀, -C₁₋₈Aliphat-NHC(=O)-NHR₀, -C₁₋₈Aliphat-NHC(=O)N(R₀)₂ oder -C₁₋₈Aliphat-NHS(=O)₁₋₂R₀ stehen; oder R₁₄ und R₁₅, oder R₀ und R₁₅', oder R₁₅' und R₁₇ zusammen einen fünf- oder sechsgliedrigen, gesättigten, partiell ungesättigten oder aromatischen, unsubstituierten oder ein- oder mehrfach substituierten Ring bilden, welcher ggf. ein oder zwei Heteroringatome umfasst unabhängig voneinander ausgewählt aus N, S und O; und
n für 0, 1 oder 2 steht; bevorzugt für 0 oder 1; bevorzugter für 0 (steht n für 0, so ergibt sich eine Bindung).

Steht beispielsweise n für 2 und X beispielsweise für -CR₁₇=CR₁₈-, -CR₁₇=N- oder -N=CR₁₈-, so ergeben sich vorzugsweise die folgenden funktionellen Gruppen:

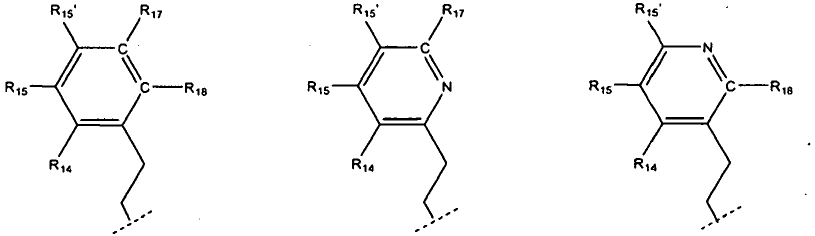

Bilden R₁₅ und R₁₅' zusammen beispielsweise einen sechsgliedrigen aromatischen Ring, welcher keine Heteroringatome aufweist, so ergeben sich jeweils die folgenden funktionellen Gruppen:

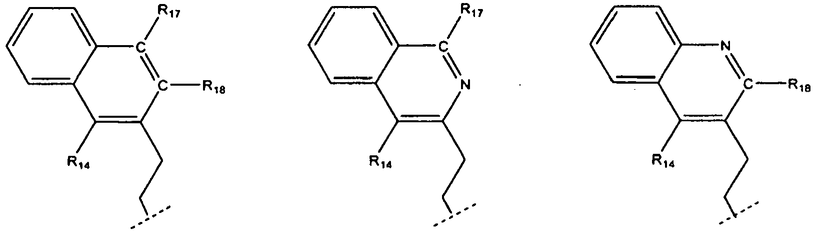

Der ggf. durch R₁₄ und R₁₅, oder R₁₅ und R₁₅', oder R₁₅' und R₁₇ zusammen gebildete fünf- oder sechsgliedrige, gesättigte, partiell ungesättigte oder aromatische Ring kann ein oder zwei Heteroringatome umfassen, welche unabhängig voneinander ausgewählt sind aus N, S und O. Ferner kann dieser gebildete Ring unsubstituiert oder ein- oder mehrfach substituiert sein, wobei die Substituenten vorzugsweise unabhängig voneinander ausgewählt sind aus der Gruppe bestehend aus -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀. -NHC(=O)OR₀, -NH-C(=O)NH₂, -NHC(=O)NHR₀, -NHC(=O)N(R₀)₂, -C₁₋₈Aliphat-NHC(=O)R₀, -C₁₋₈Aliphat-NHC(=O)OR₀, -C₁₋₈Aliphat-NHC(=O)NHR₀, -C₁₋₈Aliphat-NHC(=O)N(R₀)₂ und -C₁₋₈Aliphat-NHS(=O)₁₋₂R₀; bevorzugter -F, -Cl, -Br, -I, -CF₃, -CN und -NO₂.

Bevorzugt steht R₁₄ für -H, -F, -Cl oder -R₀; bevorzugter für -H, -F, -C₁₋₈-Aliphat, -C₁₋₈-Aliphat-Aryl oder -C₁₋₈-Aliphat-Heteroaryl.

Bevorzugt bilden R₁₅ und R₁₅' zusammen einen sechsgliedrigen, gesättigten, partiell ungesättigten oder aromatischen Ring bilden, welcher ggf. ein oder zwei Heteroringatome umfassen kann, die unabhängig voneinander ausgewählt sind aus N, S und O. Dieser gebildete Ring kann unsubstituiert oder ein- oder mehrfach substituiert sein, wobei die Substituenten vorzugsweise unabhängig voneinander ausgewählt sind aus der Gruppe bestehend aus -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀ und -NHC(=O)N(R₀)₂; bevorzugter -F, -Cl, -Br, -I, -CF₃, -CN und -NO₂.

R₁₆, R₁₇ und R₁₈ sind vorzugsweise unabhängig voneinander ausgewählt aus der Gruppe bestehend aus -H, -F, -Cl, -CN, -OH, -R₀ und -OR₀. Besonders bevorzugt sind R₁₆, R₁₇ und R₁₈ - soweit vorhanden - jeweils -H.

Bevorzugte Ausführungsformen der erfindungsgemäßen Verbindungen der allgemeinen Formel (3) haben die allgemeine Formel (3.1) oder (3.2):

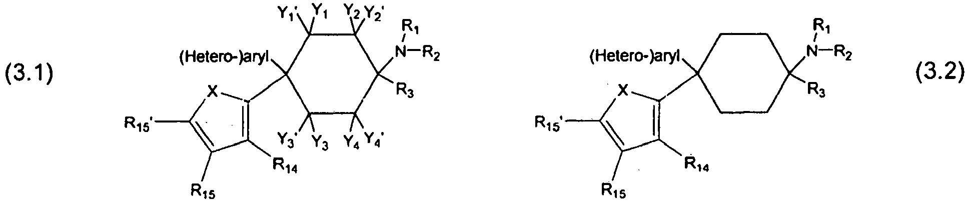

Besonders bevorzugte Ausführungsformen der erfindungsgemäßen Verbindungen der allgemeinen Formeln (3.1) und (3.2) haben die allgemeine Formel (3.1.1), (3.1.2), (3.1.3), (3.2.1), (3.2.2) oder (3.2.3): 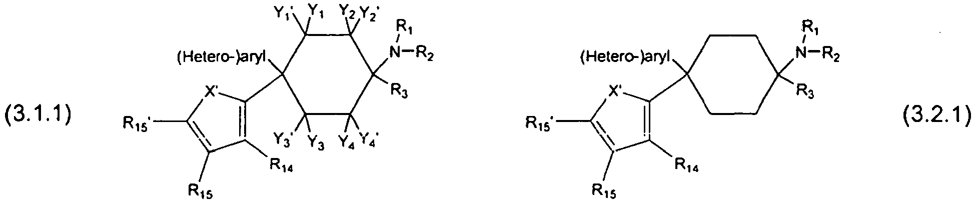 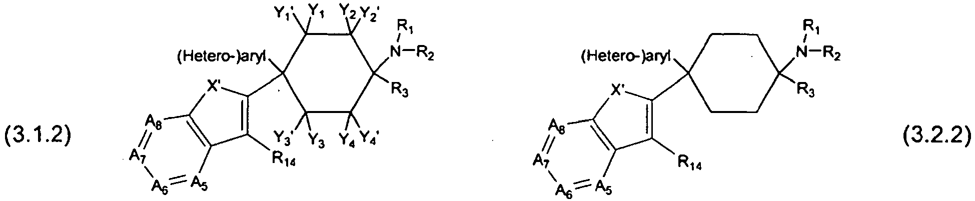 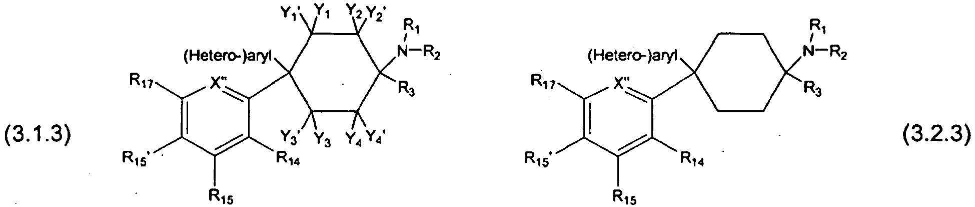 wobei
X' für -NR₁₆-, -O-, oder -S- steht; bevorzugt für -NR₁₆-; und
X" für -N= oder -CR₁₈= steht; bevorzugt für -CR₁₈-; und
A₅ für -N= oder -CR₁₉ steht;
A₆ für -N= oder -CR₂₀ steht;
A₇ für -N= oder -CR₂₁ steht;
A₈ für -N= oder -CR₂₂ steht;
mit der Maßgabe, dass höchstens zwei der Reste A₅, A₆, A₇ und A₈, vorzugsweise 0, 1 oder 2 der Reste A₅, A₆, A₇ und A₈, -N= sind.
R₁₉, R₂₀, R₂₁ und R₂₂ jeweils unabhängig voneinander für -H, -F, -Cl, -Br, -I, -NO₂, -CF₃, -OR₂₇, -SR₂₇, -SO₂R₂₇, -CN, -COOR₂₇, -CONR₂₇, -NR₂₈R₂₉, =O oder -R₀ stehen; vorzugsweise für -F, -Cl, -Br, -I, -CF₃, -CN oder -NO₂;
R₂₇ jeweils unabhängig für -H oder -R₀ steht;
R₂₈ und R₂₉ unabhängig voneinander für -H oder -R₀ stehen; oder R₁₄ und R,₅ zusammen für -CH₂CH₂OCH₂CH₂-, -CH₂CH₂NR₃₀CH₂CH₂- oder -(CH₂)₃₋₆- stehen;
R₃₀ für -H oder -C₁₋₆-Aliphat steht.

Besonders bevorzugte Ausführungsformen der Verbindungen der allgemeinen Formel (3) haben die allgemeine Formel (4):

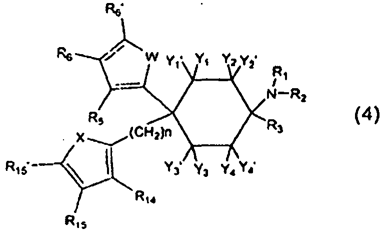

Bevorzugte Ausführungsformen der erfindungsgemäßen Verbindungen der allgemeinen Formel (4) haben die allgemeine Formel (4.1), (4.2), (4.3). (4.4), (4.5), (4.6), (4.7) oder (4.8):

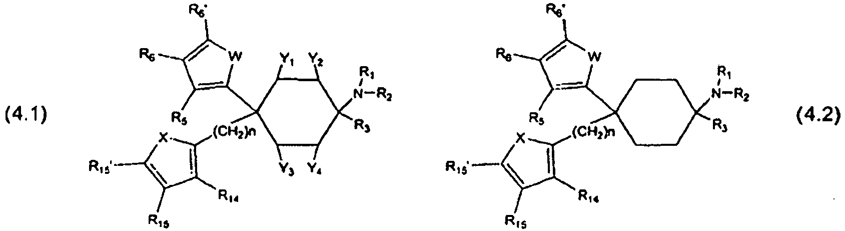

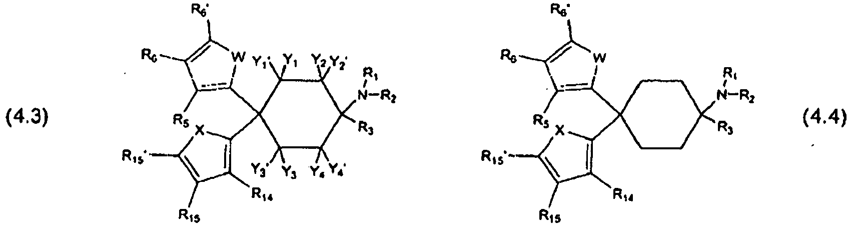

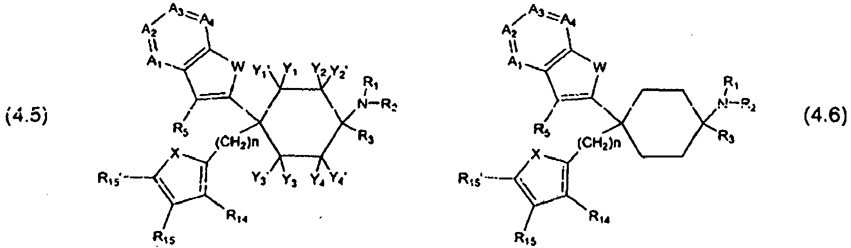

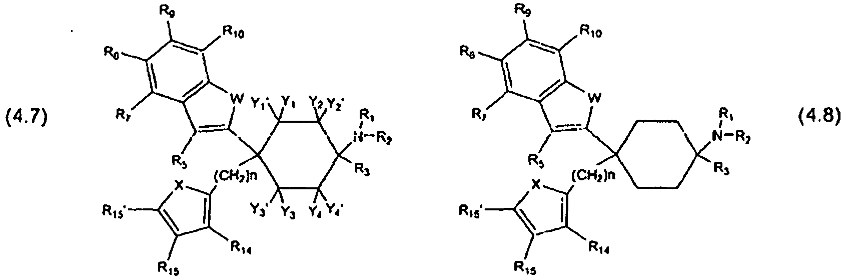

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁₋₈-Aliphat (Substituent der 1. Generation), so kann -C₁. ₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis R₃₀ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis R₃₀ jeweils nicht substituiert sein.

Erfindungsgemäß besonders bevorzugte Ausführungsformen der Verbindungen der allgemeinen Formel (4.8) sind Verbindungen der allgemeinen Formel (4.8.1) 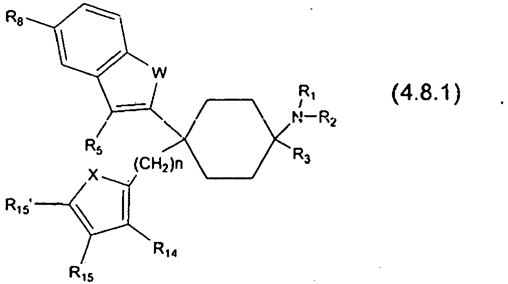 wobei
W für -0- oder -NR₁₁- steht; X für -O-, -NR₁₆- oder -CR₁₇=CR₁₈- steht;
n für 0 oder 1 steht;
R₁ für -CH₃ steht;
R₂ für -H oder -CH₃ steht; oder
R₁ und R₂ gemeinsam einen Ring bilden und für -(CH₂)₃₋₄- stehen;
R₃ für -C₁₋₈-Aliphat, -Aryl oder Heteroaryl steht; bevorzugt -C₁₋₈-Alkyl, -Phenyl, Thienyl, Furfyl oder Pyrrolyl; wobei diese unsubstituiert oder ein- oder mehrfach substituiert sind, bevorzugt mit Substituenten unabhängig voneinander ausgewählt aus der Gruppe bestehend aus -F, -Cl, -Br, -CN, -CH₃, -C₂H₅, -NH₂, -NO₂, -SH, -CF₃, -OH, -OCH₃, -OC₂H₅ und -N(CH₃)₂;
R₅ und R₁₄ unabhängig voneinander für -H, -F, -C₁₋₈-Aliphat, -C₁₋₈-Aliphat-Aryl, -C₁₋₈-Aliphat-Heteroaryl, -C₁₋₈Aliphat-NHC(=O)R₀, -C₁₋₈Aliphat-NHC(=O)OR₀, -C₁₋₈Aiphat-NHC(=O)NHR₀, -C₁₋₈Aliphat-NHC(=O)N(R₀)₂ oder -C₁₋₈Aliphat-NHS=O)₁₋₂R₀ stehen;
R₈ für -F, -Cl, -Br, -I, -CF₃, -CN oder -NO₂ steht;
R₁₁ für -H steht;
R₁₄, R₁₅ und R₁₅' unabhängig voneinander für -H, -F, -Cl, -Br, -CN, -CH₃, -C₂H₅, -NH₂, -NO₂, -SH, -CF₃, -OH, -OCH₃, -OC₂H₅ oder -N(CH₃)₂ stehen; oder R₁₅ und R₁₅' gemeinsam einen sechsgliedrigen, gesättigten, partiell ungesättigten oder aromatischen Ring bilden, welcher ggf. ein oder zwei Heteroringatome umfassen kann, die unabhängig voneinander ausgewählt sind aus N, S und O; wobei dieser gebildete Ring unsubstituiert oder ein- oder mehrfach substituiert sein kann, wobei die Substituenten unabhängig voneinander ausgewählt sind aus der Gruppe bestehend aus -F, -Cl, -Br, -I, -CF₃, -CN und -NO₂.
R₁₆ für -H steht; und
R₁₇ und R₁₈ unabhängig voneinander für -H oder -F stehen.

Ganz besonders bevorzugt sind Verbindungen aus der Gruppe:
- 1-Butyl-N,N-dimethyl-4,4-bis(3-methyl-5-(trifluormethyl)-1H-indol-2-yl)cyclohexanamin; 2-hydroxypropan-1,2,3-tricarboxylat;
- 1-Butyl-4,4-bis(5-fluor-3-methyl-1H-indol-2-yl)-N,N-dimethylcyclohexanamin;
- 1-Benzyl-N,N-dimethyl-4,4-bis(3-(2-(pyridin-4-yl)ethyl)-1H-indol-2-yl)cyclohexanamin; 2-Hydroxypropan-1,2,3-tricarboxylat;
- N,N-Dimethyl-4,4-bis(3-(2-(pyridin-4-yl)ethyl)-1H-indol-2-yl)-1-(thiophen-2-yl)cyclohexanamin;
- 2,2'-(4-Butyl-4-(pyrrolidin-1-yl)cyclohexan-1,1-diyl)bis(3-(2-(pyridin-4-yl)ethyl)-1H-indol);
- N-Methyl-1-phenyl-4,4-bis(3-(2-(pyridin-4-yl)ethyl)-1 H-indol-2-yl)cyclohexanamin;
- N,N-Dimethyl-4-(3-methyl-1H-indol-2-yl)-1-phenyl-4-(thiophen-2-yl)cyclohexanamin; 2-Hydroxypropan-1,2,3-tricarboxylat;
- N,N-Dimethyl-4-(3-methyl-1 H-indol-2-yl)-1-phenyl-4-(thiophen-2-yl)cyclohexanamin;
- N,N-Dimethyl-4-(3-methyl-1H-indol-2-yl)-1,4-diphenylcyclohexanamin; 2-Hydroxypropan-1,2,3-tricarboxylat;
- N,N-Dimethyl-4,4-bis(3-methylbenzofuran-2-yl)-1-phenylcyclohexanamin; 2-Hydroxypropan-1,2,3-tricarboxylat;
- N,N-Dimethyl-4,4-bis(3-methyl-1H-indol-2-yl)-1-phenylcyclohexanamin; 2-hydroxypropan-1,2,3-tricarboxylat;
- 1-Butyl-N,N-dimethyl-4,4-bis(3-(2-(pyridin-4-yl)ethyl)-1H-indol-2-yl)cyclohexanamin; 2-hydroxypropan-1,2,3-tricarboxylat;
- N,N,4-Trimethyl-4-(3-methyl-1H-indol-2-yl)-1-phenylcyclohexanamin;
- 4-Benzyl-N,N-dimethyl-4-(3-methyl-1H-indol-2-yl)-1-phenylcyclohexanamin;
- Dimethyl 2,2'-(2,2'-(4-butyl-4-(dimethylamino)cyclohexan-1,1-diyl)bis(1H-indol-3,2-diyl))diacetat
- 2,2'-(2,2'-(4-Bbutyl-4-(dimethylamino)cyclohexan-1,1-diyl)bis(1H-indol-3,2-diyl))diethanol
- 1-Butyl-N,N-dimethyl-4-(3-methyl-1H-indol-2-yl)-4-(3-(2-pyridin-4-yl)ethyl)-1H-indol-2-yl)cyclohexylamin
- 1-Butyl-4,4-bis-(3-(2-(3,4-dihydro-1 H-isochinolin-2-yl)ethyl)-1 H-indol-2-yl)-N,N-dimethylcyclohexylamin
- 4-(4-Methoxyphenyl)-N,N-dimethyl-4-(3-methyl-1H-indol-2-yl)-1-phenylcyclohexylamin
- 1-Butyl-N, N-dimethyl-4,4-bis-(3-methyl-1 H-indol-2-yl)cyclohexylamin
- 2-(2-(2-(4-Butyl-4-dimethylamino-1-(3-methyl-1H-indol-2-yl)cyclohexyl)-1H-indol-3-yl)ethyl)isoindolin-1,3-dion
- 2-[2-[2-[4-Butyl-4-dimethylamino-1-[3-[2-(1,3-dioxo-2H-isoindol-2-yl)-ethyl]-1H-indol-2-yl]-cyclohexyl]-1H-indol-3-yl]-ethyl]-2H-isoindole-1,3-dion
- 4-(Benzo[d][1,3]dioxol-5-yl)-N,N-dimethyl-4-(3-methyl-1H-indol-2-yl)-1-phenylcyclohexylamin
- Dimethyl 3,3'-(2-(4-butyl-4-(dimethylamino)cyclohexan-1,1-diyl)bis(1H-indol-2,3-diyl)dipropanat
- 4-(3-(2-Aminoethyl)-1H-indol-2-yl)-1-butyl-N,N-dimethyl-4-(3-methyl-1H-indol-2-yl)cyclohexylamin
- 1-(3-Fluorphenyl)- N,N-dimethyl-4-(3-methyl-1H-indol-2-yl)-4-thiophen-2-ylcyclohexylamin
- 1-Butyl-4,4-bis-(1,3-dimethyl-1H-indol-2-yl)- N,N-dimethylcyclohexylamin
- 1-Butyl-4-(1,3-dimethyl-1H-indol-2-yl)-N,N-dimethyl-4-(3-methyl-1H-indol-2-yl)cyclohexylamin
- 1-Benzyl-N,N-dimethyl-4-(3-methyl-1H-indol-2-yl)-4-(thiophen-2-yl)cyclohexylamin
- 4-(3-Methoxyphenyl)-N,N-dimethyl-4-(3-methyl-1H-indol-2-yl)-1-phenylcyclohexylamin
- N-(2-(2-(4-Butyl-4-(dimethylamino)-1-(3-methyl-1H-indol-2-yl)cyclohexyl)-1H-indol-3-yl)ethyl)cyclopentansuffonsäureamid
- 1-(3-Fluorphenyl)- N,N-dimethyl-4-(3-methyl-1 H-indol-2-yl)-4-thiophen-2-ylcyclohexylamin
- N,N-Dimethyl-1-phenyl-4,4-bis-(3-(2-pyridin-4-yl)ethyl)-1H-indol-2-yl)cyclohexylamin
- 1-(2-(2-(4-Butyl-4-(dimethylamino)-1-(3-methyl-1H-indol-2-yl)cyclohexyl)-1H-indol-3-yl)ethyl)-3-phenylharnstoff
- 1,1'-(2,2'-(2,2'-(4-Butyl-4(dimethylamino)cyclohexan-1,1-diyl)bis(1H-indol-3,2-diyl))bis(ethan-2,1-diyl)bis(3-phenylharnstoff)
- 1-Butyl-4,4-bis(5-fluor-3-methyl-1H-indol-2-yl)-N,N-dimethylcyclohexylamin
- 4-(3-(2-Aminoethyl)-1H-indol-2-yl)-1-butyl-4-(5-fluor-3-methyl-1H-indol-2-yl)-N,N-cyclohexylamin
- (Phenyl-2-(2-(4-butyl-4-(dimethylamino)-1-(5-fluor-3-methyl-1 H-indol-2-yl)cyclohexyl)-1 H-indol-3-yl)ethylcarbamat
- 1-(2-(2-(4-Butyl-4-(dimethylamino)-1-(5-fluor-3-methyl-1H-indol-2-yl)cyclohexyl)-1H-indol-3-yl)ethyl)-3-phenylharstoff
- N,N-Dimethyl-4-(3-methyl-1 H-indol-2-yl)-1-phenyl-4-(3-(2-pyridin-4-yl)ethyl)-1H-indol-2-yl)cyclohexylamin
- N,N-Dimethyl-1-phenyl-4-(3-(2-(pyridin-4-yl)-ethyl)-1H-indol-2-yl)-4-(thiophen-2-yl)-cyclohexylamin
und deren physiologisch verträgliche Salze und/oder Solvate.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Verbindungen weisen eine vergleichbare Affinität zum ORL1-Rezeptor auf wie die Verbindungen, die als Beispielverbindungen in WO 2004043967 offenbart sind. Im Vergleich zu diesen Verbindungen zeigen sie jedoch eine höhere Selektivität gegenüber dem kappa-Opioid-Rezeptor, welcher für Nebenwirkungen, wie beispielsweise Dysphorie, Sedation und Diurese verantwortlich ist. Darüber hinaus zeigen die erfindungsgemäßen Verbindungen bei günstigem Verhältnis der ORL1/µ-Affinität eine ausgewogene Affinität an den µ-Opioid-Rezeptor, welche nicht zu strak ist. Dies ist von Vorteil, da der µ-Opioid-Rezeptor mit Nebenwirkungen in Verbindung gebracht wird, insbesondere Atemdepression, Obstipation und Suchtabhängigkeit. Daher eignen sich die erfindungsgemäßen Verbindungen besonders für die Arzneimittelentwicklung.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus. Migräne. Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nichtäquimolare oder äquimolare Mischung der Diastereomere und/oder Enantiomere vorliegt.

Ein weiterer Gegenstand der Erfindung betrifft eine erfindungsgemäße Verbindung zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung betrifft ein Verfahren zur Herstellung der erfindungsgemäßen Verbindungen wie in der folgenden Beschreibung und Beispielen ausgeführt.

### Allgemeines Syntheseschema 1:

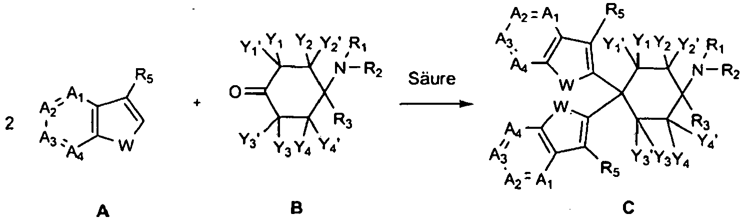

Verbindungen der allgemeinen Formel A können unter Einwirkung einer organischen Säure oder deren Trimethylsilylester oder einer anorganischen Säure oder unter Zusatz eines Übergangsmetallsalzes mit Verbindungen der allgemeinen Formel B zu Verbindungen der allgemeinen Formel C umgesetzt werden. Verbindungen der allgemeinen Formel A sind kommerziell erhältlich (exemplarische Synthesen siehe auch WO2008009416). Die Synthesen der Cycloxexanonderivate mit der allgemeinen Formel B sind in der Literatur bekannt (WO05066183, WO040043967, WO0290317, US 4065573, Lednicer et al., J. Med. Chem., 23, 1980, 424-430.)

### Allgemeines Syntheseschema 2:

Für Q= Aryl, Hetaryl

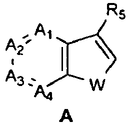

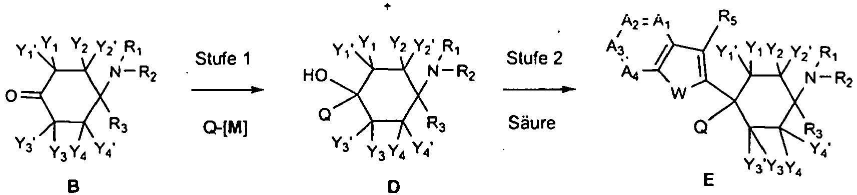

In Stufe 1 werden Metallorganylverbindungen vom Typ Q-[M] (mit z.B. [M]=Li oder [M]= MgX] im Sinne einer 1,2-Addition an Cyclohexanone der allgemeinen Formel B zu Verbindungen vom Typ D umgesezt. Verbindungen der allgemeinen Formel D können unter Einwirkung einer organischen Säure oder deren Trimethylsilylester oder einer anorganischen Säure oder unter Zusatz eines Übergangsmetallsalzes mit Verbindungen der allgemeinen Formel A zu Verbindungen der allgemeinen Formel E umgesetzt werden (Stufe 2).

### Allgemeines Syntheseschema 3.1:

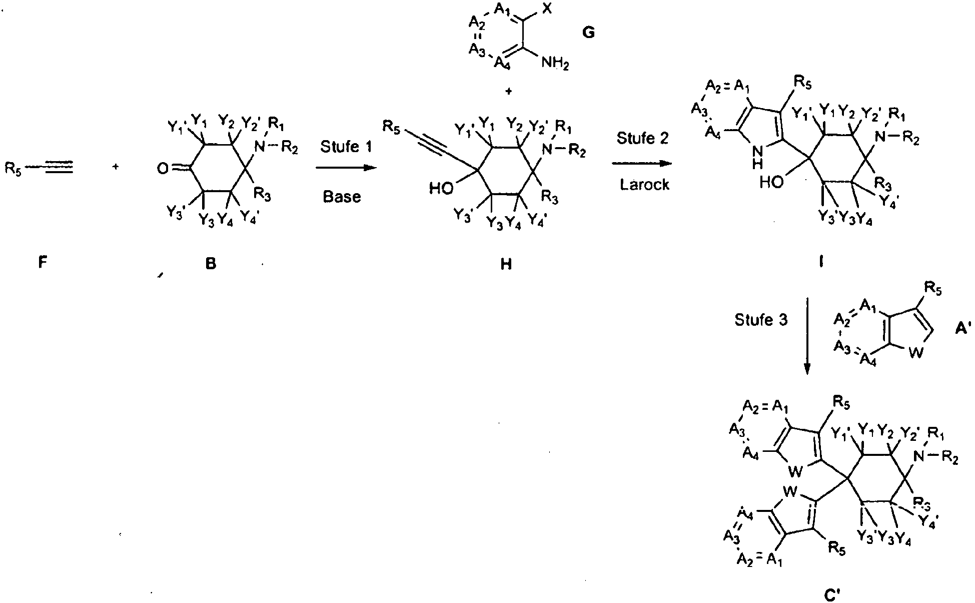

Die Umsetzung von F und B zu Verbindungen der allgemeinen Formel H kann unter Einsatz geeigneter basischer Reagenzien erfolgen (Stufe 1). In Stufe 2 werden Verbindungen der allgemeinen Formel G, worin X für einen Halogenrest oder einen Sulfonsäureester steht im Sinne einer Indolsynthese nach Larock unter Zusatz eines Palladiumkatalysators mit Alkinen der allgemeinen Formel H zu Indolen der allgemeinen Formel I umgesetzt. Verbindungen der allgemeinen Formel G sind kommerziell erhältlich (exemplarische Synthesen siehe auch WO2008009416). Die Umsetzung von I mit Verbindungen der allgemeinen Formel A' zu Verbindungen der allgemeinen Formel C' kann unter Einwirkung einer organischen Säure oder deren Trimethylsilylester oder einer anorganischen Säure oder unter Zusatz eines Übergangsmetallsalzes erfolgen (Stufe 3).

### Allgemeines Syntheseschema 3.2:

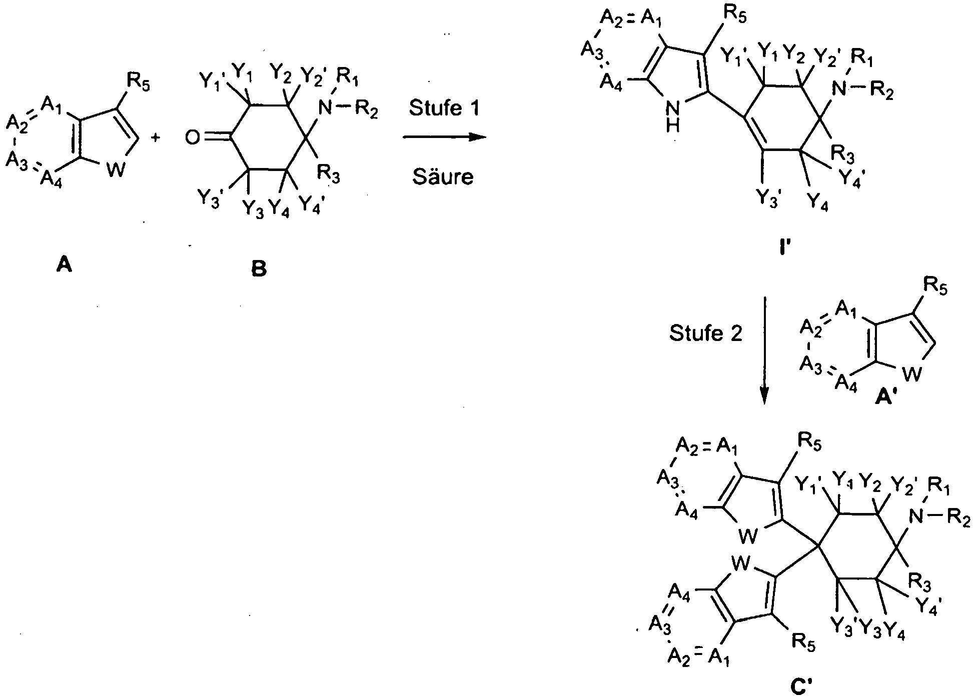

Verbindungen der allgemeinen Formel A können unter Einwirkung einer organischen Säure oder deren Trimethylsilylester oder einer anorganischen Säure oder unter Zusatz eines Übergangsmetallsalzes mit Verbindungen der allgemeinen Formel B zu Verbindungen der allgemeinen Formel I' umgesetzt werden. Verbindungen der allgemeinen Formel A sind kommerziell erhältlich (exemplarische Synthesen siehe auch WO2008009416). Die Synthesen der Cycloxexanonderivate mit der allgemeinen Formel B sind in der Literatur bekannt (WO05066183, WO040043967, WO0290317, US 4065573, Lednicer et al., J. Med. Chem., 23, 1980, 424-430.) Die Umsetzung von I' mit Verbindungen der allgemeinen Formel A' zu Verbindungen der allgemeinen Formel C' kann unter Einwirkung einer organischen Säure oder deren Trimethylsilylester oder einer anorganischen Säure oder unter Zusatz eines Übergangsmetallsalzes erfolgen (Stufe 2).

### Allgemeines Syntheseschema 4:

### Für Q= -C1-8-(Cyclo)aliphat, -C1-8-Aliphat-(Hetero)aryl

### Allgemeines Syntheseschema 4.1:

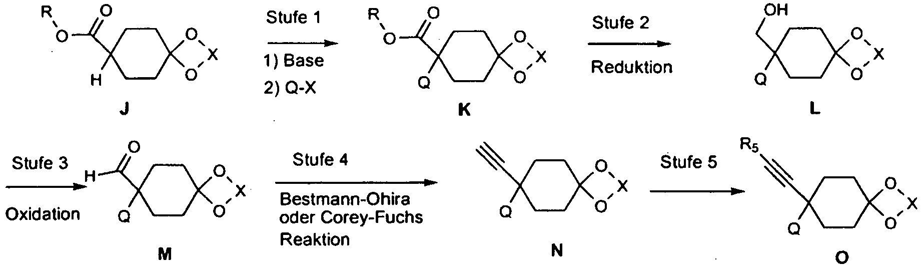

In Stufe 1 werden Verbindungen der Formel J mit (X aus der Gruppe Alkyl, Alkyl/ Alkyliden/ mit Aryl oder Alkyl (gesättigt/ungesättigt) substituiertem Alkyliden) durch Deprotonierung mit einer geeigneten Base und Umsetzung mit Elektrophilen vom Typ Q-X zu Verbindungen der allgemeinen Formel K umgesetzt. In Stufe 2 erfolgt die Reduktion des Esters. Die erhaltenen Alkohole vom Typ L lassen sich durch Oxidation zu Aldehyden M umsetzen (Stufe 3). In Stufe 4 werden Aldehyde der allgemeinen Formel M im Sinne einer Bestmann-Ohira Reaktion (Regitz, M. et al.; Chem. Ber., 1968, 101;3734-3743; Harned, A. M. et al. Tetrahedron, 2005, 61, 12093-12099) bzw. gemäß einer Corey-Fuchs Reaktion (Corey; Fuchs; THL 1972, 36, 3769-3772) zu Alkinen der allgemeinen Formel N umgesetzt. Die Einführung des Restes R₅ zu Verbindungen der allgemeinen Formel O erfolgt in Stufe 5 entweder durch Deprotonierung/ Alkylierung (R₅-X) oder durch eine Übergangsmetallvermittelte Kupplung im Sinne einer Sonogashira-Reaktion.

### Allgemeines Syntheseschema 4.2:

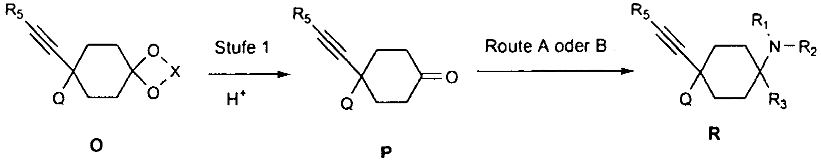

Verbindungen der Formel P können aus entsprechenden Acetalen O, oder aus deren Salzen, nach dem Fachmann bekannten Methoden z. B. durch Entschützen mittels Säuren freigesetzt werden. Dabei ist X aus der Gruppe Alkyl, Alkyl/ Alkyliden/ mit Aryl oder Alkyl (gesättigt/ungesättigt) substituiertem Alkyliden ausgewählt. Verbindungen der Formel P können zu Alkinen vom Typ R umgesetzt werden. Dies kann beispielsweise nach den folgenden zwei Routen erfolgen: (1) Route A: *Aminonitril- oder Triazol- Route und* (2) Route B: *Imin-Route* (s.u.).

### Allgemeines Syntheseschema 4.3:

### (1) Route A: Aminonitril- oder Triazol- Route

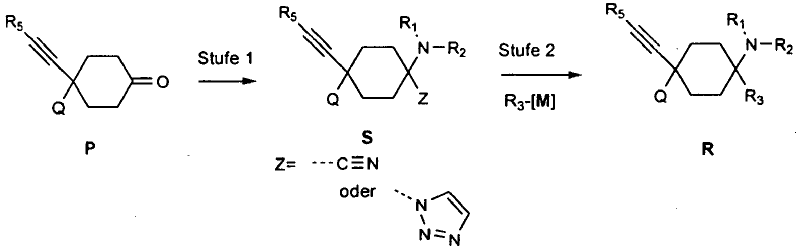

Ketone P lassen sich durch Reaktion mit Aminen und aciden Reaktanden Z-H zu Strukturen der Formel S umsetzen. Geeignete Reaktanden Z-H sind z. B. Cyanwasserstoff, 1,2,3-Triazol, Benzotriazol oder Pyrazol. Ein besonders bevorzugter Weg zu Verbindungen der Struktur S (mit Z= -CN) ist die Umsetzung von Ketonen P mit Metallcyaniden und dem entsprechenden Amin in Gegenwart von Säure. Ein weiterer besonders bevorzugter Weg zu Verbindungen der Struktur S ist die Umsetzung von Ketonen mit 1,2,3-Triazol und dem entsprechenden Amin in Gegenwart unter wasserentziehenden Bedingungen.

Aminonitrile der Formel S (mit Z= -CN) lassen sich zu Verbindungen R durch Reaktion mit entsprechenden Organometallverbindungen (R₃-[M]), vorzugsweise Grignardverbindungen umsetzen. Die Organometallverbindungen sind entweder käuflich erhältlich oder können nach bekannten Methoden hergestellt werden.

Aminotriazole der Formel S (mit Z= -Triazol) lassen sich zu Verbindungen R durch Reaktion mit entsprechenden Organometallverbindungen (R₃-[M]), vorzugsweise

Grignardverbindungen umsetzen. Die Organometallverbindungen sind entweder käuflich erhältlich oder können nach bekannten Methoden hergestellt werden.

Die Bedingungen können den angegebenen Literaturstellen entnommen werden: (a) Katritzky et al. Synthesis, 1992, 1295-1298. (b) Prashad, et al., Tetrahedron Lett. 2005, 46, 5455-5458.

### Allgemeines Syntheseschema 4.4:

### (2) Route B: Imin- Route

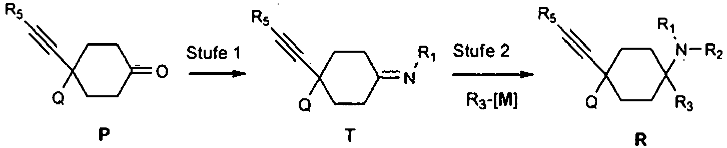

Aus dem Ketonvorläufer P wird das Imin T synthetisiert, welches unter Verwendung eines Nukleophils (R₃-[M]) in den Bausteine R überführt wird. Die benötigten Iminbausteine T können nach einer dem Fachmann bekannten Methode hergestellt werden (Layer, Chem. Rev., 1963, 8, 489-510). Für die Addition der Organometallspezies R₃-[M] an das Imin T siehe: z.B. Maddox et al., J. Med. Chem., 1965, 8, 230-235. Kudzma et al., J. Med. Chem., 1989, 32, 2534-2542.

### Allgemeines Syntheseschema 4.5:

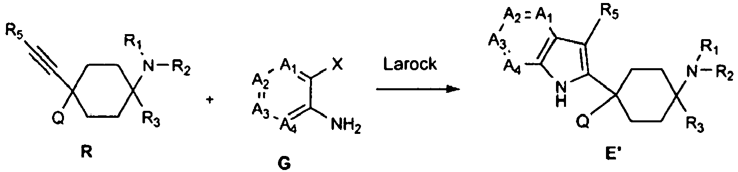

Verbindungen der allgemeinen Formel G, worin X für einen Halogenrest oder einen Sulfonsäureester steht, können im Sinne einer Indolsynthese nach Larock unter Zusatz eines Palladiumkatalysators mit Alkinen der allgemeinen Formel R zu Indolen der allgemeinen Formel E' umgesetzt werden. Verbindungen der allgemeinen Formel G sind kommerziell erhältlich oder oder ihre Herstellung ist aus dem Stand der Technik bekannt oder in für den Fachmann offensichtlicher Weise aus dem Stand der Technik ableitbar (exemplarische Synthesen siehe auch WO2008009416).

Bezüglich weiterer Einzelheiten zur Synthese der erfindungsgemäßen Verbindungen kann vollumfänglich verweisen werden auf WO2002/090317, WO2002/90330, WO2003/008370, WO2003/008731, WO2003/080557, WO2004/043899, WO2004/043900, WO2004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

### Beispiele

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### Beispiel Nr. 1

### Stufe 1

### 1-Butyl-N,N-dimethyl-4,4-bis(3-methyl-5-(trifluormethyl)-1H-indol-2-yl)cyclohexanamin

4-Butyl-4-(dimethylamino)cyclohexanon (395 mg, 2 mmol, Synthese vgl. WO2008009415, Ketonbaustein Ket-4) wurde zusammen mit 3-Methyl-5-trifluormethyl-1 H-indol (398 mg, 2 mmol, Synthese vgl. WO2008009415, Indolbaustein Ind-7) in Dichlormethan (20 ml) gelöst. Anschließend erfolgte die Zugabe von Trifluormethansulfonsäure (0,2 ml, 338 mg, 2,25 mmol). Es wurde 3 d bei RT gerührt. Zur Aufarbeitung wurde das Reaktionsgemisch mit 1 N NaOH (10 ml) versetzt und 10 min gerührt. Nach Phasentrennung wurde die wässrige Phase mit Dichlormethan (2 × 10 ml) extrahiert. Die vereinigten organischen Phasen wurden mit Natriumsulfat getrocknet, filtriert und im Vakuum eingeengt. Es wurden 741 mg hellbrauner Feststoff erhalten, der chromatographisch getrennt wurde [Kieselgel 60 (80 g); Ethylacetat/Methanol (15 : 1; 1,5 l); (10 : 1; 500 ml); (1 : 1; 500 ml)].
Ausbeute: 103 mg (9 %)
¹³C NMR (101 MHz, CDCl3) 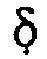 ppm: 8.9, 10.1, 14.0, 23.7, 26.6, 29.6, 29.9, 37.3, 42.4, 56.1, 107.6, 109.6, 110.6, 110.9, 115.7, 115.8, 116.0, 116.1, 117.9, 118.5, 121.0, 121.3, 121.5, 121.6, 121.8, 121.9, 122.1, 122.4, 124.1, 124.2, 126.9, 129.6, 129.8, 135.3, 135.9, 138.0, 140.5

### Stufe 2

### 1-Butyl-N,N-dimethyl-4,4-bis(3-methyl-5-(trifluormethyl)-1H-indol-2-yl)cyclohexanamin 2-Hydroxypropan-1,2,3-tricarboxylat (1:1) (Beispiel Nr. 1)

1-Butyl-N,N-dimethyl-4,4-bis(3-methyl-5-(trifluormethyl)-1 H-indol-2-yl)cyclohexanamin (100 mg, 0,17 mmol) wurde in Ethanol (10 ml) gelöst und mit Citronensäure (36 mg, 0,187 mmol), gelöst in heißem Ethanol (3 ml), versetzt. Die klare farblose Lösung wurde 48 h gerührt und anschließend eingeengt. Nach Zugabe von Diethylether (10 ml) wurde 2 h bei RT gerührt und dann der Niederschlag abgesaugt.
Ausbeute: 68 mg (53 %)
Schmelzpunkt: 177-179 °C

### Beispiel Nr. 2

### Stufe1:

### 1-Butyl-4,4-bis(5-fluor-3-methyl-1H-indol-2-yl)-N,N-dimethylcyclohexanamin

5-Fluor-3-methylindol (596 mg, 4 mmol) wurde zusammen mit 4-Butyl-4-(dimethylamino)-cyclohexanon (788 mg, 4 mmol, Synthese vgl.WO2008009415, Ketonbaustein Ket-4) in Dichlormethan (30 ml) gelöst und mit Trifluormethansulfonsäure (400 µl, 4,6 mmol) versetzt. Der Ansatz wurde 24 h bei RT gerührt. Zur Aufarbeitung wurde das Reaktionsgemisch mit 2N NaOH (30 ml) versetzt und 20 min bei RT gerührt. Nach Abtrennung der organischen Phase wurde die wässrige Phase mit Dichlormethan (3 x 15 ml) extrahiert. Die vereinigten organischen Extrakte wurden über Na₂SO₄ getrocknet und anschließend eingeengt. Das erhaltene Rohprodukt (1,4 g) wurde durch Säulenchromatographie [Kieselgel 60 (50 g); Ethylacetat (500 ml)] gereinigt.
Ausbeute: 400 mg (31 %), weißer Feststoff

### Stufe2:

### 1-Butyl-4,4-bis(5-fluor-3-methyl-1H-indol-2-yl)-N,N-dimethylcyclohexanamin; Hydrochlorid (1:1) (Beispiel Nr. 2)

1-Butyl-4,4-bis(5-fluor-3-methyl-1H-indol-2-yl)-N,N-dimethylcyclohexanamin (400 mg, 0,84 mmol) wurde in Ethylmethylketon (50 ml) gelöst. Bei RT wurde dann Me₃SiCl (214 µl, 1,68 mmol) zugetropft und 1 h gerührt. Es fiel ein weißer Niederschlag aus. Der Niederschlag wurde abgesaugt, mit Ethylmethylketon (2 × 5 ml) gewaschen und anschließend getrocknet.
Ausbeute: 323 mg (75 %), weißer Feststoff
Schmelzpunkt: 262-304 °C
¹³C NMR (101 MHz, DMSO-D6) δ ppm: 8.3, 9.1, 13.7, 22.5, 25.4, 26.9, 29.8, 30.2, 37.3, 41.1, 65.9, 102.2, 102.4, 106.3, 106.4, 106.7, 106.8, 108.3, 108.4, 108.6, 108.7, 111.4, 111.5, 111.7, 111.8, 129.4, 129.5, 129.6, 129.7, 131.3, 131.4, 138.6, 139.1, 155.5, 155.6, 157.8, 157.9

### Beispiel Nr. 3

### Stufe1:

### 1-Benzyl-N,N-dimethyl-4,4-bis(3-(2-(pyridin-4-yl)ethyl)-1 H-indol-2-yl)cyclohexanamin

3-(2-Pyridin-4-ylethyl)-1H-indol (667 mg, 3 mmol, Synthese vgl. WO2008009415, Indolbaustein Ind-14) wurde zusammen mit 4-Benzyl-4-(dimethylamino)cyclohexanon (652 mg, 3 mmol, Synthese vgl. WO2008009415, Ketonbaustein Ket-3) in abs. Dichlormethan (45 ml) gelöst und mit Trifluormethansulfonsäure (0,553 ml, 6,3 mmol) versetzt. Der Ansatz wurde 67 h bei RT gerührt, wobei ein braunes Öl ausfiel. Zur Aufarbeitung wurde die Reaktionslösung mit 1N NaOH (10 ml) und THF (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über Na2SO4 getrocknet und anschließend eingeengt. Das erhaltene Rohprodukt (1,22 g) wurde säulenchromatographisch gereinigt [Kieselgel 60 (100 g); Ethylacetat/Methanol (10 : 1, 1100 ml), Ethylacetat/Methanol (2 : 1, 500 ml), Ethylacetat/Methanol (1 : 2, 750 ml)].
Ausbeute: 152 mg
Schmelzpunkt: 314-317 °C
¹³C-NMR (101 MHz, DMSO-d6) δ ppm: 14.0, 26.3, 26.9, 29.4, 30.4, 34.5, 34.9, 37.0, 39.8, 56.9, 108.9, 109.2, 111.2, 111.4, 117.4, 117.5, 118.2, 118.3, 119.9, 120.0, 123.6, 123.7, 125.4, 127.5, 128.3, 128.6, 130.4, 134.3, 134.6, 138.7, 139.2, 142.1, 149.0, 149.1, 150.7

### Stufe 2:

### 1-Benzyl-N,N-dimethyl-4,4-bis(3-(2-(pyridin-4-yl)ethyl)-1H-indol-2-yl)cyclohexanamin; 2-Hydroxypropan-1,2,3-tricarboxylat (2:3) (Beispiel Nr. 3)

1-Benzyl-N,N-dimethyl-4,4-bis(3-(2-(pyridin-4-yl)ethyl)-1H-indol-2-yl)cyclohexanamin (128 mg, 0,182 mmol) und Citronensäure (36 mg, 0,187 mmol) wurden in Methanol (30 ml) gelöst. Die klare Lösung wurde im Vakuum eingeengt und der Rückstand in Ethanol (5 ml) gelöst. Zur Lösung wurde bei RT langsam Ethylacetat (10 ml) und Diethylether (15 ml) zugetropft. Das Citrat fiel als weißer Feststoff aus. Das Gemisch wurde 1 h bei RT gerührt, dann filtriert und mit Diethylether gewaschen.
Ausbeute: 94 mg (60 %), weißer Festoff
Schmelzpunkt: 155-165 °C

### Beispiel Nr. 4

### N,N-Dimethyl-4,4-bis(3-(2-(pyridin-4-yl)ethyl)-1H-indol-2-yl)-1-(thiophen-2-yl)cyclohexanamin (Beispiel Nr. 4)

3-(2-Pyridin-4-ylethyl)-1H-indol (667 mg, 3 mmol, Synthese vgl. WO2008009415, Indolbaustein Ind-14) wurde zusammen mit 4-(Dimethylamino)-4-(thiophen-2-yl)cyclohexanon (671 mg, 3 mmol, Synthese vgl. WO2008009415, Ketonbaustein Ket-12) in abs. Dichlormethan (45 ml) gelöst und mit Trifluormethansulfonsäure (0,660 ml, 7,43 mmol) versetzt. Der Ansatz wurde 64 h bei RT gerührt, wobei ein braunes Öl ausfiel. Zur Aufarbeitung wurde die Reaktionslösung mit 1N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über Na₂SO₄ getrocknet und anschließend eingeengt. Das erhaltene Rohprodukt (1,33 g) wurde säulenchromatographisch gereinigt [Kieselgel 60 (100 g); Ethylacetat/Methanol 10 : 1 (1650 ml), Ethylacetat/Methanol 5 : 1 (600 ml)].
Ausbeute: 99 mg, gelber Feststoff
Schmelzpunkt: 277-282 °C ¹³C-NMR (101 MHz, DMSO d6) δ ppm: 26.5, 26.6, 31.5, 32.8, 34.8, 34.9, 37.8, 38.9, 58.5, 109.4, 109.5, 111.4, 117.6, 118.4, 120.2, 123.7, 124.7, 126.3, 128.5, 128.6, 134.5, 134.6, 149.06, 149.1, 150.6.

### Beispiel Nr. 5

### 2,2'-(4-Butyl-4-(pyrrolidin-1-yl)cyclohexan-1,1-diyl)bis(3-(2-(pyridin-4-yl)ethyl)-1 H-indol) (Beispiel Nr. 5)

3-(2-Pyridin-4-ylethyl)-1 H-indol (667 mg, 3 mmol, Synthese vgl. WO2008009415, Indolbaustein Ind-14) wurde zusammen mit 4-Butyl-4-(pyrrolidin-1-yl)cyclohexanon (671 mg, 3 mmol, Synthese vgl. WO2008009415, Ketonbaustein Ket-14) in abs. Dichlormethan (45 ml) gelöst und mit Trifluormethansulfonsäure (0,613 ml, 6,9 mmol) versetzt. Der Ansatz wurde 64 h bei RT gerührt, wobei ein braunes Öl ausfiel. Zur Aufarbeitung wurde die Reaktionslösung mit 1N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über Na₂SO₄ getrocknet und anschließend eingeengt. Das erhaltene Rohprodukt (1,35 g) wurde säulenchromatographisch gereinigt [Kieselgel 60 (90 g); Ethylacetat/Methanol 1 : 1 (2400 ml)].
Ausbeute: 48 mg, beigefarbener Feststoff
Schmelzpunkt: 277-285 °C
¹³C-NMR (101 MHz, CDCl3) δ ppm: 14.1, 23.7, 24.6, 26.4, 26.6, 26.9, 27.0, 30.8, 31.3, 32.1, 35.4, 35.9, 41.5, 44.1, 54.8, 110.1, 110.9, 111.2, 111.9, 118.1, 118.4, 119.5, 119.8, 121.5, 121.8, 123.7, 129.2, 129.4, 134.1, 134.7, 137.7, 140.4, 149.5, 149.6, 150.9, 151.1.

### Beispiel Nr. 6

### N-Methyl-1-phenyl-4,4-bis(3-(2-(pyridin-4-yl)ethyl)-1H-indol-2-yl)cyclohexanamin (Beispiel Nr. 6)

3-(2-Pyridin-4-ylethyl)-1H-indol (667 mg, 3 mmol, Synthese vgl. WO2008009415, Indolbaustein Ind-14) wurde zusammen mit 4-(Methylamino)-4-phenylcyclohexanon (610 mg, 3 mmol, Synthese vgl. WO2008009415, Ketonbaustein Ket-15) in abs. Dichlormethan (45 ml) gelöst und mit Trifluormethansulfonsäure (0,613 ml, 6,9 mmol) versetzt. Der Ansatz wurde 64 h bei RT gerührt, wobei ein braunes Öl ausfiel. Zur Aufarbeitung wurde die Reaktionslösung mit 1 N NaOH (30 ml) und Methanol (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über Na₂SO₄ getrocknet und anschließend eingeengt. Das erhaltene Rohprodukt (1,47 g) wurde säulenchromatographisch gereinigt [Kieselgel 60 (90 g); Ethylacetat/Methanol 5 : 1 (1200 ml), Ethylacetat/Methanol 1 : 1 (1200 ml)].
Ausbeute: 100 mg, gelber Feststoff
Schmelzpunkt: 115-120 °C
¹³C-NMR (101 MHz, CDCl3) δ ppm: 26.7, 26.8, 26.9, 27.4, 28.8, 31.8, 32.7, 35.5, 35.7, 41.6, 56.8, 110.6, 110.8, 111.06, 111.1, 111.4, 118.2, 118.3, 119.6, 119.7, 121.7, 121.8, 123.7, 124.0, 126.3, 126.7, 127.1, 128.2, 128.3, 128.4, 129.1, 129.3, 134.4, 134.5, 137.6, 139.1, 149.48, 149.53, 150.9, 151.1

### Beispiel Nr. 7 und Bespiel Nr. 8

### Stufe 1:

### 4-(Dimethylamino)-4-phenyl-1-(thiophen-2-yl)cyclohexanol (polares und unpolares Diastereomer)

4-(Dimethylamino)-4-phenylcyclohexanon (2,17 g, 10 mmol, Synthese vgl. WO2008009415, Ketonbaustein Ket-10) wurde in absolutem THF (30 ml) vorgelegt und innerhalb von 10 min mit 2-Thienylmagnesiumbromid-Lösung (1 M in THF, 22,5 ml, 22,5 mmol) versetzt. Die Reaktionslösung wurde 2 h unter Rückfluss zum Sieden erhitzt. - Zur Aufarbeitung wurde die Lösung unter Eisbadkühlung vorsichtig mit Eisstücken und gesättigter NH₄Cl-Lösung (25 ml) versetzt. Anschließend wurde mit Ethylacetat (3 x 30 ml) extrahiert. Die vereinigten organischen Phasen wurden mit Wasser (20 ml) und gesättigter NaCl-Lösung (20 ml) gewaschen und über Natriumsulfat getrocknet. Anschließend wurden die flüchtigen Bestandteile vollständig im Vakuum entfernt. - Die chromatographische Trennung des Substanzgemisches (3,78 g) an Kieselgel 60 (150 g) erfolgte mit Ethylacetat/Methanol (1 : 1). Ausbeute (unpolares Diastereomer): 430 mg (14%), beigefarbener Feststoff
¹³C NMR (101 MHz, DMSO-D6) δ ppm: 28.7, 35.3, 37.8, 58.2, 69.8, 121.4, 123.2, 126.2, 126.4, 126.6, 127.2, 139.0, 156.4
Ausbeute (polares Diastereomer): 980 mg (33%), beigefarbener Feststoff
Schmelzpunkt: 136-141 °C
¹³C NMR (101 MHz, DMSO-D6) δ ppm: 28.3, 36.5, 37.9, 60.4, 69.5, 121.3, 123.3, 126.2, 126.3, 127.5, 127.6, 136.5, 155.8

### Stufe 2:

### N,N-Dimethyl-4-(3-methyl-1H-indol-2-yl)-1-phenyl-4-(thiophen-2-yl)cyclohexanamin (unpolares Diastereomer)

Skatol (430 mg, 3,28 mmol) wurde unter Feuchtigkeitsausschluss zusammen mit 4-(Dimethylamino)-4-phenyl-1-(thiophen-2-yl)cyclohexanol (350 mg, 1,16 mmol, polareres Diastereoisomer) in trockenem Dichlormethan (40 ml) vorgelegt und schnell mit Trifluormethansulfonsäure-trimethylsilylester (0,23 ml, 1,27 mmol) versetzt. Der Ansatz wurde bei RT 24 h gerührt. - Zur Aufarbeitung wurde der ausgefallene Niederschlag mittels einer Fritte abgetrennt und getrocknet. Dieser wurde mit Dichlormethan (10 ml) und 2N NaOH (2 ml) versetzt und die Suspension 2,5 Tage gerührt. Die organische Phase wurde abgetrennt und die wässrige Phase mit Dichlormethan (3 x 20 ml) extrahiert. Die vereinigten organischen Phasen wurden über MgSO₄ getrocknet und anschließend eingeengt.
Ausbeute (unpolares Diastereomer) 110 mg (22 %), hellgelber Feststoff
Schmelzpunkt: ab 215 °C
13C NMR (101 MHz, CDCl3) 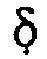 ppm: 10.1, 28.5, 34.2, 37.2, 42.4, 68.4, 105.8, 110.8, 117.7, 118.2, 119.1, 120.9, 121.9, 123.6, 126.4, 128.9, 129.1, 129.6, 129.7, 129.8, 135.2, 135.5, 152.9.

### Stufe 3:

### N,N-Dimethyl-4-(3-methyl-1H-indol-2-yl)-1-phenyl-4-(thiophen-2-yl)cyclohexanamin; 2-Hydroxypropan-1,2,3-tricarboxylat (1:1) (Beispiel Nr. 7, unpolares Diastereomer)

N,N-Dimethyl-4-(3-methyl-1H-indol-2-yl)-1-phenyl-4-(thiophen-2-yl)cyclohexanamin (90 mg, 0,22 mmol, unpolares Diastereomer) wurde in Propan-2-ol (20 ml) in der Siedehitze gelöst und mit einer heißen Lösung von Citronensäure [60 mg, 0,31 mmol, in Propan-2-ol (2 ml)] versetzt. Nach Konzentrieren des Lösungsmittels auf 1-2 ml fiel ein Niederschlag aus. Der Ansatz wurde zur Vervollständigung der Fällung 18 h bei 5 °C belassen, dann wurde der Feststoff mittels einer Fritte abgetrennt und getrocknet.
Ausbeute: 55 mg (41 %), glasiger Feststoff
1 H NMR (300 MHz, CDCl3) 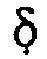 ppm: 1.55-1.78 (br s, 2H), 1.79-1.99 (br s 3H), 2.05-2.29 (br s, 6H), 2.3-2.67 (m, 7H), 2.94-3.02 (m, 3H), 6.57 (s, 1 H), 6.70-7.81 (m, 11 H), 10.77 (s, 1 H)

### Beispiel Nr. 8

### N,N-Dimethyl-4-(3-methyl-1H-indol-2-yl)-1-phenyl-4-(thiophen-2-yl)cyclohexanamin (Beispiel Nr. 8, polares Diastereomer)

Die in Stufe 2 nach Abtrennung des Niederschlags erhaltene Mutterlauge wurde mit 2N NaOH (10 ml) versetzt und 10 min gerührt. Anschließend wurde die organische Phase abgetrennt und die wässrige Phase mit Dichlormethan (4 x 20 mL) extrahiert. Die vereinigten organischen Phasen wurden über MgSO4 getrocknet und anschließend eingeengt und der Rückstand (670 mg) durch chromatographische Reinigung [Kieselgel 60 G (10 g); Ethylacetat (100 ml)] gereinigt.
Ausbeute: 252 mg (52 %)
Schmelzpunkt: 202-205 °C (aus Methanol)
13C NMR (101 MHz, CDCl3) 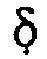 ppm: 9.6, 30.2, 33.6, 38.0, 43.4, 60.6*, 106.0*, 110.2, 117.9, 118.9, 121.2, 124.3*, 126.7, 126.8, 127.6*, 127.8*, 130.3, 133.7
* verbreiterte Signale.

### Beispiel Nr. 9

### Stufe 1:

### 4-(Dimethylamino)-1,4-diphenylcyclohexanol (polares und unpolares Diastereomer)

Zu einer Lösung von 4-(Dimethylamino)-4-phenylcyclohexanon (2,17 g, 10 mmol, Synthese vgl. WO2008009415, Ketonbaustein Ket-10) in abs. THF (20 ml) wurde eine Lösung von Phenylmagnesiumbromid (20 ml, 1 M in THF, 20 mmol) innerhalb von 10 min gegeben. Dabei erwärmte sich der Ansatz auf ca. 40 °C. Nach beendeter Zugabe wurde der Ansatz 2 h zum Sieden erhitzt. Nach ca. 1 h fiel ein Niederschlag aus. - Zur Aufarbeitung wurde der Ansatz mit Eis gekühlt und nach Erreichen von ca. 5 °C mit Eisstücken (ca. 2 g) versetzt. Anschließend wurde zu der Mischung gesättigte NH4CI-Lösung (20 ml) gegeben. Die organische Phase wurde abgetrennt, die wässrige Phase wurde mit Ethylacetat (3 x 20 ml) extrahiert. Die vereinigten organischen Extrakte wurden über MgSO₄ getrocknet und anschließend eingeengt. Der erhaltene Rückstand (3,1 g) wurde chromatographisch gereinigt [Kieselgel 60 G (10 g); Ethylacetat (100 ml), Ethylacetat/Ethanol 1 : 1 (100 ml)].
Ausbeute (unpolares Diastereoisomer): 405 mg (13%), farbloser Feststoff
Schmelzpunkt: 114-115 °C (aus Methanol)
13C NMR (101 MHz, CDCl3) 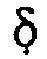 ppm: 29.2, 34.5, 38.1, 58.7, 72.9, 124.8, 126.4, 126.7. 126.8, 127.4, 128.2, 139.34, 148.9
Ausbeute (polares Diastereoisomer): 881 mg (26%), farbloser Feststoff
Schmelzpunkt: 123-126 °C (aus Methanol)
13C NMR (101 MHz, CDCl3) 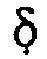 ppm: 28.6, 35.8, 38.2, 61.5, 72.3, 124.5, 126.7, 127.9, 128.0, 128.1, 135.8, 148.5

### Stufe 2:

### N,N-Dimethyl-4-(3-methyl-1H-indol-2-yl)-1,4-diphenylcyclohexanamin (ein Diastereomer)

Skatol (393 mg, 3 mmol) wurde zusammen mit dem 4-(Dimethylamino)-1,4-diphenylcyclo-hexanol (590 mg, 2 mmol, polares Diastereomer) in Dichlormethan (40 ml) vorgelegt und schnell mit Trifluormethansulfonsäure-trimethylsilylester (0,6 ml, 3,31 mmol) versetzt. Der Ansatz wurde bei RT 43 h gerührt. - Zur Aufarbeitung wurde der Ansatz mit 2N NaOH (20 ml) versetzt und 10 min gerührt. Anschließend wurde die organische Phase abgetrennt und die wässrige Phase mit Dichlormethan (3 x 20 ml) extrahiert. Die vereinigten organischen Phasen wurden über MgSO₄ getrocknet und anschließend eingeengt. Der erhaltene Rückstand (945 mg) wurde chromatographisch gereinigt [Kieselgel 60 G (10 g); Hexan/ Ethylacetat 1 : 1 (100 ml)]. Das erhaltene Amingemisch wurde in Dichlormethan (5 ml) gelöst und mit 2N HCl (5 ml) versetzt wurde. Es entstand ein unlöslicher Niederschlag (136 mg, 15 % Ausbeute, Schmelzpunkt: 303-307 °C), der mittels Filtration aus dem Phasengemisch abgetrennt wurde. Danach wurde der Feststoff mit Dichlormethan (10 ml) und 2N NaOH (10 ml) versetzt und gerührt. Nach 17 h wurde das entstandene klare Zweiphasensystem getrennt und die wässrige Phase mit Dichlormethan (3 x 20 ml) extrahiert. Die vereinigten organischen Phasen wurden über MgSO4 getrocknet und anschließend eingeengt.
Ausbeute: 14 % 114 mg (14 %)
Schmelzpunkt: 193-207 °C

### Stufe 3:

### N,N-Dimethyl-4-(3-methyl-1H-indol-2-yl)-1,4-diphenylcyclohexanamin; 2-Hydroxypropan-1,2,3-tricarboxylat (1:1) (Beispiel Nr. 9, ein Diastereomer)

N,N-Dimethyl-4-(3-methyl-1H-indol-2-yl)-1,4-diphenylcyclohexanamin (120 mg, 0,29 mmol) wurde in 2-Propanol (10 ml) in der Siedehitze gelöst und mit einer heißen Lösung von Citronensäure [70 mg, 0,36 mmol, in 2-Propanol (2 ml)] versetzt. Nach Aufkonzentration des Lösungsmittels auf ca. 5 ml fiel ein Niederschlag aus. Der Ansatz wurde zur Vervollständigung der Fällung 18 h bei 5 °C belassen, dann wurde der Feststoff mittels einer Fritte abgetrennt und getrocknet.
Ausbeute: 77 mg (44 %), glasiger Feststoff
13C NMR (101 MHz, DMSO-d6) 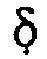 ppm: 10.0, 29.1*, 31.0, 32.4*, 37.3, 43.8*, 71.4, 105.5, 110.6, 117.4*, 118.1*, 120.5*, 125.3*, 125.9, 128.1*, 128.6, 129.0*, 129.7, 135.1, 147.3*, 171.1, 176.2*
* verbreiterte Signale.

### Beispiel Nr. 10

### Stufe 1:

### N,N-Dimethyl-4,4-bis(3-methylbenzofuran-2-yl)-1-phenylcyclohexanamin

3-Methylbenzofuran (354 mg, 3 mmol) wurde zusammen mit 4-(Dimethylamino)-4-phenyl-cyclohexanon (651 mg, 3 mmol, Synthese vgl. WO2008009415, Ketonbaustein Ket-10) in Dichlormethan (25 ml) gelöst und mit Trifluormethansulfonsäure (0,3 ml, 3,4 mmol) versetzt. Der Ansatz wurde 20 h bei RT gerührt. - Zur Aufarbeitung wurde das Reaktionsgemisch mit 2N NaOH (10 ml) versetzt. Das Gemisch wurde weitere 20 min gerührt. Nach Trennung der Phasen wurde die wässrige Phase mit Dichlormethan (3 x 20 ml) extrahiert. Die vereinigten organischen Extrakte wurden über MgSO₄ getrocknet und anschließend eingeengt. Das erhaltene Rohprodukt (950 mg) wurde säulenchromatographisch (Laufmittel: EtOAc) gereinigt und anschließend aus Ethanol (60 ml) umkristallisiert.
Ausbeute: 182 mg,
Schmelzpunkt: 164-166 °C

### Stufe 2:

### N,N-Dimethyl-4,4-bis(3-methylbenzofuran-2-yl)-1-phenylcyclohexanamin; 2-Hydroxypropan-1,2,3-tricarboxylat (1:1) (Beispiel Nr. 10)

N,N-Dimethyl-4,4-bis(3-methylbenzofuran-2-yl)-1-phenylcyclohexanamin (410 mg, 0,88 mmol) wurde in der Siedehitze in Ethanol (70 ml) gelöst und mit Citronensäure (300 mg, 1,6 mmol), gelöst in heißem Ethanol (10 ml), versetzt. Die Lösung wurde auf ca. 10 ml eingeengt und 3 h bei 5 °C aufbewahrt. Die entstandenen Kristalle wurden mittels einer Fritte abgetrennt.
Ausbeute: 349 mg (60 %)
Schmelzpunkt: 161-164 °C
13C NMR (101 MHz, DMSO-D6) 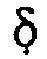 ppm: 7,4, 8.2, 28.6*, 29.9*, 37.3, 42.4, 43.6*, 71.7, 110.0, 110.6, 110.8, 111.4 119.0, 119.2, 122.3, 122.4, 123.9, 124.1, 128.5*, 130.2, 130.5, 152.0, 152.4, 171.2, 175.8
*verbreiterte Signale.

### Beispiel Nr. 11

### N,N-Dimethyl-4,4-bis(3-methyl-1 H-indol-2-yl)-1-phenylcyclohexanamin 2-Hydroxypropan-1,2,3-tricarboxylat (1:1) (Beispiel Nr. 11)

3-Methylindol (262 mg, 2 mmol) wurde zusammen mit 4-(Dimethylamino)-4-phenylcyclo-hexanon (434 mg, 2 mmol, Synthese vgl. WO2008009415, Ketonbaustein Ket-10) in Dichlormethan (20 ml) gelöst und mit Trifluormethansulfonsäure-trimethylsilylester (0,44 ml, 2,45 mmol) versetzt. Der Ansatz wurde 20 h bei RT gerührt. - Zur Aufarbeitung wurde das Reaktionsgemisch mit 2N NaOH (10 ml) versetzt. Nach Trennung der Phasen wurde die wässrige Phase mit Dichlormethan (2 x 20 ml) extrahiert. Die vereinigten organischen Extrakte wurden über Na₂SO₄ getrocknet und anschließend eingeengt. Das erhaltene Rohprodukt (638 mg) wurde säulenchromatographisch (Laufmittel: EtOAc) gereinigt. Der erhaltene Feststoff (200 mg) wurde in Ethanol (3 ml) gelöst und mit Citronensäure (48 mg, 0,25 mmol) versetzt. Die Lösung wurde 3 Tage bei RT belassen. Der Feststoff wurde mittels einer Fritte abgetrennt.
Ausbeute: 79 mg (14 %)
Schmelzpunkt: ab 168 °C.
1H NMR (400 MHz, DMSO-D6) 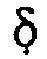 ppm: 1.78 (s, 3H), 1.93-2.15 (m, 6H), 2.26 (s, 6H), 2,45-2.70 (m, 6H) 2.83-2.95 (m, 2H), 4.14-4.67 (br s, 1H), 6.82-6.88 (m, 1 H), 6.89-6.99 (m, 2H), 7.02-7.08 (m, 1 H), 7.21-7.28 (m, 2H), 7.37-7.43 (m, 2H), 7.43-7.48 (m, 1 H), 7.48-7.56 (m, 2H), 7.60-7.70 (m, 2H),10.30 (s, 1H), 10.82 (s, 1H)

### Beispiel Nr. 12

### Stufe 1:

### 1-Butyl-N,N-dimethyl-4,4-bis(3-(2-(pyridin-4-yl)ethyl)-1H-indol-2-yl)cyclohexanamin

3-(2-Pyridin-4-ylethyl)-1 H-indol (667 mg, 3 mmol, Synthese vgl. WO2008009415, Indolbaustein Ind-14) wurde zusammen mit 4-Butyl-4-(dimethylamino)cyclohexanon (592 mg, 3 mmol, Synthese vgl. WO2008009415, Ketonbaustein Ket-4) in Dichlormethan (45 ml) gelöst und mit Trifluormethansulfonsäure (0,553 ml, 6,3 mmol) versetzt. Der Ansatz wurde 67 h bei RT gerührt, wobei ein braunes Öl ausfiel. Zur Aufarbeitung wurde die Reaktionslösung mit 1N NaOH (10 ml) und THF (10 ml) versetzt. Das Gemisch wurde weitere 60 min gerührt. Nach Trennung der Phasen wurde die wässrige Phase mit Dichlormethan (3 x 10 ml) extrahiert. Die vereinigten organischen Extrakte wurden über Na2SO4 getrocknet und anschließend eingeengt. Das erhaltene Rohprodukt (1,24 g) wurde säulenchromatographisch gereinigt [Kieselgel 60 (100 g); Ethylacetat/Methanol (10 : 1, 1200 1200 ml), Ethylacetat/Methanol (5 : 1, 600 ml), Ethylacetat/Methanol (2 : 1, 700 ml), Ethylacetat/ Methanol (1 : 2, 750 ml), Methanol (800 ml)].
Ausbeute: 121 mg,
Schmelzpunkt: 274-282 °C.
13C-NMR (101 MHz. CDCl₃) δ ppm: 14.0, 23.1, 26.0, 26.5, 26.8, 28.7, 30.7, 34.7, 35.0, 37.2, 55.5, 109.1, 109.6, 111.4, 111.5, 117.5, 117.6, 118.3, 118.4, 120.1, 123.7, 128.6, 134.5, 134.8, 139.6, 141.4, 149.1, 150.7

### Stufe 2:

### 1-Butyl-N,N-dimethyl-4,4-bis(3-(2-(pyridin-4-yl)ethyl)-1H-indol-2-yl)cyclohexanamin; 2-hydroxypropan-1,2,3-tricarboxylat (1:1) (Beispiel Nr. 12)

1-Butyl-N,N-dimethyl-4,4-bis(3-(2-(pyridin-4-yl)ethyl)-1H-indol-2-yl)cyclohexanamin (96 mg, 0,154 mmol) und Citronensäure (31 mg, 0,161 mmol) wurden in Methanol (4 ml) gelöst. Zur klaren Lösung wurde bei RT langsam Ethylacetat (4 ml) und Diethylether (16 ml) zugetropft. Es fiel ein weißes Pulver aus. Das Gemisch wurde 2 h bei RT gerührt, dann filtriert und mit Diethylether gewaschen.
Ausbeute: 100 mg (80 %), weißer Feststoff
Schmelzpunkt: 134-142 °C.

### Beispiel Nr. 13

### Stufe 1:

### Dimethyl 2,2'-(2,2'-(4-butyl-4-(dimethylamino)cyclohexan-1,1-diyl)bis(1H-indol-3,2-diyl))diacetat (Beispiel Nr. 13)

3-Indolylessigsäure-methyester (950 mg, 5,02 mmol) wurde zusammen mit dem 4-Butyl-4-(dimethylamino)cyclohexanon (395 mg, 2,00 mmol) in abs. Dichlormethan (20 ml) gelöst und mit Trifluormethansulfonsäure (0,230 ml, 2,62 mmol) versetzt. Der Ansatz wurde 111 h bei 23 °C gerührt. Zur Aufarbeitung wurde die Reaktionslösung mit 1N NaOH (10 ml) versetzt. Das Gemisch wurde 5 min gerührt. Nach Trennung der Phasen wurde die wäßrige Phase mit Dichlormethan (3 x 10 ml) extrahiert. Die vereinigten organischen Extrakte wurden über Na₂SO₄ getrocknet und anschließend eingeengt. Das erhaltene Rohprodukt (braunes Öl, 1,55 g) wurde säulenchromatographisch gereinigt [Kieselgel 60 (70 g); Ethylacetat (600 ml), Ethylacetat/Methanol 4 : 1 (1000 ml)]. Die gewünschte Bisindolverbindung wurde als weißer Feststoff (149 mg, 13 %, Schmp. 117-121 °C) erhalten.
Beispiel Nr. 13: ¹³C NMR (101 MHz, CDCl₃) δ ppm: 14.1, 23.8, 26.5, 29.7, 30.2, 30.9, 31.2, 31.4, 37.3, 44.3, 52.3, 52.4, 55.9, 101.4, 103.0, 110.8, 110.9, 117.7, 117.8, 118.9, 119.1, 121.6, 121.8, 129.0, 129.2, 135.1, 135-5, 139.5, 141.6, 174.8, 174.9
Olefin **4**: ¹³C NMR (101 MHz, CDCl₃) δ ppm: 14.2, 23.6, 25.6, 26.8, 28.5, 30.5, 32.2, 38.0, 51.9, 55.9, 104.3, 110.5, 118.7, 119.8, 122.0, 127.6, 128.4, 134.9, 137.4, 172.7

### Beispiel Nr. 14

### Stufe 1:

### 2,2'-(2,2'-(4-Bbutyl-4-(dimethylamino)cyclohexan-1,1-diyl)bis(1H-indol-3,2-diyl))diethanol (Beispiel Nr. 14)

Zu einer Suspension von Lithiumaluminiumhydrid (75 mg, 1,97 mmol) in abs. Tetrahydrofuran (10 ml) wurde unter Argon Dimethyl 2,2'-(2,2'-(4-butyl-4-(dimethylamino)cyclohexan-1,1-diyl)bis(1*H*-indol-3,2-diyl))diacetat (100 mg, 0,1791 mmol) hinzugefügt. Der Ansatz wurde 30 min bei 23 °C gerührt. Unter Kühlung wurde 1 N Natriumhydroxyd-Lösung (20 ml) zugegeben und 5 min bei Raumtemperatur gerührt. Die Mischung wurde mit Diethylether (3 × 15 ml) extrahiert. Die vereinigten organischen Phasen wurden mit Na₂SO₄ getrocknet, filtriert und die flüchtigen Bestandteile vollständig im Vakuum entfernt. Das Produkt wurde so in einer Ausbeute von 76 mg (90 %) als weißer Feststoff (Schmelzpunkt 174-178 °C) erhalten.
Beispiel Nr.14: ¹³C-NMR (101 MHz, CD₃OD) δ ppm: 14.4, 24.7, 27.5, 29.3, 29.5, 30.1, 32.1, 32.8, 37.9, 42.9, 58.2, 63.0, 63.1, 108.3, 108.5, 111.88, 111.94, 118.77, 118.80, 119.6, 119.7, 121.7, 121.8, 130.92, 130.94, 136.37, 136.41, 141.0, 141.4

### Beispiel Nr .15 und Beispiel Nr. 16

### Stufe 1:

### 1-Butyl-N,N-dimethyl-4-(3-(2-pyridin-4-yl)ethyl)-1H-indol-2-yl)cyclohex-3-enamin

3-[(2-Pyridin-4-yl)ethyl]-1*H*-indol (1,38 g, 6,21 mmol) wurde zusammen mit dem 4-Butyl-4-(dimethylamino)cyclohexanon (1,23 g, 6,23 mmol) mit HBr/Eisessig (33 % HBr, 6 ml) versetzt und 2 h bei 23 °C gerührt. Anschließend wurde das Gemisch mit Dichlormethan (100 ml) verdünnt. Unter Kühlung wurde 5N Natriumhydroxyd-Lösung (50 ml) zugegeben. Der Ansatz wurde 10 min bei Raumtemperatur gerührt. Nach Trennung der Phasen wurde die wäßrige Phase mit Dichlormethan (3 × 20 ml) extrahiert. Die vereinigten organischen Extrakte wurden über Na₂SO₄ getrocknet und anschließend eingeengt. Das erhaltene Rohprodukt (2,53 g, brauner Feststoff) wurde chromatographisch gereinigt [Kieselgel 60 (110 g); Ethylacetat/Methanol 5 : 1 (1200 ml), Methanol (1200 ml)]. Es wurde 1,53 g (61 %) der Zielverbindung (Schmelzpunkt: 146-150 °C) erhalten.
¹³C-NMR (101 MHz, CDCl₃) δ ppm: 14.2, 23.7, 25.6, 26.0, 26.9, 28.5, 30.5, 32.3, 36.4, 38-0, 55.9, 110.4, 110.6, 118.3. 119.4, 121.8, 124.4, 126.5, 128.7, 129.2, 135.1, 136.3, 149.5. 151.2

### Stufe 2:

### 1-Butyl-N,N-dimethyl-4-(3-methyl-1H-indol-2-yl)-4-(3-(2-pyridin-4-yl)ethyl)-1H-indol-2-yl)cyclohexylamin (Beispiel Nr. 15, unpolareres Diastereoisomer und Beispiel Nr. 16, polareres Diastereoisomer)

3-Methyl-1H-indol (400 mg, 3,05 mmol) wurde zusammen mit dem 1-Butyl-N,N-dimethyl-4-(3-(2-pyridin-4-yl)ethyl)-1H-indol-2-yl)cyclohex-3-enamin (605 mg, 1,51 mmol) in abs. Dichlormethan (20 ml) gelöst und mit Trifluormethansulfonsäure (0,32 ml, 3,64 mmol) versetzt. Der Ansatz wurde 112 h bei 23 °C gerührt. Zur Aufarbeitung wurde die Reaktionslösung mit 1N NaOH (20 ml) versetzt. Das Gemisch wurde 5 min gerührt. Nach der Trennung der Phasen wurde die wäßrige Phase mit Dichlormethan (3 x 15 ml) extrahiert. Die vereinigten organischen Extrakte wurden über Na₂SO₄ getrocknet und anschließend eingeengt. Das erhaltene Rohprodukt (braunes Öl, 1,12 g) wurde säulenchromatographisch gereinigt [Kieselgel 60 (60 g); Ethylacetat/Methanol 5 : 1 (1200 ml)]. Es wurden das unpolarere Diastereoisomer (136 mg, 17 %, Schmp. 193-198 °C) und das polarere Diastereoisomer (46 mg, 6 %, Schmp. 162-167 °C) als weiße Feststoffe gewonnen.
Beispiel Nr. 15: (unpolareres Diastereoisomer): ¹³C-NMR (101 MHz, CDCl₃) δ ppm: 10.4, 14.1, 23.7, 26.2, 26.6, 29.9, 30.5, 31.6, 36.0, 37.4, 42.3, 56.0, 108.7, 109.9, 110.8, 118.0, 118.3, 118.2, 119.4, 121.3, 121.7, 123.8, 129.5, 130.2, 134,0. 134.6, 136.3, 140.1, 149.4, 151.4
Beispiel Nr. 16: (polareres Diastereoisomer): ¹³C-NMR (101 MHz, CDCl₃) δ ppm: 9.3, 14.1, 23.7, 26.5, 27.0, 29.6, 30.7, 31.6, 35.4, 37.4, 41.7, 56.1, 107.0, 110.6, 111.0, 111.5, 118.0, 118.3, 119.2, 119.5, 121.3, 121.6, 123.7, 129.3, 130.4, 134.0, 134.5, 137.9, 138.8, 149.5, 151.0

### Beispiel Nr. 17

### Stufe 1:

### 2-[2-(1H-Indol-3-yl)-ethyl]-1,2,3,4-tetrahydroisochinolin

Eine Lösung von 3-(2-Brom-ethyl)-1*H*-indol (4.48 g, 20 mmol) und Isochinolin (5.33 g, 40 mmol) in abs. Dioxan (50 mL) wurde 6 h bei 80 °C gerührt. Anschließend wurde das Lösungsmittel i. Vak. entfernt, der Rückstand mit CHCl₃ (100 mL) versetzt und mit Wasser zweimal gewaschen. Die organische Phase wurde über Na₂SO₄ getrocknet, i. Vak. eingeengt und der verbliebene Rückstand durch Flash-Chromatographie mit CHCl₃/MeOH (50:1) gereinigt.
Ausbeute: 4.78 g (86 %), weißer Feststoff
¹H-NMR (DMSO-d₆): 2.76 (6 H, m); 2.95 (2 H, m); 3.66 (2 H, s); 7.06 (6 H, m); 7.18 (1 H, s); 7.34 (1 H, d); 7.56 (1 H, d); 10.77 (1 H, s).

### Stufe 2:

### 1-Butyl-4,4-bis-(3-(2-(3,4-dihydro-1H-isochinolin-2-yl)ethyl)-1H-indol-2-yl)-N,N-dimethylcyclohexylamin

Das 2-[2-(1*H*-Indol-3-yl)-ethyl]-1,2,3,4-tetrahydroisochinolin (850 mg, 3,07 mmol) wurde zusammen mit dem 4-Butyl-4-(dimethylamino)cyclohexanon (300 mg, 1,52 mmol) in abs. Dichlormethan (10 ml) gelöst und mit Trifluormethansulfonsäure (0,230 ml, 2,62 mmol) versetzt. Der Ansatz wurde 6 d bei 23 °C gerührt. Zur Aufarbeitung wurde die Reaktionslösung mit 1 N NaOH (10 ml) versetzt. Das Gemisch wurde 5 min gerührt. Nach der Trennung der Phasen wurde die wäßrige Phase mit Dichlormethan (3 x 10 ml) extrahiert. Die vereinigten organischen Extrakte wurden über Na₂SO₄ getrocknet und anschließend eingeengt. Das erhaltene Rohprodukt (braunes Öl, 1,24 g) wurde säulenchromatographisch gereinigt [Kieselgel 60 (60 g); Ethylacetat (240 ml)]. Die gewünschte Bisindolverbindung wurde als fast weißer Feststoff (89 mg, 8 %) erhalten, die laut NMR-Spektrum verunreinigt war.
Bisindolverbindung : ¹³C NMR (101 MHz, CDCl₃) δ ppm: 14.1, 22.6, 22.8, 23.8, 26.5, 28.4, 28.7, 29.5, 30.7, 31.6, 37.5, 43.1, 50.8, 51.0, 56.1, 57.0, 58.9, 59.4, 108.6, 110.0, 110.5, 110.6, 117.6, 117.8, 118.4, 118.6, 121.0, 121.2, 125.6, 125.8, 126.8, 128.9, 129.2, 129.3, 134.3, 134.6, 134.8, 135.0, 135.4, 138.3, 140.3

### Stufe 3:

### 1-Butyl-4,4-bis-(3-(2-(3,4-dihydro-1H-isochinolin-2-yl)ethyl)-1H-indol-2-yl)-N,N-dimethylcyclohexylamin Hydrochlorid (Beispiel Nr. 17)

Das 1-Butyl-4,4-bis-(3-(2-(3,4-dihydro-1*H*-isochinolin-2-yl)ethyl)-1*H*-indol-2-yl)-*N,N-*dimethylcyclohexylamin (57 mg, 0,079 mmol) wurde in Ethylmethylketon (2 ml) gelöst. Die Lösung wurde bei RT mit Chlortrimethylsilan (50 µl, 0,391 mmol) versetzt. Das klare Reaktionsgemisch wurde sofort trüb. Bei 23 °C wurde 30 min gerührt. Der weiße Niederschlag wurde abgesaugt. Der Feststoff wurde mit Ethylmethylketon (3 × 0,5 ml) gewaschen und dann getrocknet. Das Hydrochlorid (48 mg, ca. 80 %) wurde als hellbeigefarbener Feststoff erhalten.

### Beispiel Nr. 18

### Stufe 1:

### 4-Dimethylamino-1-(4-methoxyphenyl)-4-phenylcyclohexanol (unpolares Diastereoisomer und polares Diastereoisomer)

Das 4-(Dimethylamino)-4-phenylcyclohexanon (4,34 g, 20 mmol) wurde unter Ausschluß von Sauerstoff in absolutem Tetrahydrofuran (60 ml) gelöst, unter Eiskühlung mit 4-Methoxyphenylmagnesiumbromid-Lösung (90 ml, 45 mmol, 0,5N) versetzt und 2 h am Rückfluß gekocht. Zur Aufarbeitung wurde der Ansatz unter Eiskühlung mit gesättigter NH₄Cl-Lösung (50 ml) versetzt und 10 min bei Raumtemperatur gerührt. Die wäßrige Phase wurde abgetrennt und mit Ethylacetat (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (1000 ml), Methanol (1000 ml)]. Es entstanden 2,016 g (31 %, Smp. 174-175 °C) unpolareres und 2,51 g (39 %, Smp. 149-151 °C) polareres Produkt. Beide konnten aus Ethylacetat umkristallisiert werden.
unpolareres Diastereoisomer: ¹³C-NMR (101 MHz, DMSO-D₆) δ ppm: 28.7, 34.2, 37.8, 54.9, 58.0, 70.5, 113.0, 125.8, 126.1, 126.6, 127.2, 139.4, 142.7, 157.5
polareres Diastereoisomer: ¹³C-NMR (101 MHz, DMSO-D₆) δ ppm: 28.1, 35.5, 37.9, 54.9, 60.7, 70.2, 113.0, 125.5, 126.1, 127.5, 127.8, 136.6, 142.1, 157.5

### Stufe 2:

### 4-(4-Methoxyphenyl)- N,N-dimethyl-4-(3-methyl-1H-indol-2-yl)-1-phenylcyclohexylamin (Beispiel Nr. 18, Gemisch aus zwei Diastereoisomeren)

3-Methyl-1H-indol (393 mg, 3 mmol) und das unpolarere 4-Dimethylamino-1-(4-methoxyphenyl)-4-phenylcyclohexanol (651 mg, 2 mmol) wurden unter Ausschluß von Sauerstoff in absolutem Dichlormethan (50 ml) gelöst, mit Trifluormethansulfonsäure-trimethylsilylester (581 µl, 3 mmol) versetzt und 16 h bei Raumtemperatur gerührt. Zur Aufarbeitung wurde der Ansatz mit 5N NaOH (50 ml) versetzt und 1 h bei Raumtemperatur gerührt. Die wäßrige Phase wurde abgetrennt und mit Dichlormethan (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (1000 ml)]. Das Produkt wurde in einer Ausbeute von 600 mg (68 %) mit einem Schmelzpunkt von 90-95 °C als weißer Feststoff erhalten . Es handelte sich dabei um ein Gemisch aus zwei Diastereoisomeren (ca. 1 : 1), die wegen des gleichen R_{f}-Werts nicht getrennt werden konnten.
Beispiel Nr. 18: Diastereoisomerengemisch: ¹³C-NMR (101 MHz, DMSO-D₆) δ ppm: 9.9, 30.1, 31.3, 32.2, 37.7, 43.4, 54.8, 104.7, 110.5, 110.6, 113.4, 113.4, 117.0, 117.1, 117.8, 117.9, 120.0, 120.1, 126.2, 126.4, 126.8, 127.0, 127.3, 127.5, 127.6, 128.2, 129.5, 129.7, 134.6, 134.7, 139.2, 157.0, 157.1

### Beispiel Nr. 19

### Stufe 1:

### 1-Butyl-N,N-dimethyl-4,4-bis-(3-methyl-1H-indol-2-yl)cyclohexylamin (Beispiel Nr. 19)

3-Methyl-1H-indol (4 g, 30,492 mmol) wurde zusammen mit dem 4-Butyl-4-(dimethylamino)cyclohexanon (2 g, 10,14 mmol) in abs. Dichlormethan (40 ml) gelöst und mit Trifluormethansulfonsäure (1,4 ml, 14,93 mmol) versetzt. Der Ansatz wurde 6 d bei 24 °C gerührt. Zur Aufarbeitung wurde die Reaktionslösung mit 1 N NaOH (25 ml) versetzt. Das Gemisch wurde 25 min gerührt. Nach der Trennung der Phasen wurde die wäßrige Phase mit Dichlormethan (3 x 20 ml) extrahiert. Die vereinigten organischen Extrakte wurden über Na₂SO₄ getrocknet und anschließend eingeengt. Das erhaltene Rohprodukt (braunes Öl, 6,75 g) wurde säulenchromatographisch gereinigt [Kieselgel 60 (70 g); Ethylacetat/Methanol 5 : 1 (1200 ml), Ethylacetat/Methanol 2 : 1 (1200 ml)]. Die gewünschte Bisindolverbindung wurde als hellgelber Feststoff (2016 mg, 45 %, Schmp.: 192-195 °C) erhalten.
Beispiel Nr. 19: ¹³C NMR (101 MHz, CDCl₃) δ ppm: 9.4, 10.3, 14.1, 23.8, 26.6, 29.8, 29.9, 31.7, 37.3, 42.7, 56.0, 106.3, 108.3, 110.4, 110.6, 117.9, 118.2, 118.9, 119.2, 121.1, 121.5, 130.3, 130.6, 133.7, 134.4, 136.5, 138.8

### Beispiel Nr. 20

### Stufe 1:

### 2-[2-(1H-Indol-3-yl)ethyl]indan-1,3-dion

Tryptamin (3,09 g; 19 mmol) wurde in Toluol (300 ml) gelöst. Anschließend wurde Phthalsäureanhydrid (3 g; 20,2 mmol) zugegeben. Die gelbe Reaktionslösung wurde 7 h unter Rückfluß gekocht (Wasserabscheider). Der Verlauf der Reaktion wurde durch DC kontrolliert. Zur Aufarbeitung wurde Toluol vollständig abdestilliert. Der zurück gebliebene gelbe Feststoff wurde aus Cyclohexan/Chloroform (1 : 1) umkristallisiert. Es wurden 4,796 g (90 %) des Produktes erhalten.

### Stufe 2:

### 2-(2-(2-(4-Butyl-4-dimethylaminocyclohex-1-enyl)-1H-indol-3-yl)-ethyl)isoindol-1,3-dion

Das 2-[2-(1*H*-Indol-3-yl)ethyl]indan-1,3-dion (4,40 g, 15,16 mmol) wurde zusammen mit dem 4-Butyl-4-(dimethylamino)cyclohexanon (2,50 g, 12,67 mmol) mit HBr/Eisessig (33 % HBr, 15 ml) versetzt und 5 h bei 24 °C gerührt. Anschließend wurde das Gemisch mit Dichlormethan (50 ml) verdünnt. Unter Kühlung wurde 5N Natriumhydroxyd-Lösung (100 ml) zugegeben. Der Ansatz wurde 10 min bei Raumtemperatur gerührt. Nach der Trennung der Phasen wurde die wäßrige Phase mit Dichlormethan (3 × 20 ml) extrahiert. Die vereinigten organischen Extrakte wurden über Na₂SO₄ getrocknet und anschließend eingeengt. Das erhaltene Rohprodukt (5,81 g, braunes Öl) wurde chromatographisch gereinigt [Kieselgel 60 (110 g); Ethylacetat/Methanol 2 : 1 (1200 ml)]. Es wurde 1,98 g (33 %) der Zielverbindung (Schmelzpunkt 125-130 °C) erhalten.
¹³C NMR (101 MHz, CDCl₃) δ ppm: 14.2, 23.6, 24.3, 25.6, 26.6, 28.5, 30.5, 32.3, 38.1, 38.5, 56.0, 107.8, 110.4, 118.5, 119.6, 121.9, 123.1, 126.8, 128.8, 129.1, 132.3, 133.8, 134.93, 136.5, 168.2

### Stufe 3:

### 2-(2-(2-(4-Butyl-4-dimethylamino-1-(3-methyl-1H-indol-2-yl)cyclohexyl)-1H-indol-3-yl)ethyl)isoindolin-1,3-dion (Beispiel Nr. 20, Gemisch aus zwei Diastereomeren)

3-Methyl-1H-indol (1,0 g, 7,62 mmol) wurde zusammen mit dem 2-(2-(2-(4-Butyl-4-dimethylaminocyclohex-1-enyl)-1*H*-indol-3-yl)-ethyl)isoindol-1,3-dion (1,20 g, 2,56 mmol) in abs. Dichlormethan (15 ml) gelöst und mit Trifluormethansulfonsäure (0,3 ml, 3,41 mmol) versetzt. Der Ansatz wurde 6 d bei 24 °C gerührt. Zur Aufarbeitung wurde die Reaktionslösung mit 1 N NaOH (20 ml) versetzt. Das Gemisch wurde 10 min gerührt. Nach der Trennung der Phasen wurde die wäßrige Phase mit Dichlormethan (3 x 15 ml) extrahiert. Die vereinigten organischen Extrakte wurden über Na₂SO₄ getrocknet und anschließend eingeengt. Das erhaltene Rohprodukt (braunes Öl, 2,3 g) wurde säulenchromatographisch gereinigt [Kieselgel 60 (100 g); Ethylacetat/Methanol 5 : 1 (600 ml), Ethylacetat/Methanol 2 : 1 (1200 ml)]. Es wurde die gewünschte Bisindolverbindung als hellgelber Feststoff (505 mg, 33 %, Schmp. nicht bestimmbar) erhalten. Laut NMR-Spektrum war es ein Gemisch der zwei möglichen Diastereoisomeren im Verhältnis von ca. 30 : 70
Beispiel Nr. 20:¹³C NMR (101 MHz, CDCl₃) δ ppm: 9.6, 10.2, 14.1, 23.8, 24.7, 26.5, 26.6, 29.7, 30.5, 31.6, 31.8, 37.4, 38.3, 38.7, 42.3, 42.7, 56.1, 106.5, 107.4, 108.1, 109.2, 110.6, 110.72, 110.75, 110.9, 117.8, 118.1, 118.2, 118.3, 118.8, 119.0, 119.4, 119.6, 121.0, 121.30, 121.32, 121.7, 123.1, 129.7, 129.8, 130.3 130.4, 132.16, 132.22, 133.8, 134.0, 134.4 134.5, 137.8, 138.7, 140.7, 168.4

### Beispiel Nr. 21

### Stufe 1:

### 1-(Benzo[d][1,3]dioxol-5-yl)-4-(dimethylamino)-4-phenylcyclohexanol (polareres Diastereoisomer)

Das 4-(Dimethylamino)-4-phenylcyclohexanon (4,34 g, 20 mmol) wurde unter Ausschluß von Sauerstoff in absolutem Tetrahydrofuran (60 ml) gelöst, unter Eiskühlung mit 3,4-(Methylendioxy)phenylmagnesiumbromid-Lösung (45 ml, 45 mmol, 1 N) versetzt und 3 h am Rückfluß gekocht. Zur Aufarbeitung wurde der Ansatz unter Eiskühlung mit gesättigter NH₄Cl-Lösung (50 ml) versetzt und 10 min. bei Raumtemperatur gerührt. Die wäßrige Phase wurde abgetrennt und mit Ethylacetat (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (1000 ml), Methanol (1000 ml)]. Es entstanden 2,0 g (29 %, Smp. 104-107 °C) polareres Produkt. Es wurden nur Spuren an unpolarerem Produkt gefunden.
Polareres Diastereoisomer: ¹³C-NMR (101 MHz, DMSO-D₆) δ ppm: 28.1, 35.6, 37.7, 60.7, 70.5, 100.5, 105.5, 107.3, 117.2, 126.2, 126.7, 127.4, 127.5, 127.7, 127.8, 136.5, 138.2, 144.5, 145.2, 146.7

### Stufe 2:

### 4-(Benzo[d][1,3]dioxol-5-yl)- N,N-dimethyl-4-(3-methyl-1H-indol-2-yl)-1-phenylcyclohexylamin (Beispiel Nr. 21, eins von zwei möglichen Diastereoisomeren)

3-Methyl-1H-indol (393 mg, 3 mmol) und das polarere 1-(Benzo[*d*][1,3]dioxol-5-yl)-4-(dimethylamino)-4-phenylcyclohexanol (679 mg, 2 mmol) wurden unter Ausschluß von Sauerstoff in absolutem Dichlormethan (50 ml) gelöst, mit Trifluormethansulfonsäure-trimethylsilylester (581 µl, 3 mmol) versetzt und 16 h bei Raumtemperatur gerührt. Zur Aufarbeitung wurde der Ansatz mit 5N NaOH (50 ml) versetzt und 1 h bei Raumtemperatur gerührt. Die wäßrige Phase wurde abgetrennt und mit Dichlormethan (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (1000 ml)]. Das Produkt wurde in einer Ausbeute von 545 mg (60 %) mit einem Schmelzpunkt von 235-238 °C als weißer Feststoff erhalten . Es handelte sich dabei um eins von zwei möglichen Diastereoisomeren.
Beispiel Nr. 21: ¹³C-NMR (101 MHz, DMSO-D₆) δ ppm: 9.9, 30.3, 32.0, 38.0, 44.5, 60.4, 100.9, 106.6, 108.0, 110.1, 117.8, 118.9, 120.2, 121.1, 126.6, 127.3, 127.7, 130.3, 133.9, 137.4, 139.0, 145.8, 147.9

### Beispiel Nr. 22

### Stufe 1:

### 3-(1H-Indol-3-yl)propionsäuremethylester

### Unter Argon wurde 3-Indolpropionsäure (3,78 g, 20 mmol) in Methanol (50 ml) gelöst. Zu dieser Lösung wurde sehr langsam Thionylchlorid (4,7 g, 2,9 ml, 40 mmol) getropft. Dabei stieg die Temperatur bis 35 °C an. Anschließend wurde 7 h zum Rückfluß erhitzt und über Nacht bei Raumtemperatur gerührt. Im DC war vollständiger Umsatz zu erkennen. Das LC/MS zeigte geringe Spuren eines Nebenproduktes. Der Ansatz wurde unter dem Abzug eingeengt und mit Methanol codestilliert. Der Rückstand war ein braunes Öl, das so für die nächste Reaktionsstufe eingesetzt wurde.

### Stufe 2:

### Dimethyl 3,3'-(2-(4-butyl-4-(dimethylamino)cyclohexan-1,1-diyl)bis(1H-indol-2,3-diyl)dipropanat (Beispiel Nr. 22)

Unter Argon wurden das 4-Butyl-4-(dimethylamino)cyclohexanon (395 mg, 2 mmol) und der 3-(1*H*-Indol-3-yl)propionsäuremethylester (1,02 g, 5 mmol) in Dichlormethan (20 ml) gelöst. Nach Zugabe der Trifluormethansulfonsäure (115 µl, 1,3 mmol) wurde der Ansatz 4 Tage bei Raumtemperatur gerührt. Die Reaktion verlief unter LC/MS- und DC-Kontrolle. Zur Aufarbeitung wurde der Ansatz mit gesättigter Natriumhydrogencarbonatlösung (35 ml) alkalisch gemacht und 20 min gerührt. Die Phasen wurden getrennt und die wäßrige Phase wurde mit Dichlormethan (2 × 20 ml) extrahiert. Die organischen Phasen wurden getrocknet und eingeengt. Es wurde ein brauner Feststoff (1,51 g) erhalten, der chromatographisch aufgetrennt wurde [Kieselgel 60 (50 g); Ethylacetat/Methanol 4 : 1 (1000 ml), Ethylacetat/Methanol 1 : 1 (500 ml), Methanol (1000 ml)]. Das Produkt wurde in einer Ausbeute von 8 % (92 mg) erhalten. Durch Umkristallisation aus Ethylacetat und Cyclohexan (8 ml) konnte es gereinigt werden.
Beispiel Nr. 22: Ausbeute: 42 mg, Schmelzpunkt von 199-206 °C.
¹³C-NMR (101 MHz, CDCl₃) δ ppm: 13.7, 19.5, 21.2, 23.2, 25.9, 28.5, 30.7, 31.8, 34.5, 35, 37.2, 42.4, 51.4, 51.6, 111.3, 111.5, 118.1, 118.1, 119.1, 119.5, 121.8, 122.1, 128.7, 128.9, 135.1, 173.5, 173.8

### Beispiel Nr. 23

### Stufe 1:

### 2-(2-(2-(4-Butyl-4-(dimethylamino)cyclohex-1-enyl)-1H-indol-3-yl)-ethyl)isoindolin-1,3-dion

Das 2-[2-(1*H*-Indol-3-yl)ethyl]indan-1,3-dion (4,40 g, 15,16 mmol) wurde zusammen mit dem 4-Butyl-4-(dimethylamino)cyclohexanon (2,50 g, 12,67 mmol) mit HBr/Eisessig (33 % HBr, 15 ml) versetzt und 5 h bei 24 °C gerührt. Anschließend wurde das Gemisch mit Dichlormethan (50 ml) verdünnt. Unter Kühlung wurde 5N Natriumhydroxyd-Lösung (100 ml) zugegeben. Der Ansatz wurde 10 min bei Raumtemperatur gerührt. Nach der Trennung der Phasen wurde die wäßrige Phase mit Dichlormethan (3 × 20 ml) extrahiert. Die vereinigten organischen Extrakte wurden über Na₂SO₄ getrocknet und anschließend eingeengt. Das erhaltene Rohprodukt (5,81 g, braunes Öl) wurde chromatographisch gereinigt [Kieselgel 60 (110 g); Ethylacetat/Methanol 2 : 1 (1200 ml)]. Es wurden 1,98 g (33 %) der Zielverbindung (Schmelzpunkt 125-130 °C) erhalten.
¹³C NMR (101 MHz, CDCl₃) δ ppm: 14.2, 23.6, 24.3, 25.6, 26.6, 28.5, 30.5, 32.3, 38.1, 38.5, 56.0, 107.8, 110.4, 118.5, 119.6, 121.9, 123.1, 126.8, 128.8, 129.1, 132.3, 133.8, 134.93, 136.5, 168.2

### Stufe 2:

### 2-(2-(2-(4-Butyl-4-(dimethylamino)-1-(3-methyl-1H-indol-2-yl)cyclohexyl)-1H-indol-3-yl)ethyl)isoindolin-1,3-dion

3-Methyl-1 H-indol (1,0 g, 7,62 mmol) wurde zusammen mit dem 2-(2-(2-(4-Butyl-4-(dimethylamino)cyclohex-1-enyl)-1*H*-indol-3-yl)-ethyl)isoindolin-1,3-dion (1,20 g, 2,56 mmol) in abs. Dichlormethan (15 ml) gelöst und mit Trifluormethansulfonsäure (0,3 ml, 3,41 mmol) versetzt. Der Ansatz wurde 6 d bei 24 °C gerührt. Zur Aufarbeitung wurde die Reaktionslösung mit 1 N NaOH (20 ml) versetzt. Das Gemisch wurde 10 min gerührt. Nach der Trennung der Phasen wurde die wäßrige Phase mit Dichlormethan (3 x 15 ml) extrahiert. Die vereinigten organischen Extrakte wurden über Na₂SO₄ getrocknet und anschließend eingeengt. Das erhaltene Rohprodukt (braunes Öl, 2,3 g) wurde säulenchromatographisch gereinigt [Kieselgel 60 (100 g); Ethylacetat/Methanol 5 : 1 (600 ml), Ethylacetat/Methanol 2 : 1 (1200 ml)]. Es wurde die gewünschte Bisindolverbindung als hellgelber Feststoff (505 mg, 33 %) erhalten. Laut NMR-Spektrum war es ein Gemisch der zwei möglichen Diastereoisomeren im Verhältnis von ca. 30 : 70
¹³C NMR (101 MHz, CDCl₃) δ ppm: 9.6, 10.2, 14.1, 23.8, 24.7, 26.5, 26.6, 29.7, 30.5, 31.6, 31.8, 37.4, 38.3, 38.7, 42.3, 42.7, 56.1, 106.5, 107.4, 108.1, 109.2, 110.6, 110.72, 110.75, 110.9, 117.8, 118.1, 118.2, 118.3, 118.8, 119.0, 119.4, 119.6, 121.0, 121.30, 121.32, 121.7, 123.1, 129.7, 129.8, 130.3 130.4, 132.16, 132.22, 133.8, 134.0, 134.4 134.5, 137.8, 138.7, 140.7, 168.4

### Stufe 3:

### 4-(3-(2-Aminoethyl)-1H-indol-2-yl)-1-butyl-N,N-dimethyl-4-(3-methyl-1H-indol-2-yl)cyclohexylamin (Beispiel Nr. 23, Gemisch aus zwei Diastereoisomeren)

Das 2-(2-(2-(4-Butyl-4-(dimethylamino)-1-(3-methyl-1*H*-indol-2-yl)cyclohexyl)-1*H*-indol-3-yl)ethyl)isoindolin-1,3-dion (450 mg, 0,749 mmol) und Hydrazin-monohydrat (2 ml, 41,2 mmol) wurden in Methanol (20 ml) gelöst und dann 45 min unter Rückfluß gekocht. Zur Aufarbeitung wurde der Ansatz mit 1 N NaOH (50 ml) verdünnt und mit Diethylether (3 × 20 ml) extrahiert. Die vereinigten organischen Phasen wurden über Na₂SO₄ getrocknet und anschließend bis zur Trockne eingeengt. Das Produkt wurde in einer Ausbeute von 302 mg (86 %, Schmelzpunkt 105-112 °C) als hellbeigefarbener Feststoff erhalten. Laut NMR-Spektrum war es ein Gemisch der zwei möglichen Diastereoisomeren im Verhältnis von ca. 30 : 70.
Beispiel Nr. 23:¹³C NMR (101 MHz, CDCl₃) δ ppm: 9.69, 10.5, 14.0 +Schulter, 23.8 +Schulter, 26.6, 26.9, 29.0, 29.2, 29.69, 29.75, 30.6, 30.8, 31.6, 31.8, 37.3 +Schulter, 42.2, 42.9, 43.0, 56.2, 105.8, 108.1, 108.7, 110.4, 110.59, 110.66, 110.74, 117.9, 118.19, 118.27, 118.5, 118.7, 119.0, 119.14, 119.24, 121.0, 121.2, 121.5 +Schulter, 129.78, 129.87, 130.22, 130.36, 134.2, 134.7, 136.4, 137.6, 139.2, 140.0

### Beispiel Nr. 24

### Stufe 1:

### 4-(Dimethylamino)-4-(3-fluorphenyl)-1-(thiophen-2-yl)cyclohexanol (unpolareres Diastereoisomer und polareres Diastereoisomer)

Das 4-(Dimethylamino)-4-(3-fluorphenyl)cyclohexanon (2,35 g, 10 mmol) wurde in absolutem THF (30 ml) vorgelegt und innerhalb von 10 min mit 2-Thienylmagnesiumbromid-Lösung (1M in THF, 22,5 ml, 22,5 mmol) versetzt. Die Reaktionslösung wurde 2 h unter Rückfluß zum Sieden erhitzt. - Zur Aufarbeitung wurde die Lösung unter Eisbadkühlung vorsichtig mit Eisstücken und gesättigter NH₄Cl-Lösung (25 ml) versetzt. Anschließend wurde mit Ethylacetat (3 × 30 ml) extrahiert. Die vereinigten organischen Phasen wurden mit Wasser (20 ml) und gesättigter NaCl-Lösung (20 ml) gewaschen und über Natriumsulfat getrocknet. Anschließend wurden die flüchtigen Bestandteile vollständig im Vakuum entfernt. - Die chromatographische Trennung des Substanzgemisches (3 g) an Kieselgel 60 (100 g) erfolgte mit Ethylacetat (1000 ml). Das unpolarere Diastereoisomer wurde in einer Ausbeute von 17 % (540 mg) als beigefarbene Verbindung erhalten. Mit einer Ausbeute von 23 % (700 mg) konnte das polarere Diastereoisomer als beigefarbene Verbindung gewonnen werden.

### Stufe 2:

### 1-(3-Fluorphenyl)- N,N-dimethyl-4-(3-methyl-1H-indol-2-yl)-4-thiophen-2-ylcyclohexylamin (Beispiel Nr. 24, ein Diastereoisomer)

3-Methyl-1H-indol (344 mg, 2,62 mmol) wurde unter Feuchtigkeitsausschluß zusammen mit dem polareren 4-(Dimethylamino)-4-(3-fluorphenyl)-1-(thiophen-2-yl)cyclohexanol (419 mg, 1,31 mmol, AS 05766) in trockenem Dichlormethan (40 ml) vorgelegt und schnell mit Trifluormethansulfonsäure-trimethylsilylester (0,38 ml, 1,9 mmol) versetzt. Der Ansatz wurde bei RT 24 h gerührt. - Zur Aufarbeitung des Ansatzes wurde die Mischung mit 2N Natronlauge (10 ml) versetzt und 15 min gerührt. Die wäßrige Phase wurde mit Dichlormethan (2 × 30 ml) extrahiert. Die vereinigten organischen Phasen wurden nach dem Trocknen (Na₂SO₄) eingeengt, wobei ein braunes Öl (700 mg) erhalten wurde. Nach Zugabe von Methanol (10 ml) fiel ein weißer Feststoff aus, welcher abgesaugt und anschließend getrocknet wurde. Eins der beiden möglichen Diastereoisomeren konnte so in 200 mg Ausbeute (35 %) mit einem Schmelzpunkt von 211-233 °C erhalten werden .
Beispiel Nr. 24:¹³C NMR (101 MHz, CDCl₃, δ ppm): 9.9, 30.1, 32.9, 33.7, 37.7, 42.6, 59.9, 104.9, 110.7, 113.0, 113.2, 114.0, 114.3, 117.4, 118.0, 120.4, 122.5, 123.5, 126.3, 129.2, 129.3, 129.5, 134.8, 139.9, 153.2, 161.0, 163.4

### Beispiel Nr. 25 und Beispiel 26

### Stufe 1:

### 1-Butyl-4,4-bis-(1,3-dimethyl-1H-indol-2-yl)-N,N-dimethylcyclohexylamin und 1-Butyl-4-(1,3-dimethyl-1H-indol-2-yl)-N,N-dimethyl-4-(3-methyl-1H-indol-2-yl)cyclohexylamin (eins von zwei möglichen Diastereoisomeren)

Das 1-Butyl-*N,N*-dimethyl-4,4-bis-(3-methyl-1*H*-indol-2-yl)cyclohexylamin (300 mg, 0,679 mmol) wurde unter Argon in abs. Dimethylformamid (3 ml) gelöst und anschließend mit Natriumhydrid (55 - 60 %, 33 mg, 0,754 mmol) versetzt. Der Ansatz wurde 1 h bei 24 °C gerührt. Anschließend wurde bei 0 °C Methyliodid (10 mg, 0,704 mmol), gelöst in abs. Tetrahydrofuran (1 ml), zugetropft. Der Ansatz wurde 4 h bei 24 °C gerührt. Zur Aufarbeitung wurde die Reaktionsmischung mit H₂O (15 ml) versetzt und mit Dichlormethan (3 x 15 ml) extrahiert. Die vereinigten organischen Phasen wurden mit Na₂SO₄ getrocknet und dann filtriert. Die flüchtigen Bestandteile des Filtrates wurden im Vakuum vollständig entfernt. Der Rückstand (gelbes Öl, 372 mg) wurde säulenchromatographisch gereinigt [Kieselgel 60 (40 g); Cyclohexan/Diethylether 5 : 1 (1200 ml), Cyclohexan/Ethylacetat 1 : 1 (600 ml)]. Neben der Dimethylverbindung (39 mg, farbloses Öl) wurde die Monomethylverbindung als gelbes Öl (97 mg, 31 %) erhalten (eins von zwei möglichen Diastereoisomeren).

### Stufe 2:

### 1-Butyl-4,4-bis-(1,3-dimethyl-1H-indol-2-yl)- N,N-dimethylcyclohexylamin Hydrochlorid (Beispiel Nr. 25)

Das 1-Butyl-4,4-bis-(1,3-dimethyl-1*H*-indol-2-yl)- *N,N*-dimethylcyclohexylamin (39 mg, 0,083 mmol) wurde in Cyclohexan (10 ml) gelöst. Die Lösung wurde bei 24 °C mit Chlortrimethylsilan (20 µl, 0,156 mmol) versetzt. Das klare Reaktionsgemisch wurde sofort trüb. Bei 24 °C wurde 30 min gerührt. Der weiße Niederschlag wurde abgesaugt. Der Feststoff wurde mit Cyclohexan (3 × 0,5 ml) gewaschen und dann getrocknet. Das Hydrochlorid (40 mg, 95 %, Schmelzpunkt 153-157 °C) wurde als weißer Feststoff erhalten. Beispiel Nr. 25:¹³C-NMR (101 MHz, CD₃OD, δ ppm, Hydrochlorid): 12.8, 12.9, 14.2, 24.2, 26.5, 28.5, 31.0, 31.6, 32.3, 33.6, 38.5, 45.5, 68.4, 107.9, 109.8, 109.9, 110.3, 119.1, 119.2, 120.2, 120.3, 123.0, 123.4, 130.3, 130.5, 136.4, 138.7, 139.2, 139.9

### Stufe 3:

### 1-Butyl-4-(1,3-dimethyl-1H-indol-2-yl)- N,N-dimethyl-4-(3-methyl-1H-indol-2-yl)cyclohexylamin Hydrochlorid (Beispiel Nr. 26)

Das 1-Butyl-4-(1,3-dimethyl-1*H*-indol-2-yl)-*N,N*-dimethyl-4-(3-methyl-1*H*-indol-2-yl)cyclohexylamin (97 mg, 0,213 mmol) wurde in Cyclohexan (10 ml) gelöst. Die Lösung wurde bei 24 °C mit Chlortrimethylsilan (40 µl, 0,313 mmol) versetzt. Das klare Reaktionsgemisch wurde sofort trüb. Bei 24 °C wurde 30 min gerührt. Der weiße Niederschlag wurde abgesaugt. Der Feststoff wurde mit Cyclohexan (3 × 0,5 ml) gewaschen und dann getrocknet. Das Hydrochlorid (95 mg, 91 %, (Schmelzpunkt 183-187 °C) wurde als weißer Feststoff erhalten.
Beispiel Nr. 26: ¹³C-NMR (101 MHz, CD₃OD, δ ppm, Hydrochlorid): 8.5, 12.4, 14.3, 24.2, 26.7, 28.6, 31.1, 33.2, 38.4, 43.6, 68.6, 108.3, 109.8, 110.4, 111.8, 118.6, 119.2, 119.8, 120.1, 122.3, 123.2, 130.7, 131.2, 136.6, 138.3, 138.3, 140.1

### Beispiel Nr. 27

### Stufe 1:

### 4-Benzyl-4-(dimethylamino)-1-(thiophen-2-yl)cyclohexanol (unpolareres Diastereoisomer und polareres Diastereoisomer)

Das 4-Benzyl-4(dimethylamino)cyclohexanon (2,31 g, 10 mmol) wurde in absolutem THF (30 ml) vorgelegt und innerhalb von 10 min mit 2-Thienylmagnesiumbromid-Lösung (1M in THF, 22,5 ml, 22,5 mmol) versetzt. Die Reaktionslösung wurde 2 h unter Rückfluß zum Sieden erhitzt. - Zur Aufarbeitung wurde die Lösung unter Eisbadkühlung vorsichtig mit Eisstücken und gesättigter NH₄Cl-Lösung (25 ml) versetzt. Anschließend wurde mit Ethylacetat (3 × 30 ml) extrahiert. Die vereinigten organischen Phasen wurden mit Wasser (20 ml) und gesättigter NaCl-Lösung (20 ml) gewaschen und über Natriumsulfat getrocknet. Anschließend wurden die flüchtigen Bestandteile vollständig im Vakuum entfernt. - Die chromatographische Trennung des Substanzgemisches (3 g) an Kieselgel 60 (100 g) erfolgte mit Ethylacetat/Cyclohexan 10 : 1 (1000 ml). Das unpolarere Diastereoisomer wurde in einer Ausbeute von 7 % (195 mg, AS 05769) als beigefarbene Verbindung erhalten. Ein Schmelzpunkt konnte nicht bestimmt werden. Mit einer Ausbeute von 26 % (820 mg) konnte das polarere Diastereoisomer als beigefarbene Verbindung gewonnen werden.
¹³C NMR (101 MHz, DMSO-D₆, δ ppm, unpolareres Diastereoisomer): 28.2, 34.8, 36.8, 37.1, 57.3, 71.4121.5, 123.4, 125.7, 126.5, 127.9, 130.7, 139.1, 155.4
¹³C NMR (101 MHz, DMSO-D₆, δ ppm, polareres Diastereoisomer): 28.8, 35.7, 36.6, 37.3, 57.7, 71.7, 122.5, 123.8, 125.8, 127.8, 130.6, 138.7, 152.7

### Stufe 2:

### 1-Benzyl-N,N-dimethyl-4-(3-methyl-1H-indol-2-yl)-4-(thiophen-2-yl)cyclohexylamin (Beispiel Nr. 27, eins von zwei möglichen Diastereoisomeren)

3-Methyl-1H-indol (414 mg, 3,16 mmol) wurde unter Feuchtigkeitsausschluß zusammen mit dem polareren 4-Benzyl-4-(dimethylamino)-1-(thiophen-2-yl)cyclohexanol (500 mg, 1,58 mmol, AS 05770) in trockenem Dichlormethan (40 ml) vorgelegt und schnell mit Trifluormethansulfonsäure-trimethylsilylester (0,46 ml, 2,37 mmol) versetzt. Der Ansatz wurde bei RT 24 h gerührt. - Zur Aufarbeitung des Ansatzes wurde die Mischung mit 2N Natronlauge (10 ml) versetzt und 15 min gerührt. Die wäßrige Phase wurde mit Dichlormethan (2 × 30 ml) extrahiert. Die vereinigten organischen Phasen wurden nach dem Trocknen (Na₂SO₄) eingeengt, wobei ein braunes Öl (750 mg) erhalten wurde. Nach Zugabe von Methanol (10 ml) fiel ein weißer Feststoff aus, welcher abgesaugt und anschließend getrocknet wurde. Eins der beiden möglichen Diastereoisomeren konnte so in 260 mg Ausbeute (38 %) mit einem Schmelzpunkt von 119-141 °C erhalten werden.
Beispiel Nr 27: ¹³C NMR (101 MHz, CDCl₃, δ ppm): 9.7, 28.3, 31.6, 36.3, 36.8, 41.8, 56.8, 104.0, 110.7, 117.2, 117.9, 120.2, 123.5, 123.7, 125.4, 126.0, 127.5, 129.4, 130.4, 134.2, 138.7, 141.0, 151.0

### Beispiel Nr. 28

### Stufe 1:

### 4-Dimethylamino-1-(3-methoxyphenyl)-4-phenylcyclohexanol(unpolareres Diastereoisomer und polareres Diastereoisomer)

Das 4-(Dimethylamino)-4-phenylcyclohexanon (4,34 g, 20 mmol) wurde unter Ausschluß von Sauerstoff in absolutem Tetrahydrofuran (60 ml) gelöst, unter Eiskühlung mit 3-Methoxyphenylmagnesiumbromid-Lösung (45 ml, 45 mmol, 1N) versetzt und 2 h am Rückfluß gekocht. Zur Aufarbeitung wurde der Ansatz unter Eiskühlung mit gesättigter NH₄Cl-Lösung (50 ml) versetzt und 10 min. bei Raumtemperatur gerührt. Die wäßrige Phase wurde abgetrennt und mit Ethylacetat (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (1000 ml), Methanol (1000 ml)]. Es entstanden 2,5 g (38 %, Smp. 116-117 °C) unpolareres und 2,78 g (43 %, Smp. 139-140 °C) polareres Produkt. Beide konnten aus Ethylacetat umkristallisiert werden.
Unpolareres Diastereoisomer: ¹³C-NMR (101 MHz, DMSO-D₆) δ ppm: 28.6, 34.0, 37.8, 54.9, 58.0, 70.9, 110.8, 111.1, 117.1, 126.1, 126.5, 127.2, 128.7, 139.4, 152.5, 158.9
Polareres Diastereoisomer: ¹³C-NMR (101 MHz, DMSO-D₆) δ ppm: 28.0, 35.5, 37.9, 54.7, 60.7, 70.7, 110.8, 110.9, 116.6, 126.2, 127.5, 127.8, 128.7, 136.5, 151.9, 158.8

### Stufe 2:

### 4-(3-Methoxyphenyl)- N,N-dimethyl-4-(3-methyl-1H-indol-2-yl)-1-phenylcyclohexylamin (Beispiel Nr. 28, eins von zwei Diastereoisomeren)

3-Methyl-1H-indol (393 mg, 3 mmol) und das Gemisch 4-Dimethylamino-1-(3-methoxyphenyl)-4-phenylcyclohexanol (651 mg, 2 mmol) wurden unter Ausschluß von Sauerstoff in absolutem Dichlormethan (50 ml) gelöst, mit Trifluormethansulfonsäure-trimethylsilylester (581 µl, 3 mmol) versetzt und 16 h bei Raumtemperatur gerührt. Zur Aufarbeitung wurde der Ansatz mit 5N NaOH (50 ml) versetzt und 1 h bei Raumtemperatur gerührt. Die wäßrige Phase wurde abgetrennt und mit Dichlormethan (3 × 25 ml) extrahiert. Die vereinigten organischen Phasen wurden über Na₂SO₄ getrocknet und anschließend eingeengt. Es wurde ein Rohprodukt isoliert, das chromatographisch aufgetrennt wurde [Kieselgel 60 (50 g); Ethylacetat (500 ml)]. Das Produkt wurde in einer Ausbeute von 90 mg (10 %) mit einem Schmelzpunkt von 278-281 °C als weißer Feststoff erhalten. Es handelte sich dabei um eins von zwei möglichen Diastereoisomeren.
Beispiel Nr. 28: ¹³C-NMR (101 MHz, CDCl₃, δ ppm): 10.1, 30.4, 32.3, 38.0, 44.8, 55.0, 60.5, 107.4, 110.1, 110.6, 113.5, 117.5, 117.9, 118.8, 119.2, 121.1, 126.6, 127.1, 127.4, 127.7, 129.2, 130.5, 134.4, 137.1, 138.2, 159.5

### Beispiel Nr. 29

### Stufe 1:

### N-(2-(2-(4-Butyl-4-(dimethytamino)-1-(3-methy)-1H-indol-2-yl)cyclohexyl)-1H-indol-3-yl)ethyl)cyclopentansulfonsäureamid (Beispiel Nr. 29, Gemisch der zwei möglichen Diastereoisomeren)

Das 4-(3-(2-Aminoethyl)-1*H*-indol-2-yl)-1-butyl-*N,N-*dimethyl-4-(3-methyl-1*H*-indol-2-yl)cyclohexylamin (120 mg, 0,255 mmol, Gemisch der zwei möglichen Diastereoisomeren im Verhältnis von ca. 30 : 70) wurde in Dichlormethan (5 ml) mit Triethylamin (0,12 ml, 0,863 mmol) und Cyclopentansulfonylchlorid (65 mg, 0,385 mmol) versetzt. Nach einer Reaktionszeit von 24 h bei 25 °C wurde die Reaktionsmischung mit 1N Natronlauge (10 ml) versetzt und mit Dichlormethan (3 × 15 ml) extrahiert. Die vereinigten organischen Phasen wurden mit Na₂SO₄ getrocknet und dann filtriert. Die flüchtigen Bestandteile des Filtrates wurden im Vakuum vollständig entfernt. Der Rückstand (gelber Schaum, 173 mg) wurde säulenchromatographisch gereinigt [Kieselgel 60 (25 g); Ethylacetat/Methanol 4 : 1 (250 ml), Methanol/30-proz. Ammoniak 9 : 1(100 ml)]. Das Produkt wurde in einer Ausbeute von 39 mg (25 %) als beigefarbener Feststoff (Schmelzpunkt 111-115 °C, AS 11227, Gemisch der zwei möglichen Diastereoisomeren im Verhältnis von ca. 30 : 70) erhalten.
Beispiel Nr. 29: ¹³C-NMR (101 MHz, CDCl₃, δ ppm): 9.4, 10.35, 10,39, 13.99, 14.07, 23.7, 25.7 + Schulter, 26.5, 26.6, 27.1, 27.9, 28.0, 29.5, 30.6, 31.6, 37.35, 37.4, 41.7, 42.1, 43.0, 43.6, 56.03, 61.4, 61.8, 106.9, 108.5, 110.66, 110.75, 110.91, 110.95, 117.93, 118.03, 118.13, 118.33, 119.15, 119.39, 119.41, 119.79, 121.40 121.48, 121.77, 129.7, 13.2, 134.0, 134.3, 134.5, 138.8, 139.0

### Beispiel Nr. 30

### Stufe 1:

### 1-(3-Fluorphenyl)- N,N-dimethyl-4-(3-methyl-1H-indol-2-yl)-4-thiophen-2-ylcyclohexylamin 2-Hydroxypropan-1,2,3-tricarbonsäure (Beispiel Nr. 30, Diastereoisomerengemisch)

Zur Herstellung des Citrates wurde das Diastereoisomerengemisch von 1-(3-Fluorphenyl)-*N,N*-dimethyl-4-(3-methyl-1*H*-indol-2-yl)-4-thiophen-2-ylcyclohexylamin (280 mg, 0,64 mmol, AS 05768) in heißem Isopropanol (80 ml) gelöst und mit einer ebenfalls heißen, isopropanolischen Citronensäure-Lösung (187 mg, 0,97 mmol in 3 ml) versetzt. Anschließend wurde die Reaktionsmischung 16 h im Kühlschrank gelagert. Der entstandene Feststoff wurde abgesaugt. Das Citrat wurde so in einer Ausbeute von 60 mg (14 %) als weißer Feststoff (Schmelzpunkt: 99-117 °C) erhalten.
Beispiel Nr. 30: ¹³C-NMR (101 MHz, DMSO-d₆) δ ppm: 9.9, 10.0, 29.3, 32.9, 34.0, 37.5, 37.6, 42.5, 43.4, 45.4, 71.8, 104.9, 110.7, 114.4, 117.4, 117.6, 118.0, 120.5, 120.7, 123.6, 123.8, 126.4, 126.6, 129.3, 129.6, 134.5, 135.0, 160.9, 163.4, 171.2, 175.5

### Beispiel Nr. 31

### Stufe 1:

### N,N-Dimethyl-1-phenyl-4,4-bis-(3-(2-pyridin-4-yl)ethyl)-1H-indol-2-yl)cyclohexylamin (Beispiel Nr. 31)

Das 3-(2-Pyridin-4-ylethyl)-1 H-indol (667 mg, 3 mmol, Synthese vgl. WO2008009415, Indolbaustein Ind-14) (1,02 g, 4,6 mmol) und das 4-(Dimethylamino)-4-phenylcyclohexanon (1,0 g, 4,6 mmol) wurden in abs. Dichlormethan (80 ml) gelöst und mit Trifluormethansulfonsäure (1,07 ml, 1,72 g, 11,5 mmol) versetzt. Nach einer Reaktionszeit von 4 d bei Raumtemperatur wurde die dunkelbraune Reaktionsmischung mit Wasser (30 ml), 1 N Natronlauge (20 ml) und Tetrahydrofuran (20 ml) versetzt und 2,5 h gerührt. Die Phasen wurden getrennt. Die wäßrige Phase wurde mit Dichlormethan (30 ml) extrahiert. Die vereinigten organischen Phasen wurden mit Wasser (30 ml) gewaschen, mit Natriumsulfat getrocknet und eingeengt. Das erhaltene Rohprodukt (dunkelbraunes Öl, 2,08 g) wurde chromatographisch aufgetrennt [Kieselgel 60 (150 g); Ethylacetat/Methanol 15 : 1(1800 ml), Ethylacetat/Methanol 6 : 1 (600 ml), Ethylacetat/Methanol 1 : 1 (1200 ml)]. Die Bisindolverbindung wurde in einer Ausbeute von 82 mg als leicht verunreinigter beigefarbener Feststoff isoliert. Zur Reinigung der Bisindolverbindung wurde der Feststoff aus Methanol (1,5 ml) umkristallisiert (55, mg, 1,9 %, Schmelzpunkt: 305-310 °C).
Beispiel Nr. 31: ¹³C-NMR (101 MHz, DMSO-D₆, δ ppm): 26.5, 30.1, 31.6, 34.8, 37.7, 40.0, 59.1, 109.3, 109.4, 111.3, 117.5, 118.3, 120.0, 120.1, 123.7, 126.2, 127.0, 127.4, 128.4, 128.5, 134.4, 134.6, 137.7, 140.0, 149,0, 149.2, 150.6

### Beispiel Nr. 32

### Stufe 1:

### 1-(2-(2-(4-Butyl-4-(dimethylamino)-1-(3-methyl-1H-indol-2-yl)cyclohexyl)-1H-indol-3-yl)ethyl)-3-phenylharnstoff (Beispiel Nr. 32, Gemisch der zwei möglichen Diastereoisomeren)

Das 4-(3-(2-Aminoethyl)-1*H*-indol-2-yl)-1-butyl-*N,N*-dimethyl-4-(3-methyl-1*H*-indol-2-yl)cyclohexylamin (120 mg, 0,255 mmol, Gemisch der zwei möglichen, Diastereoisomeren im Verhältnis von ca. 30 : 70) wurde in Acetonitril (2,4 ml) mit Phenylisocyanat (0,042 ml, 0,386 mmol) versetzt. Nach einer Reaktionszeit von 2 h bei 24 °C wurde die Reaktionsmischung mit 1N Natronlauge (20 ml) verdünnt und mit Dichlormethan (4 × 10 ml) extrahiert. Die vereinigten organischen Phasen wurden mit Na₂SO₄ getrocknet und dann filtriert. Die flüchtigen Bestandteile des Filtrates wurden im Vakuum vollständig entfernt. Der Rückstand (gelber Feststoff, 150 mg) wurde säulenchromatographisch gereinigt [Kieselgel 60 (25 g); Cyclohexan/Ethylacetat 2 : 1 (300 ml), Ethylacetat/Methanol 1 : 1 (300 ml)]. Das Produkt wurde in einer Ausbeute von 115 mg (76 %) als weißer Feststoff (Schmelzpunkt 115-120 °C, Gemisch der zwei möglichen Diastereoisomeren im Verhältnis von ca. 25 : 75) erhalten. Beispiel Nr. 32: ¹³C-NMR (101 MHz, CDCl₃, δ ppm): 9.4, 10.3, 14.1, 22.6, 23.7, 25.2, 25.9, 26.5, 26.6, 29.5, 29.7, 30.5, 31.52, 31.56, 31.65, 37.36, 37.39, 40.5, 41.0, 42.1, 42.2, 56.0, 106.6, 108.1, 110.2, 110.9, 118.0, 118.6, 119.2, 111.31, 119.36, 119.6, 120.4, 120.2, 121.3, 121.5, 123.2, 123.4, 128.9, 129.0, 129.6, 129.7, 130.1, 131.2, 134.0, 134.5, 134.6, 137.8, 138.56, 138.61, 139.7, 140.8, 155.5, 155.8

### Beispiel Nr. 33

### Stufe 1:

### 2,2'-(2,2'-(2,2'-(4-Butyl-4-(dimethylamino)cyclohexan-1,1-diyl)bis(-1H-indol-3,2-diyl))bis(ethan-2,1-diyl)diisoindol-1,3-dion

Das 2-(2-(2-(4-Butyl-4-dimethylaminocyclohex-1-enyl)-1*H*-indol-3-yl)-ethyl)isoindol-1,3-dion (658 mg, 1,4 mmol) und das 2-(2-1*H*-indol-3-yl)ethyl)isoindolin-1,3-dion (934 mg, 3,22 mmol) wurden in abs. Dichlormethan (60 ml) gelöst und mit Trifluormethansulfonsäure (0,226 ml, 362 mg, 2,41 mmol) versetzt. Nach einer Reaktionszeit von 17 d bei Raumtemperatur wurde die dunkelbraune Reaktionsmischung mit Wasser (30 ml) und 1 N Natronlauge (10 ml) versetzt und 2 h gerührt. Die Phasen wurden getrennt. Die wäßrige Phase wurde mit Dichlormethan (30 ml) extrahiert. Die vereinigten organischen Phasen wurden mit Natriumsulfat getrocknet und eingeengt. Das erhaltene Rohprodukt (gelber Feststoff, 1,61 g) wurde chromatographisch aufgetrennt [Kieselgel 60 (120 g); Ethylacetat/Methanol 15 : 1 (1200 ml), Ethylacetat/Methanol 1 : 1 (900 ml)]. Die Bisindolverbindung wurde als gelber Feststoff in einer Ausbeute von 53 % (563 mg) mit einem Schmelzpunkt von 170-172 °C erhalten (AS 04735).

### Stufe 2:

### 2,2'-(2,2'-(4-Butyl-4(dimethylamino)cyclohexan-1,1-diyl)bis(1H-indol-3,2-diyl)diethylamin

Das 2,2'-(2,2'-(2,2'-(4-Butyl-4-(dimethylamino)cyclohexan-1,1-diyl)bis(-1*H*-indol-3,2-diyl))bis(ethan-2,1-diyl)diisoindol-1,3-dion (531 mg, 0,7 mmol wurde in Methanol (40 ml) suspendiert und mit Hydrazinhydrat (3,4 ml, 3,5 g, 70 mmol) versetzt. Beim Erwärmen entstand eine klare helle Lösung, die 1,5 h unter Rückfluß erwärmt wurde. Die Reaktionsmischung wurde abgekühlt, mit 1 N Natronlauge (70 ml) versetzt und Methanol und Hydrazin im Vakuum entfernt. Der wäßrige Rückstand wurde mit Diethylether (3 × 50 ml) extrahiert. Die organische Phase wurde mit Wasser (30 ml) gewaschen, über Natriumsulfat getrocknet und eingeengt. Der Rückstand war ein gelber Feststoff (394 mg, quantitativ). ¹³C-NMR (101 MHz, CDCl₃, δ ppm): 14.0, 23.7, 26.4, 28.0, 28.2, 29.4, 31.50, 31.57, 37.3, 42.0, 42.3, 44.1, 56.1, 104.0, 110,3, 110,5, 118.1, 118.2, 118.3, 118.5, 120.9, 121.2, 129.2, 129.3, 135.1, 135.5, 138.8, 141.0

### Stufe 3:

### 1,1'-(2,2'-(2,2'-(4-Butyl-4(dimethylamino)cyclohexan-1,1-diyl)bis(1H-indol-3,2-diyl))bis(ethan-2,1-diyl)bis(3-phenylharnstoff) (Beispiel Nr. 33)

Das 2,2'-(2,2'-(4-Butyl-4(dimethylamino)cyclohexan-1,1-diyl)bis(1*H*-indol-3,2-diyl)diethylamin (120 mg, 0,24 mmol) wurde in abs. Acetonitril (25 ml) gelöst und tropfenweise mit Phenylisocyanat (0,063 ml, 69 mg, 0,58 mmol) versetzt. Nach einer Reaktionszeit von 5 h bei Raumtemperatur wurde die klare helle Lösung eingeengt. Das erhaltene Rohprodukt (gelber Feststoff, 175 mg) wurde chromatographisch aufgetrennt [Kieseigel 60 (30 g); Ethylacetat/Methanol 4 : 1 (300 ml]. Der Harnstoff wurde als beigefarbener Feststoff in einer Ausbeute von 54 % (96 mg) erhalten.
Beispiel Nr. 33: ¹³C-NMR (101 MHz, DMSO-D₆, δ ppm): 13.8, 22.8, 25.0, 25.6, 30.3, 31.1, 34.3, 37.3, 40.7, 66.9, 107.9, 111.1, 117.6, 117.9, 118.3, 120.4, 120.8, 127.9, 128.5, 129.1, 134.6, 139.1, 140.5, 155,2

### Beispiel Nr. 34

### Stufe 1:

### 1-Butyl-4,4-bis(5-fluor-3-methyl-1H-indol-2-yl)-N,N-dimethylcyclohexylamin (Beispiel Nr. 34)

5-Fluorskatol (670 mg, 4,49 mmol) wurde zusammen mit dem 2-(2-(2-(4-Butyl-4-dimethylaminocyclohex-1-enyl)-1*H*-indol-3-yl)-ethyl)isoindol-1,3-dion (700 mg, 1,49 mmol) in abs. Dichlormethan (5 ml) gelöst und mit Trifluormethansulfonsäure (0,175 ml, 1,974 mmol) versetzt. Der Ansatz wurde 10 d bei 24 °C gerührt. Der ausgefallene hellbeigefarbene Feststoff wurde durch Filtration abgetrennt und mit Dichlormethan (3 × 0,5 ml) gewaschen. Der Feststoff (1031 mg) wurde mit 1 N Natriumhydroxidlösung (20 ml) und Dichlormethan (20 ml) 10 min gerührt. Die Phasen wurden getrennt. Die wäßrige Phase wurde mit Dichlormethan (2 × 20 ml) extrahiert. Die vereinigten organischen Extrakte wurden über Na₂SO₄ getrocknet und anschließend eingeengt. Das Produkt über Säulenchromatographie gereinigt.
Beispiel Nr. 34: 91 mg, 12%, weißer Feststoff, Schmelzpunkt: 181-186 °C
¹³C-NMR (101 MHz, CDCl₃, δ ppm): 9.28, 10.32, 14.14, 23.74, 26.58, 29.79, 31.56, 37.30, 42.55, 55.98, 102.75, 102.98, 103.11, 103.33, 106.79, 108.61, 108.65, 109.16, 109.42, 109.70, 109.96, 110.91, 111.02, 111.15, 111.24, 130.26, 130.67, 130.75, 130.83, 139.94, 131.03, 138.30, 140.72, 156.58, 156.74, 158.91, 159.08 795 mg (ca. 60 %) als gelber Feststoff, Schmelzpunkt: 103-108 °C
¹³C-NMR (101 MHz, CDCl₃, δ ppm, als Gemisch): 9.6, 14.1, 16.4, 23.7, 24.6, 26.6, 29.9, 30.2, 31.6, 31.7, 37.3, 38.3, 42.6, 42.8, 45.5, 56.0, 64.8, 102.5, 102.8, 103.3, 103.6, 106.45, 106.49, 106.73, 106.77, 109.0, 109.17, 109.23, 109.42, 109.56, 109.69, 109.74, 109.94, 110.20, 110.75, 110.91, 111.00, 111.16, 111.26, 111.35, 113.56, 113.79, 118.3, 119.6, 121.9, 123.1, 129.6, 130.24, 130.37, 130.74, 13.84, 130.94, 131.7, 132.1, 133.9, 134.4, 136.3, 137.3, 140.7, 141.1, 145.7, 156.53, 156.56, 158.85. 158.88, 168.4

### Besispiel Nr. 35

### Stufe 1:

### 4-(3-(2-Aminoethyl)-1H-indol-2-yl)-1-butyl-4-(5-fluor-3-methyl-1H-indol-2-yl)-N,N-cyclohexylamin (Beispiel Nr. 35, ein Diastereomer)

Das (710 mg, ca. 0,7 mmol) und Hydrazinhydrat (2 ml, 41,2 mmol) wurden in Methanol (20 ml) gelöst und dann 2 h unter Rückfluß gekocht. Zur Aufarbeitung wurde der Ansatz mit 1 N NaOH (50 ml) verdünnt und mit Dichlormethan (4 × 15 ml) extrahiert. Die vereinigten organischen Phasen wurden über Na₂SO₄ getrocknet und anschließend bis zur Trockne eingeengt. Das erhaltene Rohprodukt (weißer Feststoff, 583 mg) wurde säulenchromatographisch gereinigt [Kieselgel 60 (20 g); Cyclohexan/Ethylacetat 4 : 1 (250 ml), Ethylacetat (500 ml), Methanol/30-proz. Ammoniak 9 : 1 (250 ml)]. Es wurde eins von zwei möglichen Diastereoisomeren (343 mg, ca. 80 %, Schmp. 145-150 °C) als weißer Feststoff gewonnen.
Beispiel Nr. 35: ¹³C-NMR (101 MHz, CD₃OD, δ ppm): 9.46, 14.46, 24.72, 27.50, 29.52, 29.86, 32.10, 32.29, 37.99, 42.45, 42.72, 57.98, 102.88, 103.12, 107.84, 109.38, 109.65, 109.89, 111.98, 112.29, 112.39, 118.77, 119.69, 121.77, 130.83, 132.63, 131.72, 132.57, 136.33, 140.47, 142.57, 157,83, 160.13

### Beispiel Nr. 36 und Beispiel Nr. 37

### Stufe 1:

### N,N-Dimethyl-1-phenyl-4-(3-(2-pyridin-4-yl)ethyl)-1H-indol-2-yl)cyclohex-3-enamin

3-(2-Pyridin-4-ylethyl)-1H-indol (667 mg, 3 mmol, Synthese vgl. WO2008009415, Indolbaustein Ind-14) wurde zusammen mit dem 4-(Dimethylamino)-4-phenylcyclohexanon (652 mg, 3 mmol) in abs. Dichlormethan (45 ml) gelöst und mit Trifluormethansulfonsäure (0,553 ml, 6,3 mmol) versetzt. Der Ansatz wurde 64 h bei RT gerührt, wobei ein braunes Öl ausfiel. Zur Aufarbeitung wurde die Reaktionslösung mit 1N NaOH (10 ml) und THF (10 ml) versetzt. Das Gemisch wurde weitere 60 min gerührt. Nach Trennung der Phasen wurde die wäßrige Phase mit Dichlormethan (3 x 10 ml) extrahiert. Die vereinigten organischen Extrakte wurden über Na₂SO₄ getrocknet und anschließend eingeengt. Das erhaltene Rohprodukt (1,34 g) wurde säulenchromatographisch gereinigt [Kieselgel 60 (100 g); Ethylacetat/Methanol (20 : 1, 500 ml), Ethylacetat/Methanol (5 : 1, 870 ml), Ethylacetat/Methanol (2 : 1, 320 ml), Ethylacetat/Methanol (1 : 2, 550 ml)]. Die Verbindung wurde als farbloser Feststoff (339 mg, 27 %, Schmp.: 193-198 °C) erhalten.

### Stufe 2:

### N,N-Dimethyl-4-(3-methyl-1H-indol-2-yl)-1-phenyl-4-(3-(2-pyridin-4-yl)ethyl)-1H-indol-2-yl)cyclohexylamin (Beispiel Nr. 36, polares Diastereomer und Beispiel Nr. 37, unpolares Diastereomer)

Das *N,N*-Dimethyl-1-phenyl-4-(3-(2-pyridin-4-yl)ethyl)-1*H*-indol-2-yl)cyclohex-3-enamin (840 mg, 1,99 mmol) und 3-Methyl-1H-indol (651 mg, 4,97 mmol) wurden in abs. Dichlormethan (90 ml) gelöst und mit Trifluormethansulfonsäure (0,373 ml, 597 mg, 3,98 mmol) versetzt. Nach einer Reaktionszeit von 20 d bei Raumtemperatur wurde die dunkelbraune Reaktionsmischung mit Wasser (30 ml) und 1 N Natronlauge (5 ml) versetzt und 1 h gerührt. Die Phasen wurden getrennt. Die wäßrige Phase wurde mit Dichlormethan (30 ml) extrahiert. Die vereinigten organischen Phasen wurden mit Natriumsulfat getrocknet und eingeengt. Das erhaltene Rohprodukt (braunes Öl, 1,39 g) wurde chromatographisch aufgetrennt [Kieselgel 60 (110 g); Ethylacetat/Cyclohexan 4 : 1 (1000 ml), Ethylacetat (600 ml), Ethylacetat/Methanol 4 : 1 (1000 ml)]. Die unpolarere Bisindolverbindung wurde als gelber Feststoff in einer Ausbeute von 39 % (423 mg) erhalten. Der Feststoff wurde in Ethanol (4 ml) aufgenommen und 30 min auf 80 °C erwärmt. Nach Filtration des zurückgebliebenen Feststoffes wurde das unpolarere Bisindol als farbloser Feststoff in einer Ausbeute von 20 % (220 mg) mit einem Schmelzpunkt von 273-276 °C erhalten. Das polarere Bisindol wurde als leicht verunreinigter beigefarbener Feststoff in einer Ausbeute von 7 % (74 mg) mit einem Schmelzpunkt von 230-235 °C gewonnen.
Beispiel Nr. 36: ¹³C-NMR (101 MHz, DMSO-D₆, δ ppm, polareres Diastereoisomer): 9.2, 26.3, 29.9, 31.4, 34.8, 37.7, 105.3, 109.1, 111.1, 111.2, 117.3, 118.2, 120.1, 123.6, 127.6, 128.5, 129.5, 134.3, 140.4, 149.0, 150.5
Beispiel Nr. 37: ¹³C-NMR (101 MHz, DMSO-D₆, δ ppm, unpolareres Diastereoisomer): 9.3, 26.4, 29.9, 31.0, 34.9, 37.7, 105.4, 109.1, 111.0, 111.3, 117.1, 117.4, 118.1, 118.2, 120.0, 120.1, 123.6, 126.9, 127.5, 128.6, 129.4, 134.3, 134.5, 138.9, 149.0, 150.6,

### Beispiel Nr. 38

### Stufe 1:

### (Phenyl-2-(2-(4-butyl-4-(dimethylamino)-1-(5-fluor-3-methyl-1H-indol-2-yl)cyclohexyl)-1H-indol-3-yl)ethylcarbamat (Beispiel Nr. 38, eins von zwei möglichen Diastereoisomeren)

Das 4-(3-(2-Aminoethyl)-1*H*-indol-2-yl)-1-butyl-4-(5-fluor-3-methyl-1*H*-indol-2-yl)-*N,N-*cyclohexylamin (Beispiel Nr. 35) (120 mg, 0,246 mmol, eins von von zwei möglichen Disstereoisomeren) wurde in Dichlormethan (5 ml) mit Chloramelsensäurephenylester (0,045 ml, 0,359 mmol) versetzt. Nach einer Reaktionszeit von 5 h bei 24 °C wurde die Reaktionsmischung mit Dichlormethan (20 ml) verdünnt und mit 1N Natronlauge (4 × 10 ml) gewaschen. Die organische Phase wurde mit Na₂SO₄ getrocknet und dann filtriert. Die flüchtigen Bestandteile des Filtrates wurden im Vakuum vollständig entfernt. Der Rückstand (farbloses Öl, 172 mg) wurde in n-Hexan (5 ml) suspendiert. Der Ansatz wurde 2 h bei 24 °C gerührt. Das kristalline Produkt wurde filtriert, mit *n*-Hexan (2 × 0,5 ml) gewaschen und getrocknet. Der Carbamidsäureester wurde in einer Ausbeute von 111 mg (74 %) als weißer Feststoff (Schmelzpunkt 101-105 °C, eins von von zwei möglichen Diastereoisomeren) erhalten.
Beispiel Nr. 38: ¹³C-NMR (101 MHz, CDCl₃, δ ppm): 8.8, 13.9, 23.1, 25.3, 26.1, 29.1, 30.1, 31.2, 37.2, 40.9, 41.3, 55.35, 101.7, 101.9, 105.1, 107.9, 111.2 111.79, 111.89, 117.6, 118.2, 120.3, 121.2, 121.7, 124.8, 129.1, 129.2, 129.5, 129.8, 129.9, 130.8, 134.7, 138.3, 142.8, 151.0, 154.2, 155.5, 157.8

### Beispiel Nr. 39

### Stufe 1:

### 1-(2-(2-(4-Butyl-4-(dimethylamino)-1-(5-fluor-3-methyl-1H-indol-2-yl)cyclohexyl)-1H-indol-3-yl)ethyl)-3-phenylharstoff (Beispiel Nr. 39, eins von zwei möglichen Diastereoisomeren)

Das 4-(3-(2-Aminoethyl)-1*H*-indol-2-yl)-1-butyl-4-(5-fluor-3-methyl-1*H*-indol-2-yl)-*N,N-*cyclohexylamin (Beispiel Nr. 35) (120 mg, 0,246 mmol, eins von von zwei möglichen Diastereoisomeren) wurde in Acetonitril (2,4 ml) mit Phenylisocyanat (0,040 ml, 0,368 mmol) versetzt. Nach einer Reaktionszeit von 5 h bei 23 °C wurde die Reaktionsmischung mit 1 N Natronlauge (15 ml) verdünnt und mit Dichlormethan (4 × 10 ml) extrahiert. Die vereinigten organischen Phasen wurden mit Na₂SO₄ getrocknet und dann filtriert. Die flüchtigen Bestandteile des Filtrates wurden im Vakuum vollständig entfernt. Der Rückstand (farbloses Öl, 160 mg) wurde säulenchromatographisch gereinigt [Kieselgel 60 (20 g); Cyclohexan/Ethylacetat 2 : 1 (300 ml), Ethylacetat/Methanol 1 : 1 (300 ml)]. Das Produkt wurde in einer Ausbeute von 117 mg (78 %) als weißer Feststoff (Schmelzpunkt 127-132 °C, eins von von zwei möglichen Diastereoisomeren) erhalten.
Beispiel Nr. 39:¹³C-NMR (101 MHz, CDCl₃, δ ppm): 9.5, 14.0, 23.7, 26.0, 26.5, 29.5, 30.3, 31.6, 37.3, 40.6, 42.3, 56.0, 102.7, 102.9,106.7, 109.4, 109.6, 110.1, 110.9, 111.4, 111.5, 118.5, 119.7, 120.8, 121.8, 123.6, 129.0, 129.6, 130.4, 130.6, 134.5, 137.5, 138.4, 141.6, 155.6, 156.6, 159.0

### Beispiel Nr. 40

### Stufe 1:

### N,N-Dimethyl-1-phenyl-4-(3-(2-(pyridin-4-yl)-ethyl)-1H-indol-2-yl)-4-(thiophen-2-yl)-cyclohexylamin (Beispiel Nr. 40, Diastereoisomerengemisch)

3-(2-Pyridin-4-ylethyl)-1H-indol (Synthese vgl. WO2008009415, Indolbaustein Ind-14) (444 mg, 2 mmol) wurde unter Feuchtigkeitsausschluß zusammen mit dem polareren 4-(Dimethylamino)-4-phenyl-1-(thiophen-2-yl)cyclohexanol (444 mg, 1 mmol, AS 05710) in trockenem Dichlormethan (30 ml) vorgelegt und schnell mit Trifluormethansulfonsäuretrimethylsilylester (0,54 ml, 3 mmol) versetzt. Der Ansatz wurde bei RT 24 h gerührt. - Zur Aufarbeitung des Ansatzes wurde die Mischung mit 2N Natronlauge (10 ml) versetzt und 15 min gerührt. Die wäßrige Phase wurde mit Dichlormethan (2 × 30 ml) extrahiert. Die vereinigten organischen Phasen wurden nach dem Trocknen (Na₂SO₄) eingeengt, wobei ein braunes Öl (560 mg) erhalten wurde. Nach chromatographischer Reinigung des Rückstandes an Kieselgel 60 (50 g) mit Ethylacetat/Cyclohexan 1 : 1 (700 ml) konnte das Diastereoisomerengemisch in einer Ausbeute von 95 mg (19 %) als Feststoff (Schmelzpunkt: 54-60 °C) erhalten werden.
Beispiel Nr. 40: ¹³C NMR (101 MHz, CDCl₃, δ ppm): 26.0, 26.3, 29.7, 30.1, 32.8, 33.8, 35.6, 36.3, 37..3, 37.938.1, 38.3, 43.4, 59.6, 61.5, 110.7, 112.8, 113.4, 118.0, 1187, 118.7, 119.2, 121.0, 121.5, 123.9, 124.0, 126.5, 126.6, 126.8, 126.9, 127.0, 127.2, 127.1, 127.6, 127.8, 127.9, 129.2, 129.4, 135.4, 149.5, 149.6, 151.1, 151.5

### Untersuchungen zur Wirksamkeit der erfindungsgemäßen Verbindungen

### Messung der ORL1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel IIC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### Messung der kappa-Bindung

Die Bestimmung erfolgte in einem homogenen Ansatz in Mikrotiterplatten. Hierzu wurden Verdünnungsreihen der jeweils zu prüfenden Substanzen mit einer Rezeptormembranpräparation (7 µg Protein pro 250 µl Inkubationsansatz) von CHO-K1-Zellen, welche den humanen κ-Opiatrezeptor exprimieren in Gegenwart von 1 nmol/l des radioaktiven Liganden [³H]-Cl-977 sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 100 µmol/l Naloxon zugegeben. Nach Beendigung der neunzigminütigen Inkubationszeit wurden die Mikrotiterplatten für 20 Minuten bei 500 Umdrehungen/Minute abzentrifugiert und die Radioaktivität in einem ß-Counter (Microbeta-Trilux 1450, Firma PerkinElmer Wallac, Freiburg, Deutschland) vermessen. Es wurde die prozentuale Verdrängung des radioaktiven Liganden aus seiner Bindung zum humanen κ-Opiatrezeptor bei einer Konzentration der Prüfsubstanzen von 1 µmol/l bestimmt und als prozentuale Hemmung (%Hemmung) der spezifischen Bindung angegeben. Ausgehend von der prozentualen Verdrängung durch unterschiedliche Konzentrationen der zu prüfenden Verbindungen können IC₅₀ Hemmkonzentrationen berechnet werden, die eine 50-prozentige Verdrängung des radioaktiven Liganden bewirken. Durch Umrechnung mittels der Cheng-Prusoff-Beziehung können daraus die Kᵢ-Werte für die Prüfsubstanzen berechnet werden.

### Analgesieprüfung im Tail-Flick-Test an der Ratte

Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941)) untersucht. Dazu wurden weibliche Sprague Dawley Ratten mit einem Gewicht zwischen 130 und 190 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel: [(T₁ - T₀)/(T₂ - T₀)] x 100. Dabei ist die T₀ die Latenzzeit vor und T₁ die Latenzzeit nach Substanzapplikation, T₂ ist die maximale Expositionszeit (12 sec).

Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die ED₅₀-Werte mit Hilfe der Regressionsanalyse bestimmt. Die ED₅₀-Berechnung erfolgte im Wirkmaximum, 20 Minuten nach intravenöser Substanzgabe.

### Nephelometrische Löslichkeitsuntersuchung (Phosphatpuffer pH 7.4):

Diese Methode untersucht die Löslichkeit einer Substanz bei festgelegten Konzentrationen (1 µM, 3 µM, 10 µM, 30 µM und 100 µM) in 10 mM Phosphatpufferlösung bei pH 7.4. Initial wird eine 10 mM Lösung der Substanzen in DMSO benötigt, aus der 100-fach Stammlösungen der oben genannten Konzentrationslevel wiederum in DMSO hergestellt werden, die finale DMSO Konzentration im Versuchsansatz beträgt 1 % (v/v). Das Experiment wird in mehrfach Bestimmung durchgeführt. Nach Zugabe der DMSO Stammlösungen zum Puffer, wird der Ansatz 2h bei 37°C inkubiert, bevor eine Absorptionsbestimmung bei 620 nm stattfindet. Steigt die Absorption der Proben über die der reinen Puffer/DMSO Lösung an, so gilt dies als Indikator für eine Präzipitatbildung. Die untere Löslichkeitsgrenze ("lower bound") ist die Konzentration, die derjenigen mit erster Präzipitatbildung vorangegangen ist (z.B. 3 µM, wenn Präzipitatbildung bei 10 µM detektiert wurde).

Die Ergebnisse sind in nachfolgender Tabelle zusammengefasst:

| Nr. | % Hemmung (ORL1) [1 µM] | Ki (ORL1) Mittel [µM] | % Hemmung (µ) [1 µM] | Ki ( µ ) Mittel [µM] | Tail flick Ratte, i.v |
|---|---|---|---|---|---|
| 1 | 9 | 1,333 | 60 | 0,557 | nd |
| 2 | 89 | 0,167 | 92 | 0,097 | nd |
| 3 | 19 | 0,730 | 96 | 0,010 | nd |
| 4 | 93 | 0,029 | 73 | 0,143 | nd |
| 5 | 18 | 1,077 | 77 | 0,093 | nd |
| 6 | 70 | nd | 80 | nd | nd |
| 7 | 89 | 0,009 | 83 | 0,042 | 30% MPE bei 100 µg/kg |
| 8 | 31 | 0,460 | 46 | 0,590 | nd |
| 9 | 82 | 0,020 | 76 | 0,150 | nd |
| 10 | 8 | 0,283 | 3 | 0,780 | nd |
| 11 | 77 | 0,043 | 83,5 | 0,06 | nd |
| 12 | 95 | 0,013 | 100 | 0,005 | nd |
| 13 | 96 | 0,027 | 103 | 0,0009 | nd |
| 14 | 98 | 0,001 | 96 | 0,0005 | nd |
| 15 | 96 | 0,007 | 99 | 0,006 | nd |
| 16 | 84 | 0,027 | 97 | 0,011 | nd |
| 17 | 99 | nd | 99 | nd | nd |
| 18 | 80 | 0,039 | 74 | 0,078 | nd |
| 19 | 88 | 0,023 | nd | 0,022 | nd |
| 20 | 87 | 0,029 | nd | 0,012 | nd |
| 21 | 56 | 0,265 | nd | 0,47 | nd |
| 22 | 93 | 0,011 | nd | 0,003 | nd |
| 23 | 98 | 0,001 | nd | 0,001 | nd |
| 24 | 86 | nd | nd | nd | nd |
| 25 | 31 | 0,45 | nd | 0,081 | nd |
| 26 | 30 | 0,61 | nd | 0,165 | nd |
| 27 | 72 | nd | nd | nd | nd |
| 28 | 82 | nd | nd | nd | nd |
| 29 | 97 | nd | nd | nd | nd |
| 31 | 71 | 0,088 | nd | 0,028 | nd |
| 32 | 98 | 0,013 | 100 | 0,0043 | nd |
| 33 | 97 | 0,012 | 100 | 0,011 | nd |
| 34 | 56 | 0,119 | 76 | 0,21 | nd |
| 35 | 95 | nd | 98 | nd | nd |
| 36 | 61 | nd | 73 | nd | nd |
| 37 | 94 | 0,018 | 97 | 0,061 | nd |
| 38 | 83 | nd | 90 | nd | nd |
| 39 | 86 | 0,031 | 95 | 0,029 | nd |
| 40 | 82 | 0,021 | 69 | 0,717 | nd |

Die erfindungsgemäßen Verbindungen mit Q = (Hetero-)aryl vom Typ E wurden mit den ansonsten entsprechend substituierten Verbindungen vom Typ E verglichen, bei denen Q = H ist:

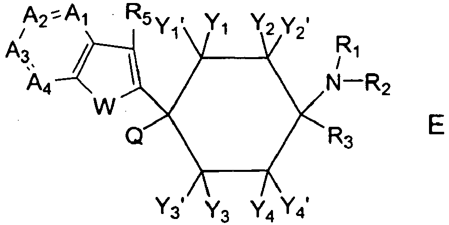

Die Ergebnisse sind in nachfolgenden Tabellen zusammengefasst:

| Nr. | Q | Ki (µ)/ Ki (ORL1) | Ki (kappa)/ Ki (ORL1) | Ki (ORL1) Mittel [µM] | Ki (µ) Mittel [µM] | Ki (kappa) Mittel [µM] |
|---|---|---|---|---|---|---|
| Bsp.-4 | 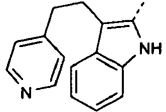 | 4.9 | 5.5 | 0,029 | 0,143 | 0,160 |
| Vergleich-1: Bsp. Nr. 304 in WO2008009415 | -H- | 1.2 | 1.0 | 0,153 | 0,197 | 0,153 |
| Bsp.-7 | 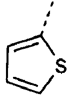 | 4.7 | 15.2 | 0,009 | 0,042 | 0,137 |
| Vergleich-2: Bsp. Nr. 17 in WO2008009415 | -H- | 0.4 | 1.7 | 0,0009 | 0,0004 | 0,0012 |
| Vergleich-3: Bsp. Nr. 18 in WO2008009415 | -H- | 0.9 | -- | 0,0140 | 0,0130 | n.d.* |
| Bsp.-9 | 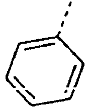 | 7.5 | 29 | 0,020 | 0,150 | 0,580 |
| Vergleich-4: Bsp. Nr. 17 in WO2008009415 | -H- | 0.4 | 1.7 | 0,0009 | 0,0004 | 0,0012 |
| Vergleich-5: Bsp. Nr. 18 in WO2008009415 | -H- | 0.9 | -- | 0,0140 | 0,0130 | n.d.* |
| Bsp.-10 | 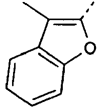 | 2.8 | -- | 0,283 | 0,780 | 4% Hemmung bei [1 µM] |
| Vergleich-6 Bsp. Nr. 226 in WO2008009415 | -H- | 0.1 | -- | 0,039 | 0,005 | 31% Hemmung bei [1 µM] |

| | | | | | | |
|---|---|---|---|---|---|---|
| n.d.- nicht bestimmt | | | | | | |

Wie die vorstehenden Vergleichsdaten belegen, weisen die erfindungsgemäßen Verbindungen (Q= Aryl/Heteroaryl) im Vergleich zu den strukturell ähnlichen Substanzen (Q= -H) eine höhere Selektivität gegenüber dem kappa-Opioidrezeptor (definiert als 1/[K_{i(ORL1)}/ Kᵢ₍ₖₐₚₚₐ₎]) auf. Darüber hinaus weisen die erfindungsgemäßen Substanzen bei günstigem Verhältnis der ORL1/µ-Affinität auch eine höhere Selektivität gegenüber dem µ-Opioidrezeptor (definiert als 1/[K_{i(ORL1)}/K_{i(µ)}]) auf.

Die erfindungsgemäßen Verbindungen vom Typ 1 mit Q = thienyl (Bsp. 7) wurden mit entsprechenden Verbindungen vom Typ 1 mit Q = H (V-7 bis V-9) verglichen:

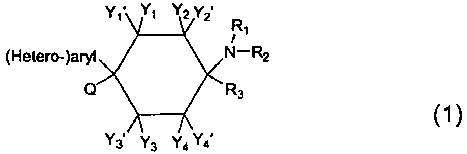

| Bsp. | Q | (Hetero-)aryl | R₁ | Nephelometrie (lower bound) µM |
|---|---|---|---|---|
| 7 | 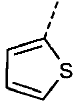 | 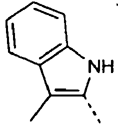 | Me | 10 |
| Vergleich-2: Bsp. Nr. 17 in WO2008009415 | H | 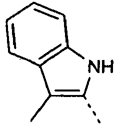 | Me | 1 |
| Vergleich-7: Bsp. Nr. 118 in WO2008009415 | H | 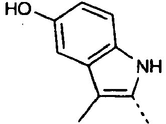 | Me | <1 |
| Vergleich-8: Bsp. Nr. 259 in WO2008009415 | H | 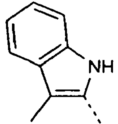 | H | 3 |

Wie der vorstehende Vergleich belegt, weist die erfindungsgemäße Verbindung aus Beispiel 7 im Vergleich zu strukturell ähnlichen Verbindungen (Q=H) eine bessere Löslichkeit in wässrigen Medien auf, was insbesondere Vorteile im Hinblick auf die Resorptionseigenschaften und/oder die Bioverfügbarkeit mit sich bringen sollte.

## Patentansprüche

1. Verbindung der allgemeinen Formel (3) 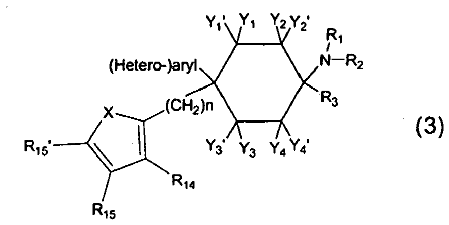 worin
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂;
oder Y₁ und Y₁', oder Y₂ und Y₂', oder Y₃ und Y₃', oder Y₄ und Y₄' gemeinsam für =O stehen;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₄CH₂CH₂- oder -(CH₂)₃₋₆- stehen;
R₃ für -R₀ steht;
R₄ für -H, -R₀ oder -C(=O)R₀ steht;
X für -O-, -S-, -NR₁₆-, -CR₁₇=CR₁₈, -CR₁₇=N- oder -N=CR₁₈- steht;
R₁₄, R₁₅, R₁₅', R₁₆, R₁₇ und R₁₈ jeweils unabhängig voneinander für -H, -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁. ₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂ stehen; oder R₁₄ und R₁₅, oder R₁₅ und R₁₅', oder R₁₅' und R₁₇ zusammen einen fünf- oder sechsgliedrigen, gesättigten, partiell ungesättigten oder aromatischen Ring bilden, welcher ggf. ein oder zwei Heteroringatome umfasst unabhängig voneinander ausgewählt aus N, S und O; und welcher unsubstituiert oder ein- oder mehrfach substituiert ist mit Substituenten unabhängig voneinander ausgewählt aus der Gruppe bestehend aus -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)-R₀, -NHC(=O)OR₀, -NH-C(=O)NH₂, -NHC(=O)NHR₀ und -NHC(=O)N(R₀)₂; bevorzugter -F, -Cl, -Br, -I, -CF₃, -CN und -NO₂;
n für 0, 1 oder 2 steht; und
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist;
wobei in Bezug auf "Aliphat" und "Cycloaliphat" unter "ein- oder mehrfach substituiert" die ein- oder mehrfache Substitution eines oder mehrerer Wasserstoffatome durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -NHS(=O)₁₋₂R₀, -Si(R₀)₃ und -PO(OR₀)₂ verstanden wird;
"(Hetero-)aryl" für Heteroaryl oder Aryl steht;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂, -C₁₋₈Aliphat-NHC(=O)R₀, -C₁₋₈Aliphat-NHC(=O)OR₀, -C₁₋₈Aliphat-NHC(=O)NHR₀, -C₁₋₈Aliphat-NHC(=O)N(R₀)₂ und -C₁₋₈Aliphat-NHS(=O)₁₋₂R₀ verstanden wird; wobei ggf. vorhandene N-Ringatome jeweils oxidiert sein können;
in Form eines einzelnen Stereoisomers oder deren Mischung, der freien Verbindungen und/oder ihrer physiologisch verträglichen Salze.

2. Verbindung nach Anspruch 1, welche die allgemeine Formel (4) 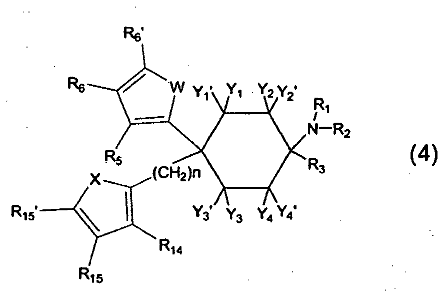 aufweist.

3. Verbindung nach Anspruch 2, welche die allgemeine Formel (4.8.1) 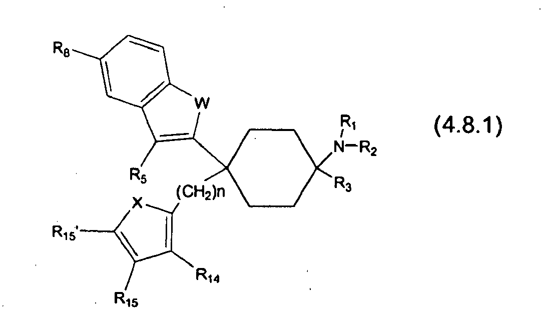 aufweist.

4. Verbindung nach Anspruch 3, wobei
W für -O- oder -NR₁₁- steht;
X für -O-, -NR₁₆- oder -CR₁₇=CR₁₈- steht;
n für 0 oder 1 steht;
R₁ für -CH₃ steht;
R₂ für -H oder -CH₃ steht; oder
R₁ und R₂ gemeinsam einen Ring bilden und für -(CH₂)₃₋₄- stehen;
R₃ für -C₁₋₈-Aliphat, -Aryl oder Heteroaryl steht, wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, -OH, -OCH₃, -OC₂H₅ und -N(CH₃)₂;
R₅ und R₁₄ unabhängig voneinander für -H, -F, -C₁₋₈-Aliphat, -C₁₋₈-Aliphat-Aryl oder -C₁₋₈-Aliphat-Heteroaryl stehen;
R₈ für -F, -Cl, -Br, -I, -CF₃, -CN oder -NO₂ steht;
R₁₁ für -H steht;
R₁₄, R₁₅ und R₁₅' unabhängig voneinander für -H, -F, -Cl, -Br, -CN, -CH₃, -C₂H₅, -NH₂, -NO₂. -SH, -CF₃, -OH, -OCH₃, -OC₂H₅ oder -N(CH₃)₂ stehen; oder R₁₅ und R₁₅' gemeinsam einen sechsgliedrigen, gesättigten, partiell ungesättigten oder aromatischen Ring bilden, welcher ggf. ein oder zwei Heteroringatome umfassen kann, die unabhängig voneinander ausgewählt sind aus N, S und O; wobei dieser gebildete Ring unsubstituiert oder ein- oder mehrfach substituiert sein kann, wobei die Substituenten unabhängig voneinander ausgewählt sind aus der Gruppe bestehend aus -F, -Cl, -Br, -I, -CF₃, -CN und -NO₂.
R₁₆ für -H steht; und
R₁₇ und R₁₈ unabhängig voneinander für -H oder -F stehen.

5. Verbindung nach Anspruch 1, ausgewählt aus der Gruppe bestehend aus
• 1-Butyl-N,N-dimethyl-4,4-bis(3-methyl-5-(trifluormethyl)-1 H-indol-2-yl)cyclohexan-amin;
• 1-Butyl-4,4-bis(5-fluor-3-methyl-1H-indol-2-yl)-N,N-dimethylcyclohexanamin:
• 1-Benzyl-N,N-dimethyl-4,4-bis(3-(2-(pyridin-4-yl)ethyl)-1H-indol-2-yl)cyclohexan-amin;
• N,N-Dimethyl-4,4-bis(3-(2-(pyridin-4-yl)ethyl)-1H-indol-2-yl)-1-(thiophen-2-yl)cyclo-hexanamin;
• 2,2'-(4-Butyl-4-(pyrrolidin-1-yl)cyclohexan-1,1-diyl)bis(3-(2-(pyridin-4-yl)ethyl)-1H-indol);
• N-Methyl-1-phenyl-4,4-bis(3-(2-(pyridin-4-yl)ethyl)-1H-indol-2-yl)cyclohexanamin;
• N,N-Dimethyl-4-(3-methyl-1 H-indol-2-yl)-1-phenyl-4-(thiophen-2-yl)cyclohexan-amin;
• N,N-Dimethyl-4-(3-methyl-1 H-indol-2-yl)-1,4-diphenylcyclohexanamin;
• N,N-Dimethyl-4,4-bis(3-methylbenzofuran-2-yl)-1-phenylcyclohexanamin;
• N,N-Dimethyl-4,4-bis(3-methyl-1 H-indol-2-yl)-1-phenylcyclohexanamin; 2-hydroxy-propan-1,2,3-tricarboxylat;
• 1-Butyl-N,N-dimethyl-4,4-bis(3-(2-(pyridin-4-yl)ethyl)-1H-indol-2-yl)cyclohexan-amin; 2-hydroxypropan-1,2,3-tricarboxylat;
• N,N,4-Trimethyl-4-(3-methyl-1H-indol-2-yl)-1-phenylcyclohexanamin;
• 4-Benzyl-N,N-dimethyl-4-(3-methyl-1H-indol-2-yl)-1-phenylcyclohexanamin;
• Dimethyl 2,2'-(2,2'-(4-butyl-4-(dimethylamino)cyclohexan-1,1-diyl)bis(1H-indol-3,2-diyl))diacetat;
• 2,2'-(2,2'-(4-Bbutyl-4-(dimethylamino)cyclohexan-1,1-diyl)bis(1H-indol-3,2-diyl))-diethanol;
• 1-Butyl-N,N-dimethyl-4-(3-methyl-1H-indol-2-yl)-4-(3-(2-pyridin-4-yl)ethyl)-1 H-indol-2-yl)cyclohexylamin;
• 1-Butyl-4,4-bis-(3-(2-(3,4-dihydro-1H-isochinolin-2-yl)ethyl)-1H-indol-2-yl)-N,N-dimethylcyclohexylamin;
• 4-(4-Methoxyphenyl)-N,N-dimethyl-4-(3-methyl-1H-indol-2-yl)-1-phenylcyclohexyl-amin;
• 1-Butyl-N,N-dimethyl-4,4-bis-(3-methyl-1H-indol-2-yl)cyclohexylamin;
• 2-(2-(2-(4-Butyl-4-dimethylamino-1-(3-methyl-1H-indol-2-yl)cyclohexyl)-1H-indol-3-yl)ethyl)isoindolin-1,3-dion;
• 2-[2-[2-[4-Butyl-4-dimethylamino-1-[3-[2-(1,3-dioxo-2H-isoindol-2-yl)-ethyl]-1H-indol-2-yl]-cyclohexyl]-1H-indol-3-yl]-ethyl]-2H-isoindole-1,3-dion;
• 4-(Benzo[d][1,3]dioxol-5-yl)-N,N-dimethyl-4-(3-methyl-1H-indol-2-yl)-1-phenyl-cyclohexylamin;
• Dimethyl 3,3'-(2-(4-butyl-4-(dimethylamino)cyclohexan-1,1-diyl)bis(1H-indol-2,3-diyl)dipropanat;
• 4-(3-(2-Aminoethyl)-1H-indol-2-yl)-1-butyl-N,N-dimethyl-4-(3-methyl-1H-indol-2-yl)cyclohexylamin;
• 1-(3-Fluorphenyl)-N,N-dimethyl-4-(3-methyl-1H-indol-2-yl)-4-thiophen-2-ylcyclo-hexylamin;
• 1-Butyl-4,4-bis-(1,3-dimethyl-1H-indol-2-yl)-N,N-dimethylcyclohexylamin;
• 1-Butyl-4-(1,3-dimethyl-1H-indol-2-yl)-N,N-dimethyl-4-(3-methyl-1H-indol-2-yl)cyclohexylamin;
• 1-Benzyl-N,N-dimethyl-4-(3-methyl-1H-indol-2-yl)-4-(thiophen-2-yl)cyclohexylamin;
• 4-(3-Methoxyphenyl)-N,N-dimethyl-4-(3-methyl-1H-indol-2-yl)-1-phenylcyclohexyl-amin;
• N-(2-(2-(4-Butyl-4-(dimethylamino)-1-(3-methyl-1H-indol-2-yl)cyclohexyl)-1H-indol-3-yl)ethyl)cyclopentansulfonsäureamid;
• 1-(3-Fluorphenyl)-N,N-dimethyl-4-(3-methyl-1H-indol-2-yl)-4-thiophen-2-ylcyclo-hexylamin;
• N,N-Dimethyl-1-phenyl-4,4-bis-(3-(2-pyridin-4-yl)ethyl)-1H-indol-2-yl)cyclohexyl-amin;
• 1-(2-(2-(4-Butyl-4-(dimethylamino)-1-(3-methyl-1H-indol-2-yl)cyclohexyl)-1H-indol-3-yl)ethyl)-3-phenylharnstoff;
• 1,1'-(2,2'-(2,2'-(4-Butyl-4(dimethylamino)cyclohexan-1,1-diyl)bis(1H-indol-3,2-diyl))bis(ethan-2,1-diyl)bis(3-phenylharnstoff);
• 1-Butyl-4,4-bis(5-fluor-3-methyl-1H-indol-2-yl)-N,N-dimethylcyclohexylamin;
• 4-(3-(2-Aminoethyl)-1H-indol-2-yl)-1-butyl-4-(5-fluor-3-methyl-1H-indol-2-yl)-N,N-cyclohexylamin;
• (Phenyl-2-(2-(4-butyl-4-(dimethylamino)-1-(5-fluor-3-methyl-1H-indol-2-yl)cyclohexyl)-1H-indol-3-yl)ethylcarbamat;
• 1-(2-(2-(4-Butyl-4-(dimethylamino)-1-(5-fluor-3-methyl-1H-indol-2-yl)cyclohexyl)-1 H-indol-3-yl)ethyl)-3-phenylharstoff;
• N,N-Dimethyl-4-(3-methyl-1H-indol-2-yl)-1-phenyl-4-(3-(2-pyridin-4-yl)ethyl)-1H-indol-2-yl)cyclohexylamin;
• N,N-Dimethyl-1-phenyl-4-(3-(2-(pyridin-4-yl)-ethyl)-1H-indol-2-yl)-4-(thiophen-2-yl)-cyclohexylamin;
und deren physiologisch verträgliche Salze.

6. Arzneimittel enthaltend wenigstens eine Verbindung nach einem der Ansprüche 1 bis 5 in Form eines einzelnen Stereoisomers oder deren Mischung, der freien Verbindungen und/oder ihrer physiologisch verträglichen Salze, sowie ggf. geeignete Zusatz- und/oder Hilfsstoffe und/oder gegebenenfalls weiterer Wirkstoffe.

7. Verbindung nach einem der Ansprüche 1 bis 5 in Form eines einzelnen Stereoisomers oder deren Mischung, der freien Verbindung und/oder ihrer physiologisch verträglichen Salze, zur Anwendung bei der Behandlung von Schmerz.

8. Verbindung nach einem der Ansprüche 1 bis 5 in Form eines einzelnen Stereoisomers oder deren Mischung, der freien Verbindungen und/oder ihrer physiologisch verträglichen Salze und/oder Solvate zur Anwendung bei der Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei der Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Anwendung bei der Behandlung damit verbundener neurodegenerativer Erkrankungen, zur Anwendung bei der Behandlung von Entzugserscheinungen und/oder zur Reduzierung des Suchtpotentials von Opioiden.

## Claims

1. Compound of the general formula (3) 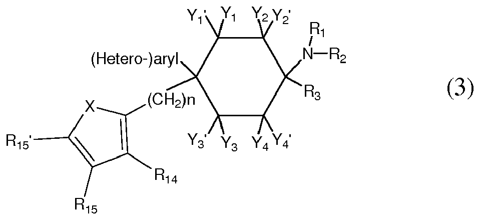 wherein
Y₁, Y₁', Y₂, Y₂', Y₃, Y₃', Y₄ und Y₄' are respectively selected independently of one another from the group comprising -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ and -NHC(=O)N(R₀)₂; or Y₁ und Y₁', or Y₂ and Y₂', or Y₃ and Y₃', or Y₄ and Y₄' jointly stand for =O;
R₀ respectively independently stands for -C₁₋₈-aliphatic, -C₃₋₁₂-cycloaliphatic, -aryl, - heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
R₁ and R₂, independently of one another, stand for -H or -R₀; or R₁ and R₂ together stand for -CH₂CH₂OCH₂CH₂-, -CH₂CH₂NR₄CH₂CH₂- or -(CH₂)₃₋₆-;
R₃ stands for -R₀;
R₄ stands for -H, -R₀ or -C(=O)R₀;
X stands for -O-, -S-, -NR₁₆-, -CR₁₇=CR₁₈-, -CR₁₇=N- or -N=CR₁₈-;
R₁₄, R₁₅, R₁₅', R₁₆, R₁₇ and R₁₈ respectively independently of one another stand for -H, -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SRₒ, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂;
or R₁₄ and R₁₅, or R₁₅ and R₁₅', or R₁₅' and R₁₇ together form a five- or six-membered, saturated, partially unsaturated or aromatic ring, which possibly comprises one or two hetero ring atoms selected independently of one another from N, S and O; and which is unsubstituted or mono- or polysubstituted with substituents selected independently of one another from the group comprising -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)-R₀, -NHC(=O)OR₀, -NH-C(=O)NH₂, -NHC(=O)NHR₀ and -NHC(=O)N(R₀)₂; more preferred -F, -Cl, -Br, -I, -CF₃, -CN and -NO₂;
n stands for 0, 1 or 2; and
wherein
"aliphatic" respectively is a branched or unbranched, saturated or a mono- or polyunsaturated, unsubstituted or mono- or polysubstituted, aliphatic hydrocarbon residue;
"cycloaliphatic" respectively is a saturated or a mono- or polyunsaturated, unsubstituted or mono- or polysubstituted, alicyclic, mono- or multicyclic hydrocarbon residue;
wherein with respect to "aliphatic" and "cycloaliphatic", "mono- or polysubstituted" is understood to mean the mono- or polysubstitution of one or more hydrogen atoms by substituents selected independently of one another from the group comprising aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -NHS(=O)₁₋₂R₀, -Si(R₀)₃ and -PO(OR₀)₂;
"(hetero-)aryl" stands for heteroaryl or aryl;
"aryl", respectively independently, stands for a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl;
"heteroaryl" stands for a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system;
wherein with respect to "aryl" and "heteroaryl", "mono- or polysubstituted" is understood to mean the mono- or polysubstitution of one or more hydrogen atoms of the ring system by substituents selected from the group comprising -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂, -C₁₋₈-aliphatic-NHC(=O)R₀, -C₁₋₈ -aliphatic-NHC(=O)OR₀, -C₁₋₈ -aliphatic-NHC(=O)NHR₀, -C₁₋₈ -aliphatic-NHC(=O)N(R₀)₂ and -C₁₋₈ -aliphatic-NHS(=O)₁₋₂R₀; wherein any N-ring atoms present can be respectively oxidised; in the form of a single stereoisomer or mixture thereof, the free compounds and/or their physiologically compatible salts.

2. Compound according to claim 1, which has the general formula (4)

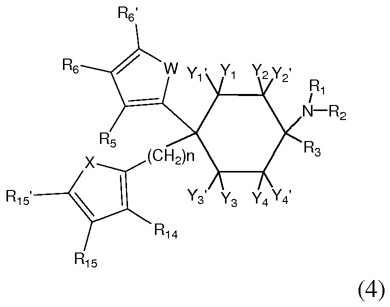

3. Compound according to claim 2, which has the general formula (4.8.1)

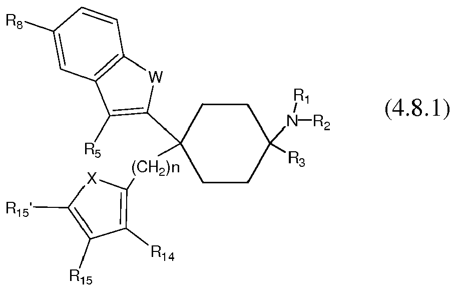

4. Compound according to claim 3, wherein
W stands for -O- or -NR₁₁-;
X stands for -O-, -NR₁₆- or -CR₁₇=CR₁₈-;
n stands for 0 or 1;
R₁ stands for -CH₃;
R₂ stands for -H or -CH₃; or
R₁ and R₂ jointly form a ring and stand for -(CH₂)₃₋₄-;
R₃ stands for -C₁₋₈-aliphatic, -aryl or heteroaryl, wherein these are unsubstituted or mono- or polysubstituted with substituents selected independently of one another from the group comprising -F, -Cl, -Br, -CN, -CH₃, -C₂H₅, -NH₂, -NO₂, -SH, -CF₃, -OH, -OCH₃, -OC₂H₅ and -N(CH₃)₂;
R₅ and R₁₄ independently of one another stand for -H, -F, -C₁₋₈-aliphatic, -C₁₋₈-aliphatic-aryl or -C₁₋₈-aliphatic-heteroaryl;
R₈ stands for -F, -Cl, -Br, -I, -CF₃, -CN or -NO₂;
R₁₁ stands for -H;
R₁₄, R₁₅ snd R₁₅' independently of one another stand for -H, -F, -Cl, -Br, -CN, -CH₃,-C₂H₅, -NH, -NO₂, -SH, -CF₃, -OH, -OCH₃, -OC₂H₅ or -N(CH₃)₂; or R₁₅ and R₁₅' jointly form a six-membered, saturated, partially unsaturated or aromatic ring, which can possibly comprise one or two hetero ring atoms, which are selected independently of one another from N, S und O; wherein this formed ring can be unsubstituted or mono- or polysubstituted, wherein the substituents are selected independently of one another from the group comprising -F, -Cl, -Br, -I, -CF₃, -CN and -NO₂.
R₁₆ stands for -H; and
R₁₇ and R₁₈ independently of one another stand for -H or -F.

5. Compound according to claim 1, selected from the group comprising
• 1-butyl-N,N-dimethyl-4,4-bis(3-methyl-5-(trifluoromethyl)-1H-indol-2-yl)cyclohexanamine; 2-hydroxypropane-1,2,3-tricarboxylate;
• 1-butyl-4,4-bis(5-fluoro-3-methyl-2H-indol-2-yl)-N,N-dimethylcyclohexanamine;
• 1-benzyl-N,N-dimethyl-4,4-bis(3-(2-(pyridin-4-yl)ethyl)-1H-indol-2-yl)cyclohexanamine; 2-hydroxypropane-1,2,3-tricarboxylate;
• N,N-dimethyl-4,4-bis(3-(2-(pyridin-4-yl)ethyl)-1H-indol-2-yl)-1-(thiophen-2-yl)-cyclohexanamine;
• 2,2'-(4-butyl-4-(pyrrolidin-1-yl)cyclohexane-1,1-diyl)bis(3-(2-(pyridin-4-yl)ethyl)-1H-indole);
• N-methyl-1-phenyl-4,4-bis(3-(2-(pyridin-4-yl)ethyl)-1H-indol-2-yl)cyclohexanamine;
• N,N-dimethyl-4-(3-methyl-1H-indol-2-yl)-1-phenyl-4-(thiophen-2-yl)cyclohexan-amine;
• N,N-dimethyl-4-(3-methyl-1H-indol-2-yl)-1,4-diphenylcyclohexanamine;
• N,N-dimethyl-4,4-bis(3-methylbenzofuran-2-yl)-1-phenylcyclohexanamine;
• N,N-dimethyl-4,4-bis(3-methyl-1H-indol-2-yl)-1-phenylcyclohexanamine; 2-hydroxypropane-1,2,3-tricarboxylate;
• 1-butyl-N,N-dimethyl-4,4-bis(3-(2-(pyridin-4-yl)ethyl)-1H-indol-2-yl)-cyclohexanamine; 2-hydroxypropane-1,2,3-tricarboxylate;
• N,N,4-trimethyl-4-(3-methyl-1H-indol-2-yl)-1-phenylcyclohexanamine;
• 4-benzyl-N,N-dimethyl-4-(3-methyl-1H-indol-2-yl)-1-phenylcyclohexanamine;
• dimethyl 2,2'-(2,2'-(4-butyl-4-(dimethylamino)cyclohexan-1,1-diyl)bis(1H-indol-3,2-diyl))diacetat e;
• 2,2'-(2,2'-(4-butyl-4-(dimethylamino)cyclohexan-1,1-diyl)bis(1H-indol-3,2-diyl))diethanol;
• 1-butyl-N,N-dimethyl-4-(3-methyl-1H-indol-2-yl)-4-(3-(2-pyridin-4-yl)ethyl)-1H-indol-2-yl)cyclohexylamine;
• 1-butyl-4,4-bis-(3-(2-(3,4-dihydro-1H-isoquinolin-2-yl)ethyl)-1H-indol-2-yl)-N,N-dimethylcyclohexylamine;
• 4-(4-methoxyphenyl)- N,N-dimethyl-4-(3-methyl-1H-indol-2-yl)-1-phenylcyclohexylamine;
• 1-butyl-N,N-dimethyl-4,4-bis-(3-methyl-1H-indol-2-yl)cyclohexylamine;
• 2-(2-(2-(4-butyl-4-dimethylamino-1-(3-methyl-1H-indol-2-yl)cyclohexyl)-1H-indol-3-yl)ethyl)isoindolin-1,3-dione
• 2-[2-[2-[4-butyl-4-dimethylamino-1-[3-[2-(1,3-dioxo-2H-isoindol-2-yl)-ethyl]-1H-indol-2-yl]-cyclohexyl]-1H-indol-3-yl]-ethyl]-2H-isoindole-1,3-dione;
• 4-(benzo[d][1,3]dioxol-5-yl)-N,N-dimethyl-4-(3-methyl-1H-indol-2-yl)-1-phenylcyclohexylamine;
• dimethyl 3,3'-(2-(4-butyl-4-(dimethylamino)cyclohexan-1,1-diyl)bis(1H-indol-2,3-diyl)dipropanate;
• 4-(3-(2-aminoethyl)-1H-indol-2-yl)-1-butyl-N,N-dimethyl-4-(3-methyl-1H-indol-2-yl)cyclohexylamine;
• 1-(3-fluorophenyl)-N,N-dimethyl-4-(3-methyl-1H-indol-2-yl)-4-thiophen-2-ylcyclohexylamine;
• 1-butyl-4,4-bis-(1,3-dimethyl-1H-indol-2-yl)-N,N-dimethylcyclohexylamine;
• 1-butyl-4-(1,3-dimethyl-1H-indol-2-yl)-N,N-dimethyl-4-(3-methyl-1H-indol-2-yl)cyclohexylamine;
• 1-benzyl-N,N-dimethyl-4-(3-methyl-1H-indol-2-yl)-4-(thiophen-2-yl)cyclohexylamine;
• 4-(3-methoxyphenyl)- N,N-dimethyl-4-(3-methyl-1H-indol-2-yl)-1-phenylcyclohexylamine;
• N-(2-(2-(4-butyl-4-(dimethylamino)-1-(3-methyl-1H-indol-2-yl)cyclohexyl)-1H-indol-3-yl)ethyl)cyclopentane sulphonamide;
• 1-(3-fluorophenyl)-N,N-dimethyl-4-(3-methyl-1H-indol-2-yl)-4-thiophen-2-ylcyclohexylamine;
• N,N-dimethyl-1-phenyl-4,4-bis-(3-(2-pyridin-4-yl)ethyl)-1H-indol-2-yl)cyclohexylamine;
• 1-(2-(2-(4-butyl-4-(dimethylamino)-1-(3-methyl-1H-indol-2-yl)cyclohexyl)-1H-indol-3-yl)ethyl)-3-phenylurea;
• 1,1'-(2,2'-(2,2'-(4-butyl-4(dimethylamino)cyclohexan-1,1-diyl)bis(1H-indol-3,2-diyl))bis(ethan-2,1-diyl)bis(3-phenylurea);
• 1-butyl-4,4-bis(5-fluoro-3-methyl-1H-indol-2-yl)-N,N-dimethylcyclohexylamine;
• 4-(3-(2-aminoethyl)-1H-indol-2-yl)-1-butyl-4-(5-fluoro-3-methyl-1H-indol-2-yl)-N,N-cyclohexylamine;
• (phenyl-2-(2-(4-butyl-4-(dimethylamino)-1-(5-fluoro-3-methyl-1H-indol-2-yl)cyclohexyl)-1H-indol-3-yl)ethyl carbamate;
• 1-(2-(2-(4-butyl-4-(dimethylamino)-1-(5-fluoro-3-methyl-1H-indol-2-yl)cyclohexyl)-1H-indol-3-yl)ethyl)-3-phenylurea;
• N,N-dimethyl-4-(3-methyl-1H-indol-2-yl)-1-phenyl-4-(3-(2-pyridin-4-yl)ethyl)-1H-indol-2-yl)cyclohexylamine;
• N,N-dimethyl-1-phenyl-4-(3-(2-(pyridin-4-yl)-ethyl)-1H-indol-2-yl)-4-(thiophen-2-yl)-cyclohexylamine
and physiologically compatible salts thereof.

6. Medication containing at least one compound according to one of claims 1 to 5 in the form of a single stereoisomer or mixture thereof, the free compounds and/or their physiologically compatible salts, and also possibly suitable additives and/or adjuvants and/or possibly further active substances.

7. Use of a compound according to one of claims 1 to 5 in the form of a single stereoisomer or mixture thereof, the free compounds and/or their physiologically compatible salts for the production of a medication for the treatment of pain.

8. Use of a compound according to one of claims 1 to 5 in the form of a single stereoisomer or mixture thereof, the free compounds and/or their physiologically compatible salts and/or solvates for the production of a medication for the treatment of anxiety conditions, stress and stress-related syndromes, depressive illnesses, epilepsy, Alzheimer's disease, senile dementia, general cognitive dysfunctions, learning and memory disabilities (as nootropic), withdrawal symptoms, alcohol and/or drug and/or medication misuse and/or dependence, sexual dysfunctions, cardiovascular diseases, hypotension, hypertension, tinitus, pruritus, migraine, hearing impairment, deficient intestinal motility, eating disorders, anorexia, bulimia, mobility disorders, diarrhea, cachexia, urinary incontinence, or as muscle relaxant, anticonvulsive or anaesthetic, or for coadministration in the treatment with an opioid analgesic or with an anaesthetic, for diuresis or anti-natriuresis, anxiolysis, for modulating movement activity, for modulating neurotransmitter release and for treating neuro-degenerative diseases associated therewith, for application in the treatment of withdrawal symptoms and/or for reducing the addiction potential of opioids.

## Revendications

1. Composé de formule générale (3) 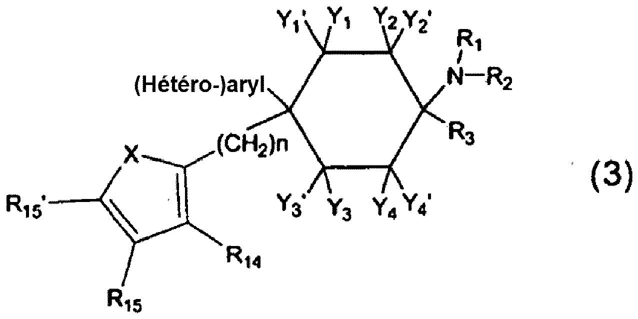 dans laquelle
Y₁, Y₁', Y₂, Y₂', Y₃, Y₃', Y₄ et Y₄' sont chacun choisis indépendamment les uns des autres dans le groupe constitué par -H, -F, -Cl, -Br, -I, -CN, -NO₂, -CHO,-R₀, -C(=O)R₀, -C(=O)H, -C(=O)-OH, -C(=O)OR₀, -C(=O)NH₂, - C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂,-NHC(=O)NHR₀ et -NHC(=O)N(R₀)₂ ; ou Y₁ et Y₁', ou Y₂ et Y₂', ou Y₃ et Y₃', ou Y₄ et Y₄' représentent ensemble =O ;
les R₀ représentent chacun indépendamment -aliphatique en C₁₋₈, -cycloaliphatique en C₃₋₁₂, -aryle, -hétéroaryle, -aliphatique en C₁₋₈-cycloaliphatique en C₃₋₁₂,-aliphatique en C₁₋₈-aryle, -aliphatique en C₁₋₈-hétéroaryle, -cycloaliphatique en C₃₋₈-aliphatique en C₁₋₈, -cycloaliphatique en C₃₋₂-aryle ou -cycloaliphatique en C₃₋₈-hétéroaryle ;
R₁ et R₂ représentent indépendamment l'un de l'autre -H ou -R₀ ; ou R₁ et R₂ représentent ensemble -CH₂CH₂OCH₂CH₂-, -CH₂CH₂NR₄CH₂CH₂- ou -(CH₂)₃₋₆- ;
R₃ représente -R₀ ;
R₄ représente -H, -R₀ ou -C(=O)R₀ ;
X représente -0-, -S-, -NR₁₆-, -CR₁₇=CR₁₈-, -CR₁₇=N- ou - N=CR₁₈- ;
R₁₄, R₁₅, R₁₅', R₁₆, R₁₇ et R₁₂ représentent chacun indépendamment les uns des autres -H, -F, -Cl, -Br, -I, -CN, -NO₂, -CHO, -R₀, -C(=O)R₀, -C(=O)H, -C(=O)OH,-C(=O)OR₀, -C(=O)NH₂, -C(=O)NHR₀, -C(=O)-N(R₀)₂, -OH,-O(CH₂)₁₋₂O-, -OR₀, -OC(=O)H, -OC(=O)R₀, -OC(=O)OR₀,-OC(=O)NHR₀, -OC(=O)N(R₀)₂, -SH, -SR₀, -SO₃H, -S (=0) ₁₋₂-R₀, -S(=O)₁₋₂NH₂, -NH₂, -NHR₀, -N(R₀)₂, -N⁺(R₀)₃, -N⁺(R₀)₂O⁻,-NHC(=O)R₀, -NHC(=O)OR₀, -NH-C(=O)NH₂, -NHC(=O)NHR₀,-NHC(=O)N(R₀)₂ ; ou R₁₄ et R₁₅, ou R₁₅ et R₁₅', ou R₁₅' et R₁₇ forment ensemble un cycle à cinq ou six éléments, saturé, partiellement insaturé ou aromatique, qui comprend éventuellement un ou deux hétéroatomes de cycle choisis indépendamment les uns des autres parmi N, S et 0 ; et qui est non substitué ou substitué une ou plusieurs fois avec des substituants choisis indépendamment les uns des autres dans le groupe constitué par -F, -Cl, -Br, -I, -CN, -NO₂, -CHO, -R₀, - C(=O)R₀, -C(=O)H, -C(=O)OH, -C(=O)OR₀, -C(=O)NH₂, C(=O)NHR₀, -C(=O)-N(R₀)₂, -OH, -O(CH₂)₁₋₂O-, -OR₀,-OC(=O)H, -OC(=O)R₀, -OC(=O)OR₀, -OC(=O)NHR₀, -OC(=O) - N(R₀)₂, -SH, -SR₀, -SO₃H, -S (=O)₁₋₂-R₀, -S (=O)₁₋₂NH₂, -NH₂, -NHR₀, -N(R₀)₂, -N⁺(R₀)₃, -N⁺(R₀)₂O-, -NHC(=O)-R₀,-NHC(=O)OR₀, -NH-C(=O)NH₂, -NHC(=O)NHR₀ et-NHC(=O)N(R₀)₂ ; de préférence -F, -Cl, -Br, -I, -CF₃,-CN et -NO₂ ;
n représente 0, 1 ou 2 ;
« aliphatique » étant à chaque fois un radical hydrocarboné aliphatique ramifié ou non ramifié, saturé ou mono- ou polyinsaturé, non substitué ou substitué une ou plusieurs fois ;
« cycloaliphatique » étant à chaque fois un radical hydrocarboné alicyclique, mono- ou multicyclique, saturé ou mono- ou polyinsaturé, non substitué ou substitué une ou plusieurs fois ;
en ce qui concerne « aliphatique » et « cycloaliphatique », « substitué une ou plusieurs fois » étant compris comme la substitution individuelle ou multiple d'un ou de plusieurs atomes d'hydrogène par des substituants choisis indépendamment les uns des autres dans le groupe constitué par -F, -Cl, -Br, -I,-CN, -NO₂, -CHO, =0, -R₀, -C(=O)R₀, -C(=O)H, -C(=O)OH,-C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀,-OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, NHC(=O)OR₀, -NHC(=O)NH₂, -NHC(=O)-NHR₀, -NH-C(=O)N(R₀)₂, -NHS(=O)₁₋₂R₀, -Si(R₀)₃ et -PO(OR₀)₂ ;
« (hétéro-)aryl » représente hétéroaryle ou aryle ;
« aryle » représente à chaque fois indépendamment un système cyclique carbocyclique contenant au moins un cycle aromatique, mais sans hétéroatomes dans ce cycle, les radicaux aryle pouvant éventuellement être condensés avec des systèmes cycliques saturés, (partiellement) insaturés ou aromatiques supplémentaires, et chaque radical aryle pouvant être non substitué ou substitué une ou plusieurs fois, les substituants de l'aryle pouvant être identiques ou différents, et pouvant se situer à toute position quelconque et possible de l'aryle ;
« hétéroaryle » représente un radical aromatique cyclique de 5, 6 ou 7 éléments, qui contient 1, 2, 3, 4 ou 5 hétéroatomes, les hétéroatomes étant identiques ou différents et représentant l'azote, l'oxygène ou le soufre, et l'hétérocycle pouvant être non substitué ou substitué une ou plusieurs fois ; les substituants pouvant être identiques ou différents, et pouvant se situer à toute position quelconque et possible de l'hétéroaryle en cas de substitution sur l'hétérocycle ; et l'hétérocycle pouvant également faire partie d'un système bi- ou polycyclique ;
en ce qui concerne « aryle » et « hétéroaryle », « substitué une ou plusieurs fois » étant compris comme la substitution individuelle ou multiple d'un ou de plusieurs atomes d'hydrogène du système cyclique par des substituants choisis dans le groupe constitué par - F, -Cl, -Br, -I, -CN, -NO₂, -CHO, =0, -R₀, -C(=O)R₀,-C(=O)H, -C(=O)OH, -C(=O)OR₀, -C(=O)NH₂, -C(=O)NHR₀,-C(=O)-N(R₀)₂, -OH, -O(CH₂)₁₋₂O-, -OR₀, -OC(=O)H,-OC(=O)R₀, -OC(=O)OR₀, -OC(=O)NHR₀, -OC(=O)N(R₀)₂, -SH,-SR₀, -SO₃H, -S(=O)₁₋₂-R₀, -S(=O)₁₋₂NH₂, -NH₂, -NHR₀,-N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃,-PO(OR₀)₂, -aliphatique en C₁₋₈-NHC(=O)R₀, -aliphatique en C₁₋₈-NHC(=O)OR₀, -aliphatique en C₁₋₈-NHC(=O)NHR₀,-aliphatique en C₁₋₈-NHC(=O)N(R₀)₂ et -aliphatique en C₁₋₈-NHS(=O)₁₋₂R₀ ; des atomes N de cycle éventuellement présents pouvant à chaque fois être oxydés ;
sous la forme d'un stéréoisomère individuel ou de leur mélange, des composés libres et/ou de leurs sels physiologiquement compatibles.

2. Composé selon la revendication 1, qui présente la formule générale (4)

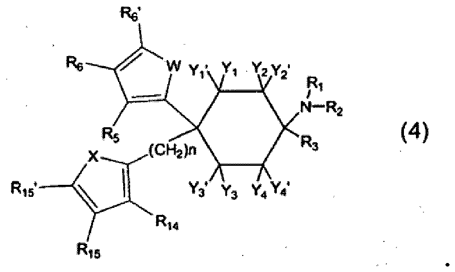

3. Composé selon la revendication 2, qui présente la formule générale (4.8.1)

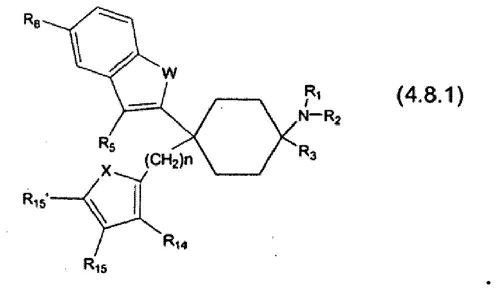

4. Composé selon la revendication 3, dans lequel
W représente -0- ou -NR₁₁- ;
X représente -0-, -NR₁₆- ou -CR₁₇=CR₁₈- ;
n représente 0 ou 1 ;
R₁ représente -CH₃ ;
R₂ représente -H ou -CH₃ ; ou
R₁ et R₂ forment ensemble un cycle et représentent-(CH₂)₃₋₄- ;
R₃ représente -aliphatique en C₁₋₈, -aryle ou-hétéroaryle, ceux-ci étant non substitués ou substitués une ou plusieurs fois avec des substituants choisis indépendamment les uns des autres dans le groupe constitué par -F, -Cl, -Br, -CN, -CH₃, -C₂H₅, -NH₂, -NO₂, -SH, -CF₃, -OH, -OCH₃, -OC₂H₅ et -N(CH₃)₂ ;
R₅ et R₁₄ représentent indépendamment l'un de l'autre - H, -F, -aliphatique en C₁₋₈, -aliphatique en C₁₋₈-aryle ou -aliphatique en C₁₋₈-hétéroaryle ;
R₈ représente -F, -Cl, -Br, -I, -CF₃, -CN ou -NO₂ ;
R₁₁ représente -H ;
R₁₄, R₁₅ et R₁₅' représentent indépendamment les uns des autres -H, -F, -Cl, -Br, -CN, -CH₃, -C₂H₅, -NH₂, -NO₂,-SH, -CF₃, -OH, -OCH₃, -OC₂H₅ ou -N(CH₃)₂ ; ou R₁₅ et R₁₅' forment ensemble un cycle à six éléments, saturé, partiellement insaturé ou aromatique, qui peut éventuellement comprendre un ou deux hétéroatomes de cycle, qui sont choisis indépendamment les uns des autres parmi N, S et 0 ; ce cycle formé pouvant être non substitué ou substitué une ou plusieurs fois, les substituants étant choisis indépendamment les uns des autres dans le groupe constitué par -F, -Cl, -Br, -I,-CF₃, -CN et -NO₂,
R₁₆ représente -H ; et
R₁₇ et R₁₈ représentent indépendamment l'un de l'autre -H ou -F.

5. Composé selon la revendication 1, choisi dans le groupe constitué par :
• la 1-butyl-N,N-diméthyl-4,4-bis(3-méthyl-5-(trifluorométhyl)-1H-indol-2-yl)cyclohexanamine ;
• la 1-butyl-4,4-bis(5-fluoro-3-méthyl-1H-indol-2-yl)-N,N-diméthylcyclohexanamine ;
• la 1-benzyl-N,N-diméthyl-4,4-bis(3-(2-(pyridin-4-yl)éthyl)-1H-indol-2-yl)cyclohexanamine ;
• la N,N-diméthyl-4,4-bis(3-(2-(pyridin-4-yl)éthyl)-1H-indol-2-yl)-1-(thiophén-2-yl)cyclohexanamine ;
• le 2,2'-(4-butyl-4-(pyrrolidin-1-yl)cyclohexane-1,1-diyl)bis(3-(2-(pyridin-4-yl)éthyl)-1H-indole) ;
• la N-méthyl-1-phényl-4,4-bis(3-(2-(pyridin-4-yl)éthyl)-1H-indol-2-yl)cyclohexanamine ;
• la N,N-diméthyl-4-(3-méthyl-1H-indol-2-yl)-1-phényl-4-(thiophén-2-yl)cyclohexanamine ;
• la N,N-diméthyl-4-(3-méthyl-1H-indol-2-yl)-1,4-diphénylcyclohexanamine ;
• la N,N-diméthyl-4,4-bis(3-méthylbenzofuran-2-yl)-1-phénylcyclohexanamine ;
• la N,N-diméthyl-4,4-bis(3-méthyl-1H-indol-2-yl)-1-phénylcyclohexanamine ; 1,2,3-tricarboxylate de 2-hydroxypropane ;
• la 1-butyl-N,N-diméthyl-4,4-bis(3-(2-(pyridin-4-yl)éthyl)-1H-indol-2-yl)cyclohexanamine ; 1,2,3-tricarboxylate de 2-hydroxypropane ;
• la N,N,4-triméthyl-4-(3-méthyl-1H-indol-2-yl)-1-phénylcyclohexanamine ;
• la 4-benzyl-N,N-diméthyl-4-(3-méthyl-1H-indol-2-yl)-1-phénylcyclohexanamine ;
• le 2,2'-(2,2'-(4-butyl-4-(diméthylamino)cyclohexane-1,1-diyl)bis(1H-indol-3,2-diyl))diacétate de diméthyle ;
• le 2,2'-(2,2'-(4-butyl-4-(diméthylamino)cyclohexane-1,1-diyl)bis(1H-indol-3,2-diyl))-diéthanol ;
• la 1-butyl-N,N-diméthyl-4-(3-méthyl-1H-indol-2-yl)-4-(3-(2-pyridin-4-yl)éthyl)-1H-indol-2-yl)cyclohexylamine ;
• la 1-butyl-4,4-bis-(3-(2-(3,4-dihydro-1H-isoquinolin-2-yl)éthyl)-1H-indol-2-yl)-N,N-diméthylcyclohexylamine ;
• la 4-(4-méthoxyphényl)-N,N-diméthyl-4-(3-méthyl-1H-indol-2-yl)-1-phénylcyclohexylamine ;
• la 1-butyl-N,N-diméthyl-4,4-bis-(3-méthyl-1H-indol-2-yl)cyclohexylamine ;
• la 2-(2-(2-(4-butyl-4-diméthylamino-1-(3-méthyl-1H-indol-2-yl)cyclohexyl)-1H-indol-3-yl)éthyl)isoindoline-1,3-dione ;
• la 2-[2-[2-[4-butyl-4-diméthylamino-1-[3-[2-(1,3-dioxo-2H-isoindol-2-yl)-éthyl]-1H-indol-2-yl]-cyclohexyl]-1H-indol-3-yl]-éthyl]-2H-isoindole-1,3-dione ;
• la 4-(benzo[d][1,3]dioxol-5-yl)-N,N-diméthyl-4-(3-méthyl-1H-indol-2-yl)-1-phényl-cyclohexylamine ;
• le 3,3'-(2-(4-butyl-4-(diméthylamino)cyclohexane-1,1-diyl)bis(1H-indol-2,3-diyl)dipropanate de diméthyle ;
• la 4-(3-(2-aminoéthyl)-1H-indol-2-yl)-1-butyl-N,N-diméthyl-4-(3-méthyl-1H-indol-2-yl)cyclohexylamine ;
• la 1-(3-fluorophényl)-N,N-diméthyl-4-(3-méthyl-1H-indol-2-yl)-4-thiophén-2-ylcyclohexylamine ;
• la 1-butyl-4,4-bis-(1,3-diméthyl-1H-indol-2-yl)-N,N-diméthylcyclohexylamine ;
• la 1-butyl-4-(1,3-diméthyl-1H-indol-2-yl)-N,N-diméthyl-4-(3-méthyl-1H-indol-2-yl)cyclohexylamine ;
• la 1-benzyl-N,N-diméthyl-4-(3-méthyl-1H-indol-2-yl)-4-(thiophén-2-yl)cyclohexylamine ;
• la 4-(3-méthoxyphényl)-N,N-diméthyl-4-(3-méthyl-1H-indol-2-yl)-1-phénylcyclohexylamine ;
• l'amide de l'acide N-(2-(2-(4-butyl-4-(diméthylamino)-1-(3-méthyl-1H-indol-2-yl)cyclohexyl)-1H-indol-3-yl)éthyl)cyclopentanesulfonique ;
• la 1-(3-fluorophényl)-N,N-diméthyl-4-(3-méthyl-1H-indol-2-yl)-4-thiophén-2-ylcyclohexylamine ;
• la N,N-diméthyl-1-phényl-4,4-bis-(3-(2-pyridin-4-yl)éthyl)-1H-indol-2-yl)cyclohexylamine ;
• la 1-(2-(2-(4-butyl-4-(diméthylamino)-1-(3-méthyl-1H-indol-2-yl)cyclohexyl)-1H-indol-3-yl)éthyl)-3-phénylurée ;
• la 1,1'-(2,2'-(2,2'-(4-butyl-4(diméthylamino)cyclohexane-1,1-diyl)bis(1H-indol-3,2-diyl))bis(éthane-2,1-diyl)bis(3-phénylurée) ;
• la 1-butyl-4,4-bis(5-fluoro-3-méthyl-1H-indol-2-yl)-N,N-diméthylcyclohexylamine ;
• la 4-(3-(2-aminoéthyl)-1H-indol-2-yl)-1-butyl-4-(5-fluoro-3-méthyl-1H-indol-2-yl)-N,N-cyclohexylamine ;
• le carbamate de (phényl-2-(2-(4-butyl-4-(diméthylamino)-1-(5-fluoro-3-méthyl-1H-indol-2-yl)cyclohexyl)-1H-indol-3-yl)éthyle ;
• la 1-(2-(2-(4-butyl-4-(diméthylamino)-1-(5-fluoro-3-méthyl-1H-indol-2-yl)cyclohexyl)-1H-indol-3-yl)éthyl)-3-phénylurée ;
• la N,N-diméthyl-4-(3-méthyl-1H-indol-2-yl)-1-phényl-4-(3-(2-pyridin-4-yl)éthyl)-1H-indol-2-yl)cyclohexylamine ;
• la N,N-diméthyl-1-phényl-4-(3-(2-(pyridin-4-yl)-éthyl)-1H-indol-2-yl)-4-(thiophén-2-yl)-cyclohexylamine ;
et leurs sels physiologiquement compatibles.

6. Médicament contenant au moins un composé selon l'une quelconque des revendications 1 à 5 sous la forme d'un stéréoisomère individuel ou de leur mélange, des composés libres et/ou de leurs sels physiologiquement compatibles, ainsi qu'éventuellement des additifs et/ou adjuvants appropriés et/ou éventuellement d'autres agents actifs.

7. Composé selon l'une quelconque des revendications 1 à 5 sous la forme d'un stéréoisomère individuel ou de leur mélange, des composés libres et/ou de leurs sels physiologiquement compatibles, destinés à une utilisation pour le traitement de la douleur.

8. Composé selon l'une quelconque des revendications 1 à 5 sous la forme d'un stéréoisomère individuel ou de leur mélange, des composés libres et/ou de leurs sels et/ou solvates physiologiquement compatibles, destinés à une utilisation pour le traitement des états d'anxiété, du stress et des syndromes associés avec le stress, des dépressions, de l'épilepsie, de la maladie d'Alzheimer, de la démence sénile, des dysfonctionnements cognitifs généraux, des difficultés d'apprentissage et de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse, l'anxiolyse, pour la modulation de l'activité motrice, pour la modulation de la distribution des neurotransmetteurs et le traitement des maladies neurodégénératives associées, pour le traitement des phénomènes de sevrage et/ou pour la réduction du potentiel d'accoutumance des opioïdes.
